# EUROPÄISCHE PATENTSCHRIFT

(11) **EP 1 599 452 B1**
(45) Veröffentlichungstag und Bekanntmachung des Hinweises auf die Patenterteilung: **20.06.2007**
(21) Anmeldenummer: 04712490.4
(22) Anmeldetag: 19.02.2004
(51) Int. Cl.: C07D 263/32, C07D 413/04, A61K 31/421, A61K 31/422, A61P 3/10, A61P 9/10

(54) **3-(2-PHENYL-OXAZOL-4- YLMETHOXY)-CYCLOHEXYLMETHOXY -ESSIGSÄURE DERIVATE UND VERWANDTE VERBINDUNGEN ALS PPAR MODULATOREN ZUR BEHANDLUNG VON TYP 2 DIABETES UND ATHEROSKLEROSE**
3-(2-PHENYL-OXAZOL-4-YL METHOXY) CYCLOHEXYLMETHOXY ACETIC ACID DERIVATIVES AND RELATED COMPOUNDS USED AS PPAR MODULATORS FOR TREATING TYPE 2 DIABETES AND ARTERIOSCLEROSIS
DERIVES D'ACIDE ACETIQUE 3-(2-PHENYL-OXAZOL-4- YLMETHOXY)-CYCLOHEXYLMETHOXY ET COMPOSES APPARENTES COMME MODULATEURS PPAR POUR TRAITER LE DIABETE DE TYPE 2 ET L'ATHEROSCLEROSE

(30) Priorität: 27.02.2003 DE 10308355
(43) Veröffentlichungstag der Anmeldung: 30.11.2005
(73) Patentinhaber: Sanofi-Aventis Deutschland GmbH, 65929 Frankfurt am Main (DE)
(72) Erfinder: STAPPER, Christian, 55118 Mainz (DE); GRETZKE, Dirk, 60596 Frankfurt (DE); GLOMBIK, Heiner, 65719 Hofheim (DE); FALK, Eugen, 60529 Frankfurt (DE); GOERLITZER, Jochen, 60594 Frankfurt am Main (DE); KEIL, Stefanie, 65719 Hofheim (DE); SCHAEFER, Hans-Ludwig, 65239 Hochheim (DE); WENDLER, Wolfgang, 65618 Selters (DE)
(86) Internationale Anmeldenummer: PCT/EP2004/001579
(87) Internationale Veröffentlichungsnummer: WO 2004/076427

(56) Entgegenhaltungen:
- EP-A- 1 067 109
- EP-A- 1 108 713
- WO-A-00/64876
- WO-A-02/16331
- WO-A-02/051820

## Beschreibung

Die Erfindung betrifft Cycloalkyl-methoxy substituierte Essigsäurederivate, Verfahren zu ihrer Herstellung und ihre Anwendung als Arzneimittel sowie deren physiologisch verträgliche Salze und physiologisch funktionelle Derivate.

Es sind bereits strukturähnliche Verbindungen zur Behandlung von Hyperlipidämie und Diabetes im Stand der Technik beschrieben (WO 2000/64876).

Der Erfindung lag die Aufgabe zu Grunde, Verbindungen zur Verfügung zu stellen, die eine therapeutisch verwertbare Modulation des Lipid- und/oder Kohlehydratstoffwechsels erlauben und sich somit zur Prävention und/oder Behandlung von Krankheiten wie Typ 2 Diabetes und Atherosklerose sowie deren vielfältigen Folgeerkrankungen eignen.

Überraschenderweise wurde eine Serie von Verbindungen gefunden, die die Aktivität von PPAR Rezeptoren modulieren. Insbesondere eignen sich die Verbindungen zur Aktivierung von PPARalpha und PPARgamma, wobei das Ausmaß der relativen Aktivierung je nach Verbindungen unterschiedlich sein kann.

Die Erfindung betrifft daher Verbindungen der Formel I 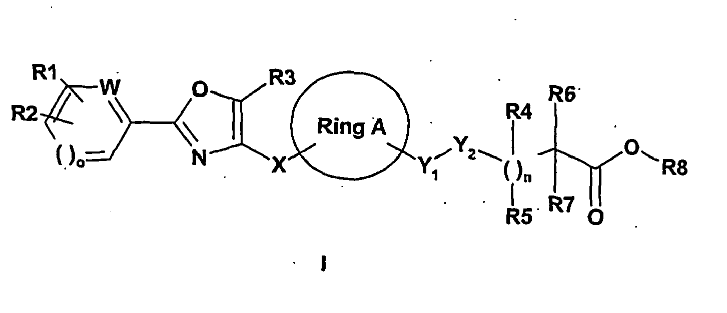 worin bedeuten:
- Ring A: Cyclodexan-1,3-diyl
- R1, R2: unabhängig voneinander H, F, Cl, Br, CF₃, OCF₃, (C1-C6)-Alkyl, O-(C1-C6)-Alkyl, SCF₃, SF₅, OCF₂-CHF₂, (C6-C10)-Aryl, (C6-C10)-Aryloxy, OH, NO₂; oder
- R1 und R2: zusammengenommen mit dem Phenyl-, Pyridin-, 1-H-Pyrrol-, Thiophen- oder Furan-Ring kondensiertes, teilweise oder nicht gesättigtes bicyclisches (C6-C10)-Aryl, (C5-C11)-Heteroaryl;
- R3: H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5-C6)-Heteroaryl oder (C1-C3)-Alkyl, das durch F ganz oder teilweise substituiert ist;
- o: O oder 1;
- W: CH, N, falls o = 1;
- W: O, S, NR10, falls o = 0;
- X: (C1-C6)-Alkandiyl, wobei in der Alkandiylgruppe ein oder mehrere Kohlenstoffatome durch Sauerstoffatome ersetzt sein können;
- Y1: (CR13R14)p;
- p: 1, 2;
- Y2: CH2, O, S, SO, SO2, NR9;-
- n: 0-2;
- R4: H, (C1-C6)-Alkyl; oder F, falls Y2 ungleich O, NR9;
- R5: H, (C1-C6)-Alkyl; oder F, falls Y2 ungleich O, NR9;
- R6: H, (C1-C6)-Alkyl; oder F, falls n ungleich 0;
- R7: H, F (falls n ungleich 0), (C1-C6)-Alkyl, (C1-C6)-Alkoxy, (C2-C6)-Alkenyl, (C2-C6)-Alkinyl, (C3-C8)-Cycloalkyl, Phenyl, wobei Alkyl gegebenenfalls substituiert sein kann durch ein oder mehrere Reste aus der Reihe Hydroxy, Phenyl, (C5-C11)-Heteroaryl, (C1-C6)-Alkoxy und NR11 R12, und Phenyl gegebenfalls substituiert sein kann durch ein oder mehrere Reste aus der Reihe Hydroxy, (C1-C6)-Alkoxy, F und CF₃; mit der Maßgabe, dass R7 ungleich NR11 R12 oder (C1-C6)-Alkoxy, falls R6 = F ist;
- R6 und R7: zusammen mit dem sie tragenden C-Atom (C3-C8)-Cycloalkyl;
- R8: H, (C1-C6)-Alkyl;
- R9: H, (C1-C6)-Alkyl, (C2-C6)-Alkenyl, (C2-C6)-Alkinyl, Aryl-(C1-C4)alkyl, CO-(C1-C6)-Alkyl, CO-(C6-C10)-Aryl, CO-(C1-C6)-Alkyl-(C6-C10)-Aryl, CO-(C5-C11)-Heteroaryl, C(O)-O-(C1-C6)-Alkyl, C(O)-O-(C1-C6)-Alkyl-(C6-C10)-Aryl, C(O)-O-(C6-C10)-Aryl, C(O)-O-(C5-C11)-Heteroaryl, SO2-(C1-C6)-Alkyl, SO2-(C1-C6)-Alkyl-(C6-C10)-Aryl, SO2-(C1-C6)-Alkyl-SO2-(C1-C6)-alkyl, SO2-(C6-C10)-Aryl, SO2-(C5-C11)-Heteroaryl, wobei Aryl und Heteroaryl gegebenenfalls substituiert sein können durch (C1-C6)-Alkyl, O-(C1-C6)-Alkyl, F, Cl, CO-(C1-C6)-Alkyl;
- R10: H, (C1-C6)-Alkyl, (C1-C6)-Alkyl-Phenyl;
- R11: H, (C1-C6)-Alkyl, (C1-C6)-Alkyl-Phenyl;
- R12: H, (C1-C6)-Alkyl, (C1-C6)-Alkyl-Phenyl;
- R13: H, (C1-C6)-Alkyl;
- R14: H, (C1-C6)-Alkyl;
sowie deren physiologisch verträgliche Salze.

Bevorzugt sind Verbindungen der Formel I, in denen
- Ring A: Cyclohexan-1,3-diyl
- X: (C1-C6)-Alkandiyl, wobei in der Alkandiylgruppe das C1- oder C2-Kohlenstoffatom (zum Ring A) durch Sauerstoffatom ersetzt ist.

Besonders bevorzugt sind die Verbindungen der Formel I, worin ein oder mehrere Reste die folgenden Bedeutungen haben:
- Ring A: Cyclohexan-1,3-diyl; oder
- R1: F, Br, CF₃, OCF₃, (C1-C6)-Alkyl, O-(C1-C6)-Alkyl, Phenyl; oder
- R1 und R2: zusammen mit dem Phenylring = Naphthyl; oder
- R1: in meta- oder in para-Stellung; oder
- R2: Wasserstoff ist; oder
- R3: H, (C1-C6)-Alkyl, (C3-C8)-Cycloalkyl, (C1-C3)-Alkyl-(C5-C6)-Cycloalkyl, Phenyl, (C1-C3)-Alkyl-Phenyl; oder
- W: CH, falls o = 1; oder
- X: CH₂-O oder CH₂-O-CH₂; oder
- n: 0; oder
- R6: H, (C1-C6)-Alkyl; oder
- R6 und R7: zusammen mit dem sie tragenden C-Atom (C3-C6)-Cycloalkyl, besonders Cyclopentyl; oder
- R7: (C1-C6)-Alkyl, wobei Alkyl gegebenenfalls substituiert sein kann durch ein oder mehrere Reste aus der Reihe Hydroxy, Phenyl, (C5-C11)-Heteroaryl, (C1-C6)-Alkoxy und NR11 R12, mit.
- R11 und R12: H, (C1-C6)-Alkyl; oder
- R13 und R14: Wasserstoff sind.

Besonders bevorzugt sind ferner die Verbindungen der Formel I, worin bedeuten
- R7: (C1-C4)-Alkyl, (C1-C4)-Alkyl-O-(C1-C4)-Alkyl oder Benzyl;
ganz besonders bevorzugt
- R7: (C1-C4)-Alkyl oder, Benzyl.

Ganz besonders bevorzugt sind ferner die Verbindungen der Formel I,
worin bedeuten
- Ring A: cis-Cyclohexan-1,3-diyl
- R1, R2: unabhängig voneinander H, F, CF3, (C1-C6)-Alkyl, O-(C1-C6)-Alkyl, Phenyl, oder
- R1 und R2: zusammengenommen mit dem Phenylring = Naphthyl;
- R3: (C1-C6)-Alkyl, (C3-C8)-Cycloalkyl, Phenyl;
- o: O oder 1
- W: CH, falls o = 1;
- W: O, S, falls o = 0;
- X: CH2-O, CH2-O-CH2;
- Y1: CH2,;
- Y2: CH2, O, S, SO, SO2, NR9;
- n: 0;
- R4: H;
- R5: H;
- R6: H, (C1-C6)-Alkyl, Benzyl;
- R7: H, (C1-C6)-Alkyl, (C3-C6)-Cycloalkyl, Phenyl, Benzyl,
- R6 und R7: zusammen mit dem sie tragenden C-Atom (C3-C6)-Cycloalkyl;
- R8: H;
- R9: H; (C1-C6)-Alkyl, das gegebenenfalls substituiert sein kann durch (C3-C6)-Cycloalkyl, Phenyl, (C5-C6)-Heteroaryl; CO-(C1-C6)-Alkyl, CO-(C1-C6)-Alkyl-Phenyl, CO-Phenyl, C(O)-O-(C1-C6)-Alkyl, CO-NH-Phenyl, SO2-(C1-C4)-Alkyl, SO2-(C1-C4)-Alkyl-SO2-(C1-C4)-alkyl, SO2-Tolyl, wobei Phenyl wiederum substituiert sein kann durch O-(C1-C3)-Alkyl;
sowie deren physiologisch verträgliche Salze.

Die Alkylreste in den Substituenten R1, R2, R3, R4, R5 , R6, R7, R8, R9, R10, R11, R12, R13 und R14 können sowohl geradkettig wie verzweigt sein.

Unter Aryl wird ein aromatisches carbocyclisches mono- oder bicyclisches RingSystem verstanden, das 6 bis 10 Atome im Ring oder in den Ringen enthält.

Heteroaryl ist ein mono- oder bicyclisches aromatisches Ringsystem mit 4 bis 11 Ringgliedern, worin mindestens ein Atom im Ringsystem ist ein Heteroatom aus der Reihe N; O und S.

Die Verbindungen der Formel I enthalten mindestens zwei Assymmetriezentren und können darüber hinaus mehr enthalten. Daher können die Verbindungen der Formel I in Form ihrer Racemate, racemischen Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, ,Ethansulfon-, Fumar-, Glucon-, Glykol-, lsethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

### Verwendung

Diese Erfindung bezieht sich weiterhin auf die Verwendung von Verbindungen der Formeln I und ihren pharmazeutischen Zusammensetzungen als PPAR-Rezeptor-Liganden. Die erfindungsgemäßen PPAR-Rezeptor-Liganden eignen sich als Modulatoren der Aktivität der PPAR Rezeptoren.
Peroxisomen-Proliferatoren-Aktivierte Rezeptoren (PPAR) sind durch Liganden aktivierbare Transkriptionsfaktoren, die zur Klasse der Kern-Hormon-Rezeptoren gehören. Es existieren drei PPAR Isoformen, PPARalpha, PPARgamma und PPARdelta, die von verschiedenen Genen kodiert werden (Peroxisome proliferator-activated receptor (PPAR): structure, mechanisms of activation and diverse functions: Motojima K, Cell Struct Funct. 1993 Oct; 18(5): 267-77).
Von PPARgamma existieren zwei Varianten, PPARgamma₁ und gamma₂, die das Ergebnis eines alternativen Einsatzes von Promotoren und einer differenziellen mRNA-Spleißung (Vidal-Puig et al. J. Clin. Invest., 97:2553-2561, 1996) sind. Die verschiedenen PPAR Rezeptoren besitzen eine unterschiedliche Gewebsverteilung und modulieren unterschiedliche physiologische Funktionen. Die PPAR-Rezeptoren spielen eine Schlüsselrolle bei unterschiedlichen Aspekten der Regulation einer Vielzahl von Genen, deren Genprodukte direkt oder indirekt am Lipid- und Kohlehydratstoffwechsel entscheidend beteiligt sind. So spielen z. B. PPARalpha Rezeptoren bei der Regulation des Fettsäurekatabolismus oder des Lipoproteinstoffwechsels in der Leber eine wichtige Rolle, während PPARgamma zum Beispiel bei der Regulation der Fettzelldifferenzierung entscheidend beteiligt ist. Darüberhinaus sind PPAR Rezeptoren aber auch an der Regulation vieler weiterer physiologischer Prozesse, auch solcher die nicht direkt mit dem Kohlehydrat- oder Lipidstoffwechsel in Verbindung stehen, beteiligt. Die Aktivität der unterschiedlichen PPAR Rezeptoren kann durch verschiedene Fettsäuren, Fettsäurederivate sowie synthetische Verbindungen in unterschiedlichem Ausmaß moduliert werden. Entsprechende Reviews über Funktionen, physiologische Wirkung und Pathophysiologie siehe: Joel Berger et al., Annu. Rev. Med. 2002, 53,409-435; Timothy Wilson et al. J. Med. Chem., 2000, Vol. 43, No. 4, 527-550; Steven Kliewer et al., Recent Prog Horm Res. 2001; 56: 239-63.
Die vorliegende Erfindung betrifft Verbindungen der Formel I, die sich zur Modulierung der Aktivität von PPAR Rezeptoren eignen, insbesondere der Aktivität von PPARalpha und PPARgamma. Je nach Profil der Modulation sind die Verbindungen der Formel I zur Behandlung, Kontrolle und Prohylaxe nachfolgend beschriebener Indikationen, sowie einer Reihe anderer, damit verbundener pharmazeutischer Anwendungen geeignet (siehe z.B. Joel Berger et al., Annu. Rev. Med. 2002, 53, 409 -435; Timothy Wilson et al. J. Med. Chem., 2000, Vol. 43, No. 4, 527-550; Steven Kliewer et al., Recent Prog Horm Res. 2001; 56: 239-63; Jean-Charles Fruchart, Bart Staels and Patrick Duriez: PPARS, Metabolic Disease and Arteriosclerosis, Pharmacological Research, Vol. 44, No. 5, 2001; Sander Kersten; Beatrice Desvergne & Walter Wahli: Roles of PPARs in health and disease, NATURE, VOL 405 ,25 MAY 2000 ; Ines Pineda Torra, Giulia Chinetti, Caroline Duval, Jean-Charles Fruchart and Bart Staels: Peroxisome proliferator-activated receptors: from transcriptional control to clinical practice, Curr Opin Lipidol 12: 2001, 245-254).
Besonders geeignet sind solche Verbindungen zur Behandlung und/oder Prävention von
1. - Störungen des Fettsäurestoffwechsels und Glucoseverwertungsstörungen
   - Störungen, bei denen Insulin Resistenz eine Rolle spielt
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der β-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzimone, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrome X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - lschämie/Reperfusions Syndrom
   - Akutes respiratorisches Distress Syndrom (ARDS) ("Schocklunge")

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg und 50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster ,enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formeln I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.
Bei einer Ausführungsform der Erfindung werden die Verbindungen der Formel in Kombination mit Substanzen, die Einfluss haben auf die hepatische Glukoseproduktion, wie z.B. Glycogen Phosphorylase Inhibitoren (siehe: WO 01/94300, WO 02/096864, WO 03/084923, WO 03/084922, WO 03/104188)
Bei einer Ausführungsform werden die Verbindungen der Formel in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor (siehe z.B. US 6,245,744, US 6,221,897, US 6,277,831, EP 0683 773, EP 0683 774) verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties &Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromak^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, *β*3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1 H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR*-β-*Agonisten) verabreicht.

Bei einer Ausführungsform der Erfindung ist der weitere Wirkstoff Leptin.

Bei einer Ausführungsform ist der weitere Wirkstoff Dexamphatamin, Amphetamin, Mazindol oder Phentermin.
Bei einer Ausführungsform werden die Verbindungen der Formel I in Kombination mit Medikamenten mit Wirkungen auf das Herz-Kreislauf- und das Blutgefäß-System, wie verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Die Wirksamkeit der Verbindungen wurde wie folgt getestet:

### Bestimmung von EC50-Werten von PPAR-Agonisten im zellulären PPARalpha-Test

### Prinzip

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als PPARalpha-Reporterzellinie bezeichnet wird. Sie enthält zwei genetische Elemente, ein Luziferase-Reporterelement (pdeltaM-GAL4-Luc-Zeo) und ein PPARalpha-Fusionsprotein (GR-GAL4-humanPPARalpha-LBD), welches die Expression des Luziferase-Reporterelementes in Abhängigkeit eines PPARalpha-Liganden vermittelt. Das stabil und konstitutiv exprimierte Fusionsprotein GR-GAL4-humanPPARalpha-LBD bindet im Zellkern der PPARalpha-Reporterzellinie über den GAL4-Proteinanteil an die GAL4-DNA-Bindungsmotife 5'-oberhalb des Luziferase-Reporterelementes, das im Genom der Zellinie integriert ist. Ohne Zugabe eines PPARalpha-Liganden ist die Expression des Luziferase-Reportergens nur gering, sofern im Test fettsäuredepletiertes fötales Kälberserum (cs-FKS) verwendet wird. PPARalpha-Liganden binden und aktivieren das PPARalpha-Fusionsprotein und bewirken darüber die Expression des Luciferasereportergens. Die gebildete Luziferase lässt sich über ein entsprechendes Substrat mittels Chemilumineszenz nachweisen.

### Konstruktion der Zelllinie

Die PPARalpha-Reporterzellinie wurde in 2 Stufen hergestellt: Zuerst wurde das Luziferase-Reporterelement konstruiert und stabil in HEK-Zellen transfiziert. Dazu wurden fünf Bindungsstellen des Hefe-Transkriptionsfaktors GAL4 (jeweils 5'-CGGAGTACTGTCCTCCGAG-3') 5'-oberhalb eines 68 bp langen minimalen MMTV-Promotors (Genbank-Accession # V01175) einkloniert. Der minimale MMTV-Promotorabschnitt enthält eine CCAAT-Box und ein TATA-Element, um eine effiziente Transkription durch die RNA-Polymerase II zu ermöglichen. Die Klonierung und Sequenzierung des GAL4-MMTV-Konstruktes erfolgte analog wie bei Sambrook J. et. al. beschrieben (Molecular cloning, Cold Spring Harbor Laboratory Press, 1989). Danach wurde 3'-unterhalb des GAL4-MMTV-Elementes das gesamte Luziferasegen von Photinus pyralis (Genbank-Accession # M15077) einkloniert. Nach der Sequenzierung wurde das aus fünf GAL4-Bindungsstellen, MMTV-Promotor und Luziferasegen bestehende Luziferase-Reporterelement in ein Zeozin-Resistenz vermittelndes Plasmid umkloniert, um zu dem Plasmid pdeltaM-GAL4-Luc-Zeo zu gelangen. Dieser Vektor wurde nach den Angaben in Ausubel, F.M. et al. (Current protocols in molecular biology, Vol. 1-3, John Wiley & Sons, Inc., 1995) in HEK-Zellen transfiziert. Danach wurde unter Verwendung von zeozinhaltigem Medium (0,5 mg/ml) ein geeigneter stabiler Zellklon selektioniert, der eine möglichst niedrige Basalexpression des Luziferasegens zeigte.
In einem zweiten Schritt wurde das PPARalpha-Fusionsprotein (GR-GAL4-humanPPARalpha-LBD) in den beschriebenen stabilen Zellklon eingebracht. Dazu wurde zunächst die für die N-terminalen 76 Aminosäuren des Glukocorticoid-Rezeptors (Genbank-Accession # P04150) kodierende cDNA mit dem für die Aminosäuren 1-147 des Hefetranskriptionsfaktors GAL4 (Genbank-Accession # P04386) kodierenden cDNA-Abschnitt verknüpft. Am 3'-Ende dieses GR-GAL4-Konstruktes wurde die cDNA der Ligandenbindungsdomäne des humanen PPARalpha-Rezeptors einkloniert (Aminosäuren S167-Y468; Genbank-Accession # S74349). Das so hergestellte Fusionskonstrukt (GR-GAL4-humanPPARalpha-LBD) wurde in das Plasmid pcDNA3 (Firma Invitrogen) umkloniert, um darin eine konstitutive Expression durch den Cytomegalovirus-Promotor zu ermöglichen. Dieses Plasmid wurde mit einer Restriktionsendonuklease linearisiert und stabil in den bereits beschriebenen, das Luziferase-Reporterelement enthaltenden Zellklon transfiziert. Durch Selektion mit Zeozin (0,5 mg/ml) und G418 (0,5 mg/ml) wurde die fertige PPARalpha-Reporterzellinie isoliert, die ein Luziferase-Reporterelement enthält und konstitutiv das PPARalpha-Fusionsprotein (GR-GAL4-humanPPARalpha-LBD) exprimiert.

### Durchführung des Tests

Die Aktivität von PPARalpha-Agonisten wird in einem 3-Tagestest bestimmt, der nachfolgend beschrieben ist:

### Tag 1

Die PPARalpha-Reporterzellinie wird bis zu einer 80 %-igen Konfluenz in DMEM-Medium (# 41965-039, Invitrogen) kultiviert, das mit folgenden Zusätzen versetzt ist: 10% cs-FKS (fötales Kälberserum; #SH-30068.03, Hyclone), 0,5 mg/ml Zeozin (#R250-01, Invitrogen), 0,5 mg/ml G418 (#10131-027, Invitrogen), 1% Penicillin-Streptomycin-Lösung (#15140-122, Invitrogen) und 2 mM L-Glutamin (#25030-024, Invitrogen). Die Kultivierung erfolgt in Standard-Zellkulturtlaschen (# 353112, Becton Dickinson) in einem Zellkulturbrutschrank bei 37°C in Anwesenheit von 5% CO₂. Die zu 80% konfluenten Zellen werden einmal mit 15 ml PBS gewaschen (#14190-094, Invitrogen), mit 3 ml Trypsinlösung (#25300-054, Invitrogen) für 2 min bei 37°C behandelt, in 5 ml des beschriebenen DMEM-Mediums aufgenommen und in einem Zellzählgerät gezählt. Nach der Verdünnung auf 500.000 Zellen/ml werden jeweils 35.000 Zellen pro well einer 96 well-Mikrotiterplatte mit klarem Plastikboden (#3610, Corning Costar) ausgesät. Die Platten werden für 24 h in einem Zellkulturbrutschrank bei 37°C und 5% CO₂ inkubiert.

### Tag 2

Zu testende PPARalpha-Agonisten werden in einer Konzentration von 10 mM in DMSO gelöst. Diese Stocklösung wird in DMEM-Medium (#4.1965-039, Invitrogen) verdünnt, das mit 5 % cs-FKS (#SH-30068.03, Hyclone), 2 mM L-Glutamin (#25030-024, Invitrogen) und den bereits beschriebenen Antibiotika (Zeozin, G418, Penicillin und Streptomycin) versetzt ist.
Testsubstanzen werden in 11 verschiedenen Konzentrationen im Bereich von 10 µM bis 100 pM getestet. Potentere Verbindungen werden in Konzentrationsbereichen von 1 µM bis 10 pM oder zwischen 100 nM und 1 pM geprüft.
Das Medium der an Tag 1 ausgesäten PPARalpha-Reporterzellinie wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden. Jede Platte wurde mit einem Standard PPARalpha-Agonisten belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37 °C und 5 % CO₂ inkubiert.

### Tag 3

Die mit den Testsubstanzen behandelten PPARαλπηα-Reporterzellen werden aus dem Brutschrank entnommen und das Medium abgesaugt. Zur Lyse der Zellen werden 50 µl Bright Glo Reagens (Firma Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Messzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von PPAR-Agonisten werden mit dem Program XL.Fit nach Vorgabe des Herstellers (Firma IDBS) berechnet.

Die PPARalpha-EC50-Werte für die Verbindungen der Beispiele 1 bis 85 liegen in diesem Assay im Bereich von 0,07nM bis >10 µM.

Die Ergebnisse für die Aktivität einiger erfindungsgemäßer Verbindungen der Formel I sind in der folgenden Tabelle I angegeben:

**Tabelle I**

| Beispiel Nr. | EC50 PPARalpha [nM] |
|---|---|
| 4 | 25 |
| 8a | 1,8 |
| 9 | 4,3 |
| 16 | 4,0 |
| 22 | 0,07 |
| 24 | 0,3 |
| 33 | 17 |
| 38 | 8,5 |
| 42 | 79 |
| 51 | 12 |
| 64 | 0,4 |
| 74 | 0,4 |

Aus der Tabelle I ist ersichtlich, dass die erfindungsgemäßen Verbindungen der Formel I den PPARalpha-Rezeptor aktivieren und damit zum Beispiel analog zu klinisch verwendeten Fibraten im Organismus eine Triglyceridsenkung bewirken (siehe z.B. J.-Ch. Fruchard et al.,: PPARS, Metabolic Disease and Atherosclerosis, Pharmacological Research, Vol. 44, No. 5, 2001; S. Kersten et al.: Roles of PPARs in health and disease, NATURE, VOL 405 ,25 MAY 2000 ;I. Pineda et al.: Peroxisome proliferator-activated receptors: from transcriptional control to clinical practice,Curr Opin Lipidol 12: 2001, 245-254).

### Bestimmung von EC50-Werten von PPAR-Agonisten im zellulären PPARgamma - Test

### Prinzip

Zur Bestimmung der zellulären PPARgamma Aktivität von PPAR-Agonisten wird ein transientes Transfektionssystem eingesetzt. Es basiert auf der Verwendung eines Luziferase-Reporterplasmides (pGL3basic-5xGAL4-TK) und eines PPARgamma-Expressionsplasmides (pcDNA3-GAL4-humanPPARgammaLBD). Beide Plasmide werden vorübergehend (= transient) in humane embryonale Nierenzellen (HEK-Zellen) transfiziert. In diesen Zellen wird dann das Fusionsprotein GAL4-humanPPARgammaLBD exprimiert, das an die GAL4-Bindungsstellen des Reporterplasmides bindet. In Anwesenheit eines PPARgamma-aktiven Liganden wird durch das aktivierte Fusionsprotein GAL4-humanPPARgammaLBD die Expression des Luziferase-Reportergens induziert, was sich nach Zugabe eines Luziferasesubtrates in Form eines Chemilumineszenzsignals nachweisen lässt. Im Unterschied zur stabil transfizierten PPARalpha-Reporterzellinie werden beim zellulären PPARgamma-Test die beiden Komponenten (Luziferase-Reporterplasmid und PPARgamma-Expressionsplasmid) transient in HEK-Zellen transfiziert, weil die stabile und permanente Expression des PPARgamma-Fusionsproteins zelltoxisch ist.

### Konstruktion der Plasmide

Das Luziferase-Reporterplasmid pGL3basic-5xGAL4-TK basiert auf dem Vektor pGL3basic der Firma Promega. Zur Herstellung des Reporterplasmides wurden fünf Bindungsstellen des Hefe-Transkriptionsfaktors GAL4 (jede Bindungsstelle mit der Sequenz 5'-CTCGGAGGACAGTACTCCG-3'), 5'-oberhalb zusammen mit einem 160 bp langen Thymidinkinase-Promotorabschnitt (Genbank-Accession # AF027128) in pGL3basic einkloniert. 3'-unterhalb des Thymidinkinasepromotors liegt das gesamte Luziferasegen von Photinus pyralis (Genbank-Accession # M15077), welches bereits Bestandteil des verwendeten Plasmides pGL3basic ist. Die Klonierung und Sequenzierung des Reporterplasmides pGL3basic-SxGAL4-TK erfolgte analog wie bei Sambrook J. et. al. beschrieben (Molecular cloning, Cold Spring Harbor Laboratory Press, 1989).
Zur Herstellung des PPARgamma-Expressionsplasmides pcDNA3-GAL4-humanPPARgammaLBD wurde in das Plasmid pcDNA3 (Firma Invitrogen) 3'-unterhalb des Cytomegalovirus-Promotors zunächst die für die Aminosäuren 1-147 des Hefetranskriptionsfaktors GAL4 (Genbank-Accession # P04386) kodierende cDNA einkloniert. 3'-unterhalb der GAL4-DNA-Bindungsdomäne wurde anschließend die cDNA der Ligandenbindungsdomäne (LBD) des humanen PPARgamma-Rezeptors kloniert (Aminosäuren 1152-Y475; Accession # g1480099). Klonierung und Sequenzierung des PPARgamma-Expressionsplasmides pcDNA3-GAL4-humanPPARgammaLBD erfolgten wiederum analog wie bei Sambrook J. et. al. beschrieben (Molecular cloning, Cold Spring Harbor Laboratory Press, 1989). Neben dem Luziferase-Reporterplasmid pGL3basic-5xGAL4-TK und dem PPARgamma-Expressionsplasmid pcDNA3-GAL4-humanPPARgammaLBD werden für den zellulären PPARgamma-Test noch das Referenzplasmid pRL-CMV (Firma Promega), sowie das Plasmid pBluescript-SK(+) der Firma Stratagene verwendet. Alle vier Plasmide wurden vor der Transfektion in HEK-Zellen mit einem Plasmidpräparationskit der Firma Qiagen präpariert, der eine möglichst endotoxinfreie Plasmidqualität gewährleistet.

### Durchführung des Tests

Die Aktivität von PPARgamma-Agonisten wird in einem 4-Tagestest bestimmt, der nachfolgend beschrieben ist. Vor der Transfektion werden HEK-Zellen in DMEM-Medium (# 41965-039, Invitrogen) kultiviert, das mit folgenden Zusätzen versetzt ist: 10% FKS (#16000-044, Invitrogen), 1% Penicillin-Streptomycin-Lösung (#15140-122, Invitrogen) und 2 mM L-Glutamin (#25030-024, Invitrogen).

### Tag 1

Zunächst wird Lösung A hergestellt, ein Transfektionsgemisch, das neben DMEM-Medium alle vier bereits beschriebenen Plasmide enthält. Für einen Test werden pro 96-well Mikrotiterplatte folgende Mengen zum Ansetzen von 3 ml Lösung A verwendet: 2622 µl antibiotika- und serumfreies DMEM-Medium (# 41965-039, Invitrogen), 100 µl Referenzplasmid pRL-CMV (1 ng/µl), 100 µl Luziferase-Reporterplasmid pGL3basic-5xGAL4-TK (10 ng/µl), 100 µl PPARgamma-Expressionsplasmid pcDNA3-GAL4-humanPPARgammaLBD (100 ng/µl) und 78 µl Plasmid pBluescript-SK(+) (500 ng/µl). Danach werden pro 96-well Mikrotiterplatte 2 ml Lösung B durch Mischen von 1,9 ml DMEM-Medium (# 41965-039, Invitrogen) mit 100 µl PolyFect-Transfektionsreagens (Firma Qiagen) hergestellt. Anschließend werden 3 ml Lösung A mit 2 ml, Lösung B zu 5 ml Lösung C versetzt, durch mehrfaches Pipettieren gründlich gemischt und für 10 min bei Raumtemperatur inkubiert.
80% konfluente HEK-Zellen aus einer 175 cm² großen Zellkulturflasche werden einmal mit 15 ml PBS gewaschen (#14190-094, Invitrogen) und mit 3 ml Trypsinlösung (#25300-054, Invitrogen) für 2 min bei 37°C behandelt. Danach werden die Zellen in 15 ml DMEM-Medium (# 41965-039, Invitrogen) aufgenommen, weiches mit 10% FKS (# 16000-044, Invitrogen); 1 % Penicillin-Streptomycin-Lösung (#15140-122, Invitrogen) und 2 mM L-Glutamin (#25030-024, Invitrogen) versetzt ist. Nach dem Zählen der Zellsuspension im Zellzählgerät wird die Suspension auf 250.000 Zellen/ml verdünnt. Für 1 Mikrotiterplatte werden 15 ml dieser Zellsuspension mit 5 ml der Lösung C vermengt. Pro well einer 96 well-Mikrotiterplatte mit klarem Plastikboden (#3610, Corning Costar) werden 200 µl der Suspension ausgesät. Die Platten werden für 24 h in einem Zellkulturbrutschrank bei 37°C und 5 % CO₂ inkubiert.

### Tag 2

Zu testende PPAR-Agonisten werden in einer Konzentration von 10 mM in DMSO gelöst. Diese Stocklösung wird in DMEM-Medium (# 41965-039, Invitrogen) verdünnt, welches mit 2 % Ultroser (#12039-012, Biosepra), 1 % Penicillin-Streptomycin-Lösung (#15140-122, Invitrogen) und 2 mM L-Glutamin (#25030-024, Invitrogen) versetzt ist. Testsubstanzen werden in insgesamt 11 verschiedenen Konzentrationen im Bereich von 10 µM bis 100 pM getestet. Potentere Verbindungen werden in Konzentrationsbereichen von 1 µM bis 10 pM geprüft.
Das Medium der an Tag 1 transfizierten und ausgesäten HEK-Zellen wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Jede Platte wird mit einem Standard PPARgamma-Agonisten belegt, der ebenfalls in 11 verschiedenen Konzentrationen verdünnt wird, um die Funktionsfähigkeit des Tests in jeder Einzelplatte nachzuweisen. Die Testplatten werden für 48 h in einem Brutschrank bei 37 °C und 5 % CO₂ inkubiert.

### Tag 4

Nach dem Absaugen des Mediums werden gemäß den Angaben des Herstellers pro well je 50 µl Dual-Glo^{™} Reagens (Dual-Glo^{™} Luciferase Assay System; Firma Promega) zugegeben, um die Zellen zu lysieren und das Substrat für die in den Zellen gebildete Firefly-Luziferase (Photinus pyralis) zur Verfügung zu stellen. Nach einer 10 minütigen Inkubation im Dunkeln bei Raumtemperatur wird die Firefly-Luziferasevermittelte Chemilumineszenz im Messgerät gemessen (1 sec. Meßzeit/well; Trilux der Firma Wallac). Danach wird pro well je 50 µl des Dual-Glo^{™} Stop & Glo Reagens (Dual-Glo^{™} Luciferase Assay System; Firma Promega) zugegeben, um die Aktivität der Firefly-Luziferase abzustoppen und das Substrat für die vom Referenzplasmid pRL-CMV aus exprimierten Renilla-Luciferase zur Verfügung zu stellen. Nach einer weiteren 10 minütigen Inkubation im Dunkeln bei Raumtemperatur wird erneut für 1 sec/well die durch die Renilla-Luziferase vermittelte Chemilumineszenz im Messgerät gemessen.

### Auswertung

Die Rohdaten des Lumineszenzmeßgerätes werden in ein Microsoft Excel-File transferiert. Für jeden Meßpunkt, der sich von einem well der Mikrotiterplatte ableitet, wird der Quotient Firefly/Renilla-Luciferaseaktivität bestimmt. Aus den Quotienten werden die Dosis-Wirkungskurven und EC50-Werte von PPAR-Agonisten mit dem Program XL.Fit nach Vorgabe des Herstellers (Firma IDBS) berechnet. Mit den in dieser Anmeldung beschriebenen PPAR-Agonisten wurden PPARgamma-EC50-Werte im Bereich von 0,08nM bis >10 µM gemessen.

Die nachfolgend aufgeführten Beispiele dienen zur Erläuterung der Erfindung, ohne diese jedoch einzuschränken.

**Tabelle II:**

| | | | | | | | | | | | |
|---|---|---|---|---|---|---|---|---|---|---|---|
| 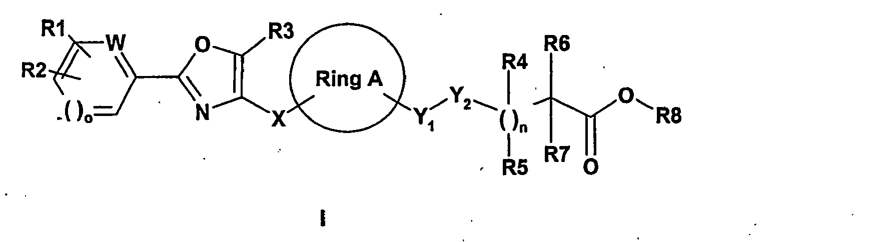 | | | | | | | | | | | |
| Im folgenden sind: Ring A = cis-Cyclohexan-1,3-diyl, R4 = R5 = H und R8 = H. | | | | | | | | | | | |

| **Bsp** | **R1** | **R2** | **R3** | **W** | **X** | **Y1** | **Y2** | **n** | **R6** | **R7** | **R9** |
|---|---|---|---|---|---|---|---|---|---|---|---|
| 1 | p-CH3 | H | CH3 | CH | CH2O | CH2 | O | 0 | H | n-C3H7 | - |
| 2 | p-CH3 | H | CH3 | CH | CH2O | CH2 | O | 0 | H | CH3 | - |
| 3 | p-CH3 | H | CH3 | CH | CH2O | CH2 | O | 0 | H | C2H5 | - |
| 4 | p-CH3 | H | CH3 | CH | CH2O | CH2 | O | 0 | H | PhCH2 | - |
| 5 | m-OCH3 | H | CH3 | CH | CH2O | CH2 | O | 0 | H | CH3 | - |
| 6 | m-CH3 | H | CH3 | CH | CH2O | CH2 | O | 0 | H | CH3 | - |
| 7 | m-CF3 | H | CH3 | CH | CH2O | CH2 | O | 0 | H | CH3 | - |
| 8 | p-CH3 | H | CH3 | CH | CH2O | CH2 | O | 0 | CH3 | CH3 | - |
| 8a | p-CH3 | H | CH3 | CH | CH2O | CH2 | O | 0 | CH3 | CH3 | - |
| 9 | p-CH3 | H | CH3 | CH | CH2O | CH2 | O . | 0 | Cyclopentyl | | - |
| 10 | p-F | H | CH3 | CH | CH2O | CH2 | O | 0 | CH3 | CH3 | - |
| 11 | m-OCH3 | H | CH3 | CH | CH2O | CH2 | O | 0 | CH3 | CH3 | - |
| 12 | m-CF3 | H | CH3 | CH | CH2O | CH2 | O | 0 | CH3 | CH3 | - |
| 13 | 5-CH3 | H | CH3 | O | CH2O | CH2 | O | 0 | CH3 | CH3 | - |
| 14 | p-OCH3 | m-OCH3 | CH3 | CH | CH2O | CH2 | O | 0 | CH3 | CH3 | - |
| 15 | p-CH3 | H | Ph | CH | CH2O | CH2 | O | 0 | CH3 | CH3 | - |
| 16 | p-CF3 | H | CH3 | CH | CH2O | CH2 | O | 0 | CH3 | CH3 | - |
| 17 | p-OCH3 | H | CH3 | CH | CH2O | CH2 | O | 0 | CH3 | CH3 | - |
| 18 | H | H | CH3 | S | CH2O | CH2 | O | 0 | CH3 | CH3 | - |
| 19 | m-OCF3 | H | CH3 | CH | CH2O | CH2 | O | 0 | CH3 | CH3 | - |
| 20 | p-i-C3H7 | H | CH3 | CH | CH2O | CH2 | O | 0 | CH3 | CH3 | - |
| 21 | m-CH3 | H | CH3 | CH | CH2O | CH2 | O | 0 | CH3 | CH3 | - |
| 22 | p-CH3 | H | CH3 | CH | CH2O | CH2 | S | 0 | H | H | - |
| 23 | p-CH3 | H | CH3 | CH | CH2O | CH2 | S | 0 | H | CH3 | - |
| 24 | p-CH3 | H | CH3 | CH | CH2O | CH2 | S | 0 | H | C2H5 | - |
| 25 | p-CH3 | H | CH3 | CH | CH2O | CH2 | S | 0 | H | n-C5H11 | - |
| 26 | p-CH3 | H | CH3 | CH | CH2O | CH2 | S | 0 | H | i-C3H7 | - |
| 27 | p-CH3 | H | CH3 | CH | CH2O | CH2 | S | 0 | CH3 | CH3 | - |
| 28 | p-CH3 | H | CH3 | CH | CH2O | CH2 | S | 0 | H | Ph | - |
| 29 | p-CH3 | H | CH3 | CH | CH2O | CH2 | S | 0 | H | cycl-C6H11 | - |
| 30 | p-CH3 | H | CH3 | CH | CH2O | CH2 | S | 0 | H | n-C3H7 | - |
| 31 | p-CH3 | H | CH3 | CH | CH2O | CH2 | S | 0 | Cyclobutyl | | - |
| 32 | p-CH3 | H | CH3 | CH | CH2O | CH2 | SO | 0 | H | H | - |
| 33 | p-CH3 | H | CH3 | CH | CH2O | CH2 | SO | 0 | H | n-C5H11 | - |
| 34 | p-CH3 | H | CH3 | CH | CH2O | CH2 | SO | 0 | CH3 | CH3 | - |
| 35 | p-CH3 | H | CH3 | CH | CH2O | CH2 | SO | 0 | H | i-C3H7 | - |
| 36 | p-CH3 | H | CH3 | CH | CH2O | CH2 | SO | 0 | H | n-C3H7 | - |
| 37 | p-CH3 | H | CH3 | CH | CH2O | CH2 | SO2 | 0 | H | H | - |
| 38 | p-CH3 | H | CH3 | CH | CH2O | CH2 | SO2 | 0 | H | n-C5H11 | - |
| 39 | p-CH3 | H | CH3 | CH | CH2O | CH2 | SO2 | 0 | CH3 | CH3 | - |
| 40 | p-CH3 | H | CH3 | CH | CH2O | CH2 | SO2 | 0 | H | i-C3H7 | - |
| 41 | p-CH3 | H | CH3 | CH | CH2O | CH2 | SO2 | 0 | H | n-C3H7 | - |
| 42 | p-CH3 | H | CH3 | CH | CH2O | CH2 | NH | 0 | H | (S)-i-C3H7 | - |
| 43 | p-CH3 | H | CH3 | CH | CH2O | CH2 | NR9 | 0 | H | (S)-i-C3H7 | COCH3 |
| 44 | p-CH3 | H | CH3 | CH | CH2O | CH2 | NR9 | 0 | H | (S)-i-C3H7 | COPh |
| 45 | p-CH3 | H | CH3 | CH | CH2O | CH2 | NR9 | 0 | H | (S)-i-C3H7 | SO2CH3 |
| 46 | p-CH3 | H | CH3 | CH | CH2O | CH2 | NR9 | 0 | H | (S)-i-C3H7 | SO2CH2-SO2CH3 |
| 47 | p-CH3 | H | CH3 | CH | CH2O | CH2 | NR9 | 0 | H | (S)-i-C3H7 | SO2(p-Tol) |
| 48 | p-CH3 | H | CH3 | CH | CH2O | CH2 | NR9 | 0 | H | (S)-i-C3H7 | COOMe |
| 49 | p-CH3 | H | CH3 | CH | CH2O | CH2 | NR9 | 0 | H | H | H |
| 50 | p-CH3 | H | CH3 | CH | CH2O | CH2 | NR9 | 0 | H | (S)-i-C3H7 | CH3 |
| 51 | p-CH3 | H | CH3 | CH | CH2O | CH2 | NR9 | 0 | H | (S)-i-C3H7 | CH2Ph |
| 52 | p-CH3 | H | CH3 | CH | CH2O | CH2 | NR9 | 0 | H | H | CH2Ph |
| 53 | p-CH3 | H | CH3 | CH | CH2O | CH2 | NR9 | 0 | H | H | 2- Thienyl-methyl |
| 54 | p-CH3 | H | CH3 | CH | CH2O | CH2 | NR9 | 0 | H | H | CH2(C6H11 ) |
| 55 | p-CH3 | H | CH3 | CH | CH2OCH2 | CH2 | O | 0 | CH3 | CH3 | - |
| 56 | p-CH3 | H | CH3 | CH | CH2OCH2 | CH2 | O | 0 | CH3 | C2H5 | - |
| 57 | p-Ph | H | CH3 | CH | CH2OCH2 | CH2 | O | 0 | CH3 | CH3 | - |
| 58 | H | H | CH3 | CH | CH2OCH2 | CH2 | O | 0 | CH3 | CH3 | - |
| 59 | 2-Naphthyl | | CH3 | CH | CH2OCH2 | CH2OCH2 | O | 0 | CH3 | CH3 | - |
| 60 | m-OMe | H | C2H5 | CH | CH2OCH2 | CH2 | O | 0 | CH3 | CH3 | - |
| 61 | p-i-C3H7 | H | CH3 | CH | CH2OCH2 | CH2 | O | 0 | CH3 | CH3 | - |
| 62 | p-CH3 | H | Cy | CH | CH2OCH2 | CH2 | O | 0 | CH3 | CH3 | - |
| 63 | p-i-C3H7 | H | C2H5 | CH | CH2OCH2 | CH2 | O | 0 | CH3 | CH3 | - |
| 64 | 2-Naphthyl | | C2H5 | CH | CH2OCH2 | CH2OCH2 | O | 0 | CH3 | CH3 | - |
| 65 | p-CH3 | H | C2H5 | CH | CH2OCH2 | CH2 | O | 0 | CH3 | CH3 | - |
| 66 | m-CF3 | H | i-C3H7 | CH | CH2OCH2 | CH2 | O | 0 | CH3 | CH3 | - |
| 67 | m-OCH3 | H | CH3 | CH | CH2O | CH2 | CH2 | 0 | H | H | - |
| 68 | m-OCH3 | H | CH3 | CH | CH2O | CH2 | CH2 | 0 | H | CH3 | - |
| 69 | m-OCH3 | H | CH3 | CH | CH2O | CH2 | CH2 | 0 | H | C2H5 | - |
| 70 | m-OCH3 | H | CH3 | CH | CH2O | CH2 | CH2 | 0 | H | n-C3H7 | - |
| 71 | p-CH3 | H | CH3 | CH | CH2O | CH2 | CH2 | 0 | CH3 | CH3 | - |
| 72 | m-CF3 | H | CH3 | CH | CH2O | CH2 | CH2 | 0 | CH3 | CH3 | - |
| 73 | p-CH3 | H | CH3 | CH | CH2O | CH2 | CH2 | 0 | H | i-C3H7 | - |
| 74 | m-OCH3 | H | CH3 | CH | CH2O | CH2 | CH2 | 0 | H | i-C3H7 | - |
| 75 | p-CH3 | H | CH3 | CH | CH2O | CH2 | CH2 | 0 | H | PhCH2 | - |
| 76 | p-CH3 | H | CH3 | CH | CH2O | CH2 | CH2 | 0 | H | i-C4H9 | - |
| 77 | m-CF3 | H | CH3 | CH | CH2O | CH2 | CH2 | 0 | n-C3H7 | n-C3H7 | - |
| 78 | p-CH3 | H | CH3 | CH | CH2O | CH2 | CH2 | 0 | Cyclopentyl | | - |
| 79 | m-CH3 | H | CH3 | CH | CH2O | C2H4 | NR9 | 0 | H | (S)-i-C3H7 | 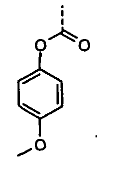 |
| 80 | m-CH3 | H | CH3 | CH | CH2O | C2H4 | NR9 | 0 | Cyclopentyl | | 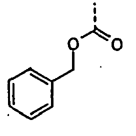 |
| 81 | m-CH3 | H | CH3 | CH | CH2O | C2H4 | NR9 | 0 | Cyclopentyl | | 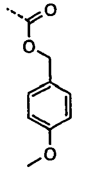 |
| 82 | m-CH3 | H | CH3 | CH | CH2O | C2H4 | NR9 | 0 | (R)-PhCH2 | Me | 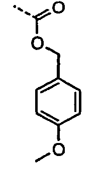 |
| 83 | m-CH3 | H | CH3 | CH | CH2O | C2H4 | NR9 | 0 | H | H | 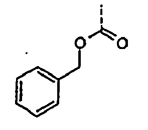 |
| 84 | m-CH3 | H | CH3 | CH | CH2O | C2H4 | NR9 | 0 | H | H | 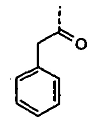 |
| 85 | m-CH3 | H | CH3 | CH | CH2O | C2H4 | NR9 | 0 | H | H | 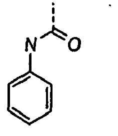 |

| | | | | | | | | | | | |
|---|---|---|---|---|---|---|---|---|---|---|---|
| Die gestrichelte Linie gibt die Verknüpfungsstelle an. | | | | | | | | | | | |

### Verfahren

Die erfindungsgemäßen Verbindungen der Formel I können entsprechend den folgenden Reaktionsschemata erhalten werden:

### Verfahren A

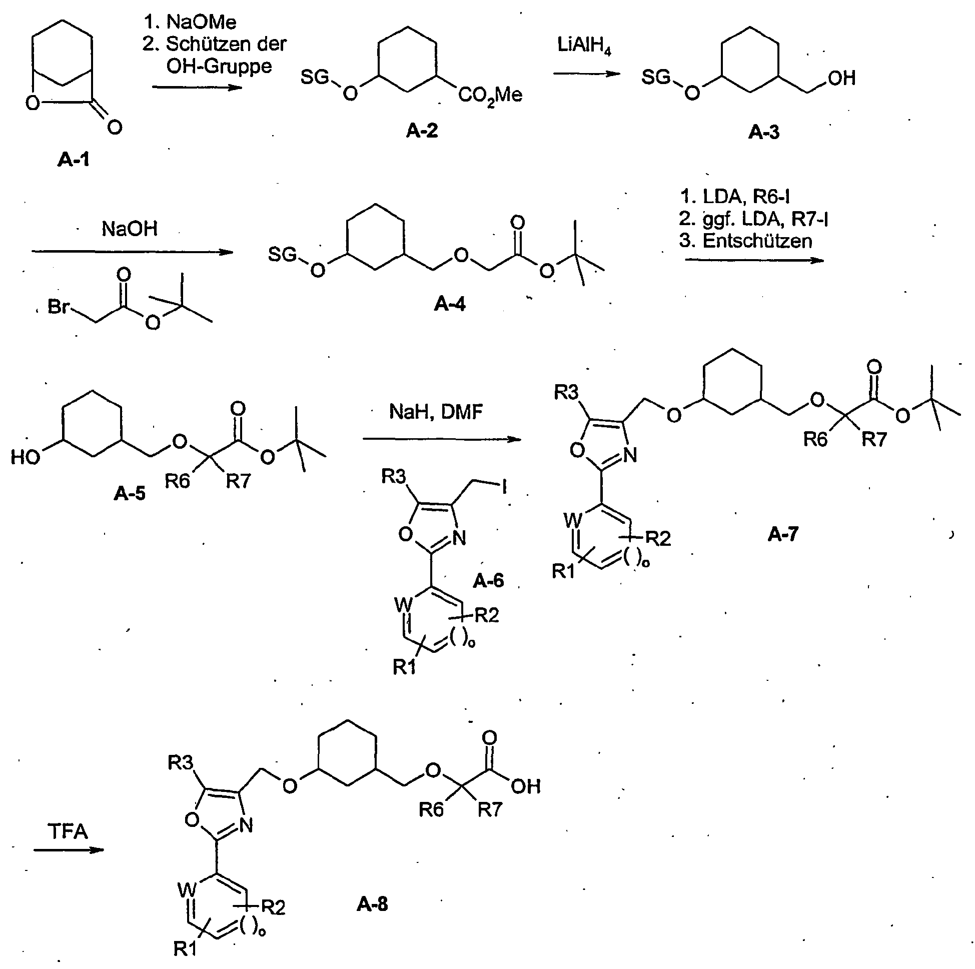

Die Verbindung A-1 wird in einem Alkohol R-8OH umgesetzt. Das so erhaltene Produkt wird an der sekundären Hydroxylgruppe geschützt (z. B. durch Rühren bei RT mit TBDPSCI und Imidazol in DMF), wobei die Verbindung A-2 erhalten wird, worin R8 die oben beschriebene Bedeutung hat. A-2 wird in einem etherischen Lösungsmittel mit Lithiumaluminiumhydrid zur Verbindung A-3 reduziert. Die Verbindung A-3 wird in einem Zweiphasensystem aus Toluol und 50%-iger Natriumhydroxidlösung bei 10 °C mit Bromessigsäure-tert-butylester und Tetrabutylammoniumhydrogensulfat zur Verbindung A-4 umgesetzt.
Die Verbindung A-4 wird in Tetrahydrofuran mit Lithiumdiisopropylamid und einem Alkyliodid der allgemeinen Formel R6-1, worin R6 die oben beschriebene Bedeutung hat, umgesetzt. In einigen Beispielen wird die so erhaltene Verbindung in Tetrahydrofuran mit Lithiumdiisopropylamid und einem weiteren Alkyliodid der allgemeinen Formel R7-1, worin R7 die oben beschriebene Bedeutung hat, umgesetzt. Die Schutzgruppe wird abgespalten, wobei die Verbindung der allgemeinen Formel A-5 erhalten wird.
Die Verbindung A-5 wird in Methyl-tert-bütylether oder Dimethylformamid mit Natriumhydrid und der Verbindung A-6 (siehe Verfahren A), worin R1, R2, R3 und W die oben beschriebenen Bedeutungen haben, zur Verbindung A-7 umgesetzt.
Das Produkt A-7 wird mehrere Stunden in Trifluoressigsäure oder HCl/Dioxan gerührt. Dabei wird die Verbindung der allgemeinen Formel A-8 erhalten.
Nach diesem Verfahren können die Beispiele 31 bis 51 synthetisiert werden.

### Verfahren B:

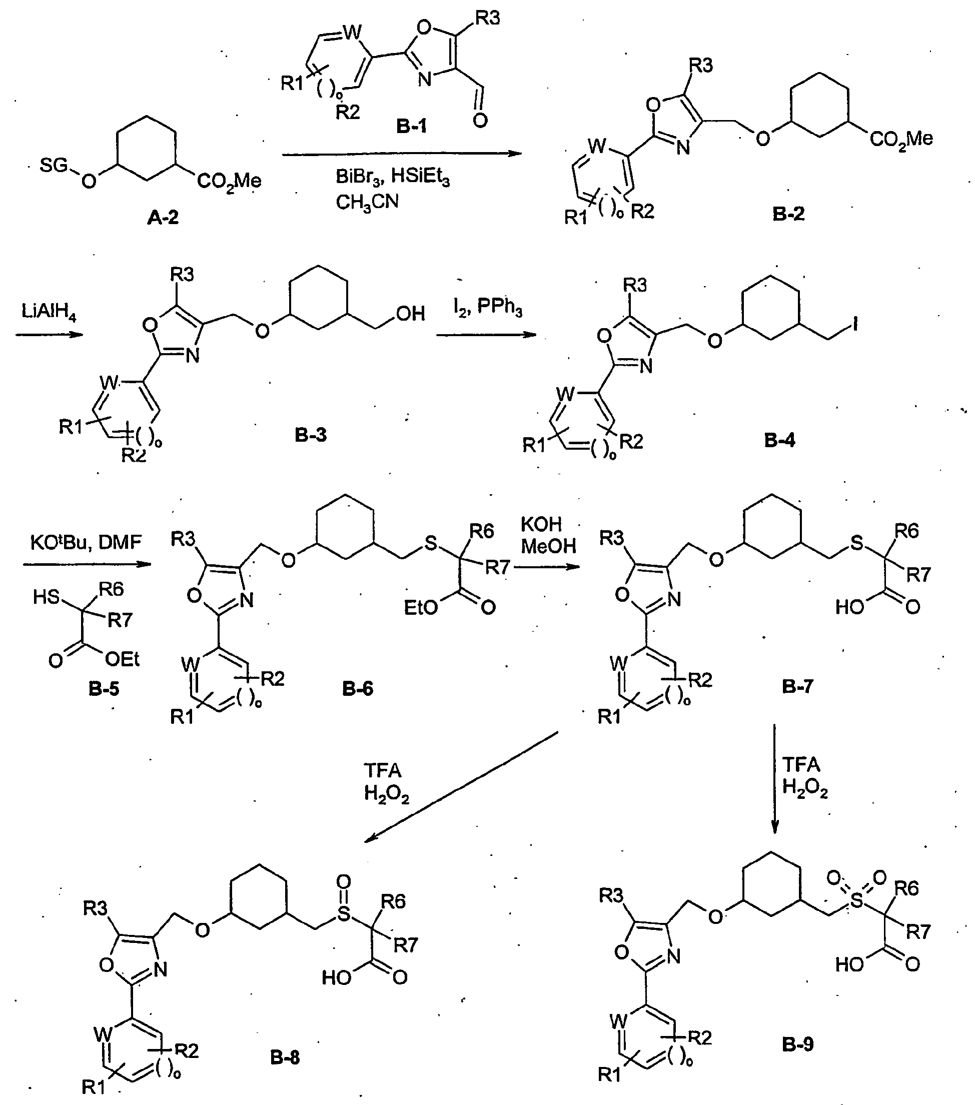

Die Verbindung A-2, wobei SG = tert-Butyldimethylsilyl ist, wird mit Wismuttribromid, Triethylsilan und einer Verbindung der allgemeinen Formel B-1, worin R1, R2, W und R3 die oben beschriebenen Bedeutungen haben, in Acetonitril bei Raumtemperatur zur Verbindung B-2 umgesetzt.
Die Verbindung B-2 wird mit Lithiumaluminiumhydrid in Diethylether oder THF zur Verbindung B-3 reduziert. Die Verbindung B-3 wird mit Triphenylphosphin und Iod in Toluol bei Raumtemperatur zur Verbindung B-4 umgesetzt.
Die Verbindung B-4 wird mit der Verbindung der allgemeinen Formel B-5, wobei R6 und R7 die oben beschriebenen Bedeutungen haben, zur Verbindung B-6 umgesetzt. Der Ester wird gespalten, indem die Verbindung B-6 mehrere Stunden in einer Mischung aus Methanol und konzentrierter Kalilauge oder Lithiumhydroxid in THF/Methanol/Wasser gerührt wird. Dabei wird die Verbindung B-7 erhalten.
In einigen Beispielen wird die Verbindung B-7 mit einem Äquivalent
Wasserstoffperoxid in Trifluoressigsäure bei Raumtemperatur zur Verbindung der allgemeinen Formel B-8, worin R1, R2, R3, R6, W und R7 die oben beschriebenen Bedeutungen haben, oxidiert.
In einigen Beispielen wird die Verbindung B-7 mit drei Äquivalenten
Wasserstoffperoxid in Trifluoressigsäure bei Raumtemperatur zur Verbindung der allgemeinen Formel B-9, worin R1, R2, R3; R6, W und R7 die oben beschriebenen Bedeutungen haben, oxidiert.
Nach diesem Verfahren können die Beispiele 52 bis 71 synthetisiert werden.

### Verfahren C:

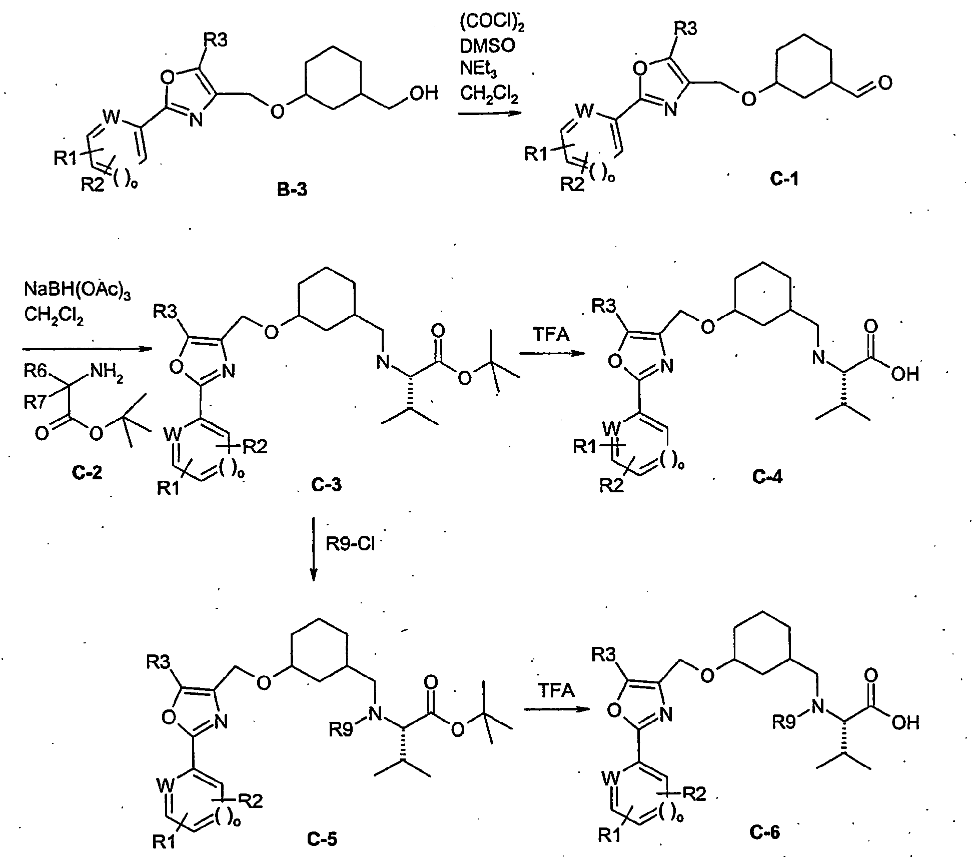

Die Verbindung B-3 (s. Verfahren B) wird mit Oxalylchlorid, Triethylamin und Dimethylsulfoxid in Dichlormethan bei -78°C zum Aldehyd C-1 oxidiert. Dieser wird mit Natriumtriacetoxyborhydrid und der Verbindung der allgemeinen Formel C-2, wobei, R6 und R7 die oben beschriebenen Bedeutungen haben, zur Verbindung C-3 umgesetzt.
Die Verbindung C-3 wird durch mehrstündiges Rühren in Trifluoressigsäure zur Verbindung C-4 umgesetzt.
In einigen Beispielen wird die Verbindung C-3 mit Acylchloriden, Sulfonylchloriden oder Chlorameisensäureestern der allgemeinen Formel R9-Cl, wobei R9 die oben beschriebene Bedeutung hat, in Dichlormethan in Gegenwart von Pyridin zur
Verbindung C-5 umgesetzt. Die Verbindung C-5 wird durch mehrstündiges Rühren in Trifluoressigsäure zur Verbindung C-6 umgesetzt.
Nach diesem Verfahren können die Beispiele 72 bis 78 synthetisiert werden.

### Verfahren D:

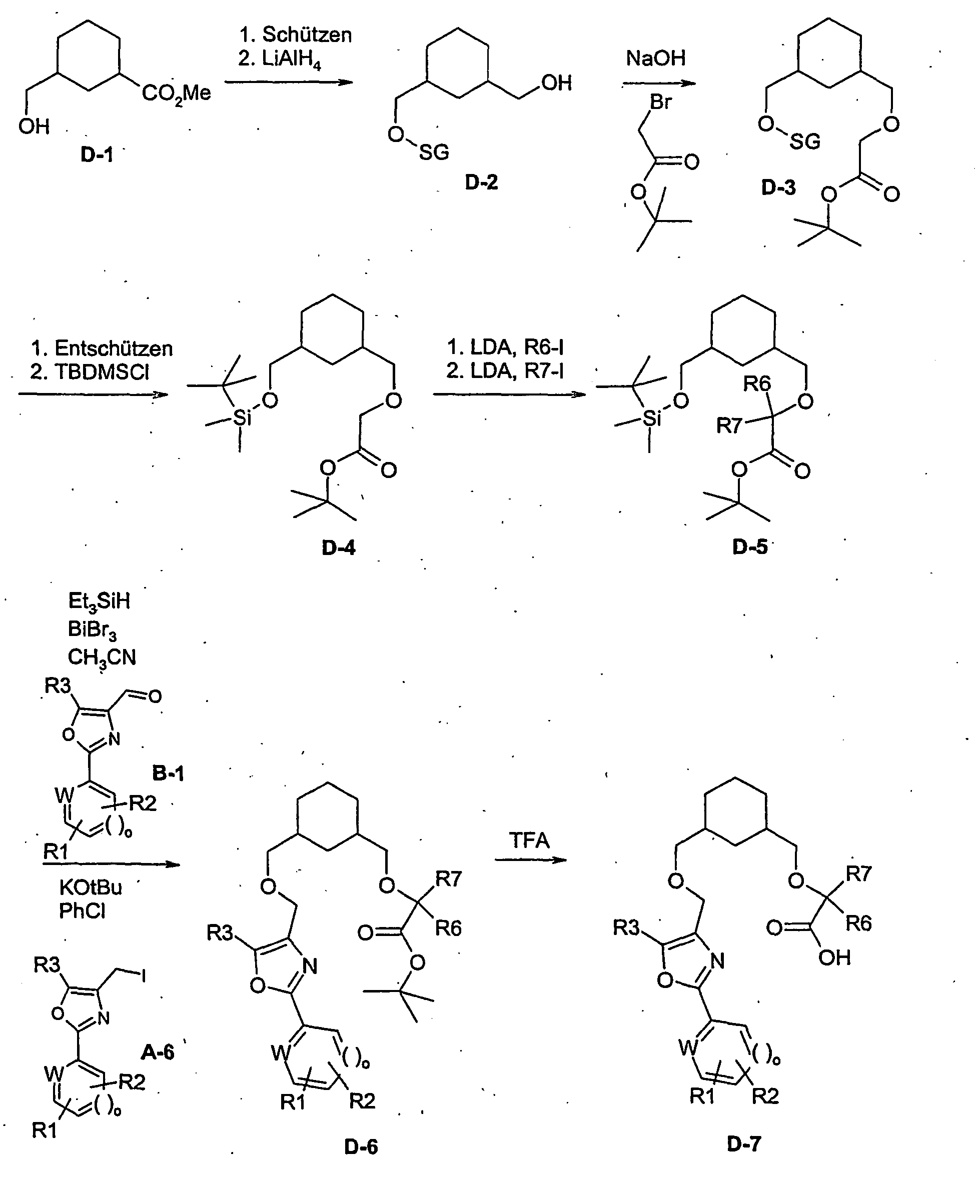

Die Verbindung D-1 wird an der Hydroxylgruppe mit einer geeigneten Schutzgruppe geschützt, beispielsweise mit der Methoxymethylschutzgruppe. Anschließend wird die Carboxylgruppe mit Lithiumaluminiumhydrid in Diethylether zur Verbindung D-2 umgesetzt. Diese wird mit Bromessigsäure-tert-butylester und Tetrabutylammoniumhydrogensulfat in einem Zweiphasensystem Toluol /50%-ige Natronlauge zur Verbindung D-3 umgesetzt.
Die Verbindung D-3 wird entschützt (beispielsweise mit konzentrierter Salzsäure in Tetrahydrofuran im Falle der Methoxymethylschutzgruppe) und anschließend mit tert-Butyldimethylsilylchlorid und Imidazol in Dimethylformamid zur Verbindung D-4 umgesetzt.
Die Verbindung D-4 wird mit Lithiumdiisopropylamid in Tetrahydrofuran bei 0°C deprotoniert und mit einem Alkyliodid der allgemeinen Formel R6-I, wobei R6 die oben beschriebene Bedeutung hat, umgesetzt. Anschließend wird die entstandene Verbindung mit Lithiumdiisopropylamid in Tetrahydrofuran bei 0°C deprotoniert und mit einem Alkyliodid der allgemeinen Formel R7-I, wobei R7 die oben beschriebene Bedeutung hat, zur Verbindung D-5 umgesetzt.
Die Verbindung D-5 wird mit Wismuttribromid, Triethylsilan und der Verbindung B-1 (siehe Verfahren B) in Acetonitril bei Raumtemperatur oder - nach Spaltung der Silylschutzgruppe mit TBAF in THF - mit Kalium-tert-butylat und der Verbindung A-6 (s. Verfahren A) zur Verbindung D-6 umgesetzt.
Die Verbindung D-6 wird durch Rühren in Trifluoressigsäure zur Verbindung D-7 umgesetzt.
Nach diesem Verfahren können die Beispiele 79 und 80 synthetisiert werden.

### Verfahren E:

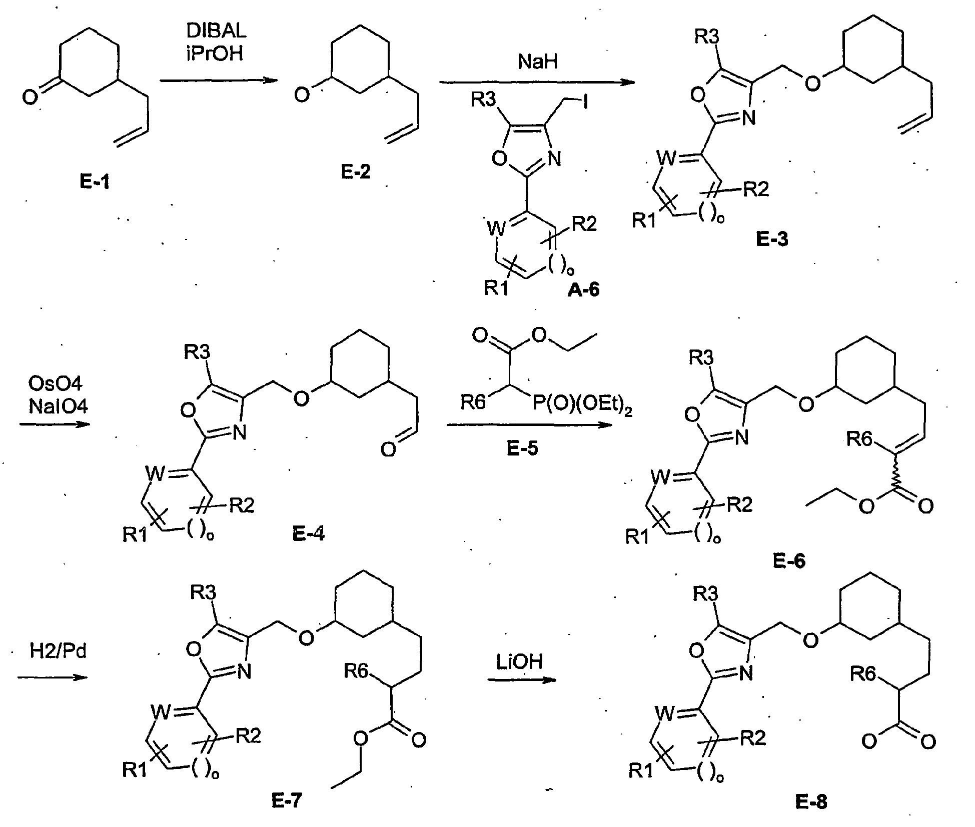

Die Verbindung E-1 wird mit Diisobutylaluminiumhydrid und Isopropanol in Diethylether zur Verbindung E-2 reduziert. Diese wird mit der Verbindung der allgemeinen Formel A-6 und Natriumhydrid in Dimethylformamid zur Verbindung E-3 umgesetzt.
Die Verbindung E-3 wird mit Osmiumtetroxid und Natriumperiodat in Diethylether zum Aldehyd E-4 umgesetzt. Diese Verbindung wird in einer Homer-Emmons-Wadsworth-Reaktion mit einem Triethylphosphonoessigsäureester der allgemeinen Formel E-5, worin R6 die oben beschriebene Bedeutung hat, zur Verbindung E-6 umgesetzt. Die Verbindung E-6 wird mit Wasserstoff an Palladium/Kohle zur Verbindung E-7 hydriert, und anschließend wird der Ester mit Lithiumhydroxid zur Säure E-8 verseift.
Nach diesem Verfahren können die Beispiele 81 bis 84 synthetisiert werden.

### Verfahren F:

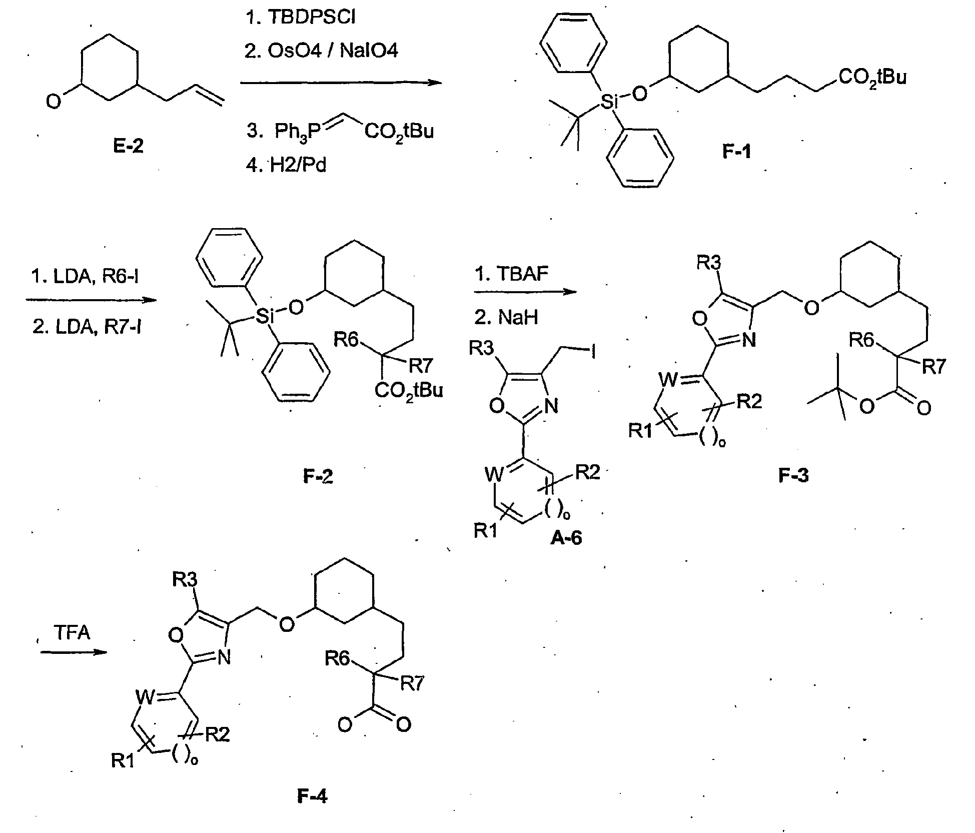

Die Verbindung E-2 wird mit tert-Butyldiphenylsilylchlorid und Imidazol als Base in Dimethylformamid umgesetzt, aufgearbeitet und dann mit Osmiumtetroxid und Natriumperiodat in Diethylether umgesetzt. Die so erhaltene Verbindung wird mit Triphenylphosphoranylidenessigsäure-tert-butylester und nButhyllithium in einer Wittig-Reaktion umgesetzt und anschließend mit Wasserstoff an Palladium/Kohle zur Verbindung F-1 hydriert.
Die Verbindung F-1 wird mit Lithiumdiisopropylamid in Tetrahydrofuran bei 0°C deprotoniert und mit einem Alkyliodid der allgemeinen Formel R6-I, wobei R6 die oben beschriebene Bedeutung hat, umgesetzt. Anschließend wird die entstandene Verbindung mit Lithiumdiisopropylamid in Tetrahydrofuran bei 0°C deprotoniert und mit einem Alkyliodid der allgemeinen Formel R7-I, wobei R7 die oben beschriebene Bedeutung hat, zur Verbindung F-2 umgesetzt.
Die Verbindung F-2 wird zur Entschützung mit Tetrabutylammoniumfluorid in Tetrahydrofuran umgesetzt. Anschließend wird der so erhaltene Alkohol mit Natriumhydrid und der Verbindung A-6 in Dimethylformamid zur Verbindung F-3 umgesetzt.
Der tert-Butylester wird gespalten, indem die Verbindung F-3 in Trifluoressigsäuremehrere Stunden gerührt wird, wobei die Verbindung F-4 erhalten wird.
Nach diesem Verfahren wurden die Beispiele 85 bis 92 synthetisiert.

### Verfahren G:

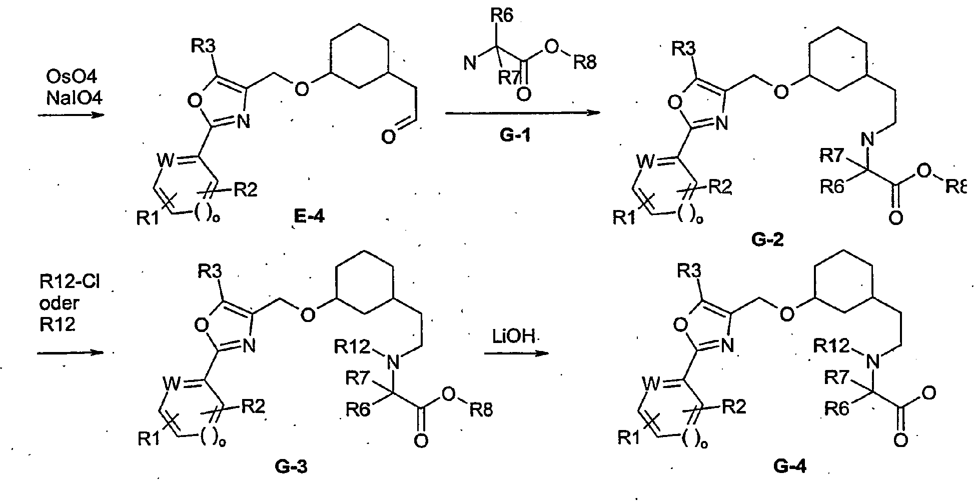

Die Verbindung E-4 wird mit einem Aminosäureester der allgemeinen Formel G-1, worin R6, R7 und R8 die oben angegebene Bedeutung haben, in Gegenwart einer Borhydridreagenzes (z. B. Natriumtriacetoxyborhydrid) zur Verbindung G-2 umgesetzt.

Die Verbidnung G-2 wird mit einem Chlorid R12-Cl, worin R12 die oben angegebene Bedeutung hat, zur Verbindung G-3 umgesetzt (R12 kann auch Isocyanat oder Isothiocyanat sein). Anschließend wird die Verbindung G-3 mit LiOH zur Verbindung G-4 verseift.

### Verfahren H:

Dieses Verfahren dient zur Synthese der Bausteine A-6 und B-2, worin R1, R2, W und R3 die oben genannten Bedeutungen haben.

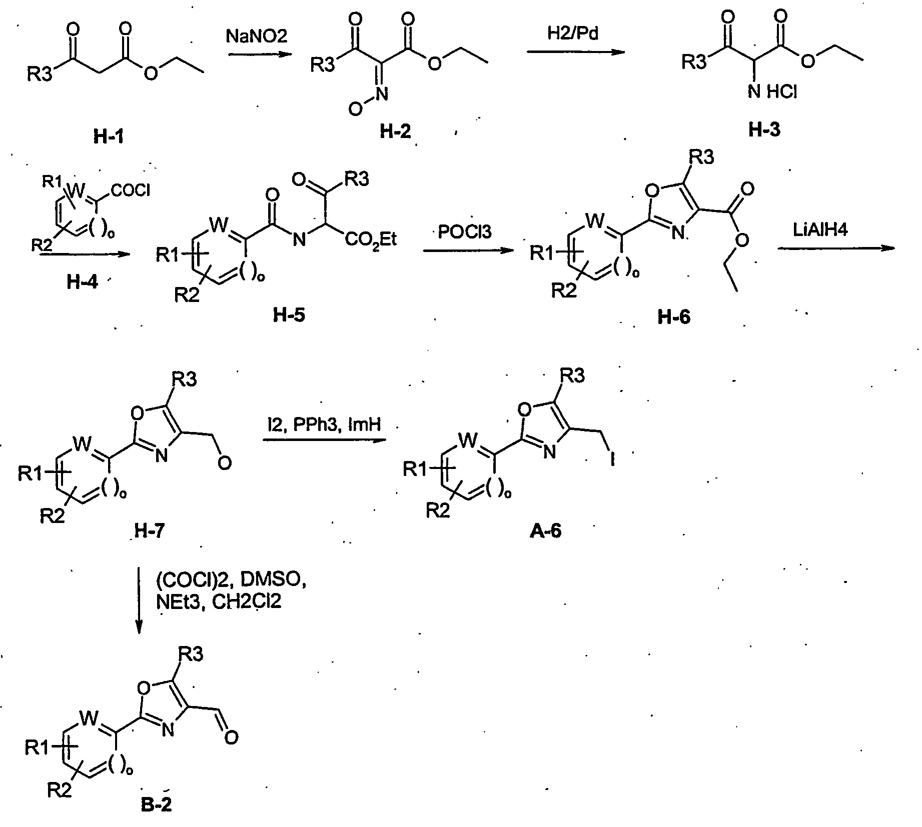

Der Ester H-1, worin R3 die oben genannte Bedeutung hat, wird mit Natriumnitrit und Salzsäure zum Oxim H-2 umgesetzt, welches durch Hydrierung mit Wasserstoff an Palladium/Kohle zum Amin H-3 reduziert wird.

Die Verbindung H-3 wird mit Säurechloriden der allgemeinen Formel H-4, worin R1, W und R2 die oben genannten Bedeutungen haben, und Base (beispielsweise Triethylamin) zur Verbindung H-5 umgesetzt.
Die Verbindung H-5 wird durch Erhitzen in Phosphorylchlorid zur Verbindung H-6 umgesetzt.
Der Ester H-6 wird mit Lithiumaluminiumhydrid in Diethylether zum Alkohol H-7 reduziert. Dieser wird mit Iod, Imidazol (ImH) und Triphenylphosphin in das Iodid A-6 überführt.
Alternativ wird die Verbindung H-7 mit Oxalylchlorid, Dimethylsulfoxid und Triethylamin in Dichlormethan bei -78°C zum Aldehyd B-1 oxidiert.

### Verfahren J:

Dieses Verfahren dient zur Synthese des Bausteins A-6, worin R1, R2, W und R3 die oben genannten Bedeutungen haben.

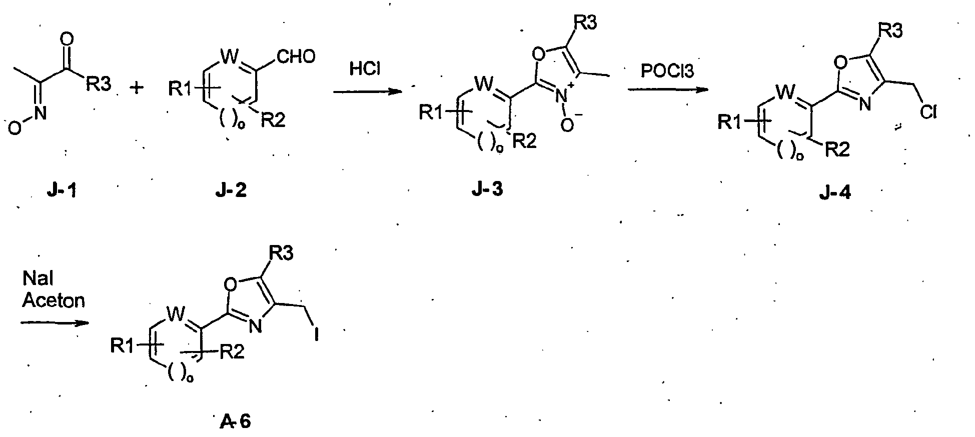

Die Verbindung J-1 wird mit dem Aldehyd J-2, worin R1, R2, W und R3 die oben beschriebenen Bedeutungen haben, in Ethanol mit Chlorwasserstoff zur Verbindung J-3 umgesetzt.
Die Verbindung J-3 wird in Phosphorylchlorid zum Sieden erhitzt, wobei die Verbindung J-4 erhalten wird. Diese wird mit Natriumiodid in Aceton zum Sieden erhitzt. Dabei erhält man die Verbindung A-6.

Die verwendeten Abkürzungen stehen für:
- Ac: Acetyl
- Bn: Benzyl
- iBu: Isobutyl
- tBu: tert-Butyl
- BuLi: n-Butyllithium
- Bz: Benzoyl
- Cy: Cyclohexyl
- DC: Dünnschichtchromatographie
- DCI: direkte chemische Ionisation (bei MS)
- DCM: Dichlormethan
- DMAP: 4-N,N-Dimethylaminopyridin
- DMF: N,N-Dimethylformamid
- DMSO: Dimethylsulfoxid
- EE: Essigsäureethylester
- EDC: N'-(3-Dimethylaminopropyl)-N-ethylcarbodiimid x HCl
- EI: Elektronenstoß-lonisation (bei MS)
- eq: Äquivalent
- ESI: Elektronenspray-lonisation (bei MS)
- Et: Ethyl
- ges.: gesättigt
- h: Stunde
- HATU: O-(7-Azabenzotriazol-1-yl)-N,N,N',N'-tetramethyluronium-Hexafluorophosphat
- HOBt: 1-Hydroxy-1H-benzotriazol x H2O
- HPLC: Hochdruck-, Hochleistungsflüssigchromatographie
- LC-MS: Flüssigchromatographie-gekoppelte Massenspektroskopie
- Me: Methyl
- MS: Massenspektroskopie
- Mscl: Methansulfonylchlorid
- NMR: Kernresonanzspektroskopie
- Pd/C: Palladium auf Kohle
- iPr: Isopropyl
- nPr: n-Propyl
- Rf: Retentionszeit (bei DC)
- RT: Raumtemperatur
- TBAF: Tetrabutylammoniumfluorid
- TBAI: Tetrabutylammoniumiodid
- TBDPSCI: tert.Butyldiphenylsilylchlorid
- TBDMSCI: tert.Butyldimethylsilylchlorid
- THF: Tetrahydrofuran
- Tr: Trityl

Andere Verbindungen können entsprechend den oben genannten Verfahren hergestellt werden.

### Bausteinsynthese nach Verfahren H:

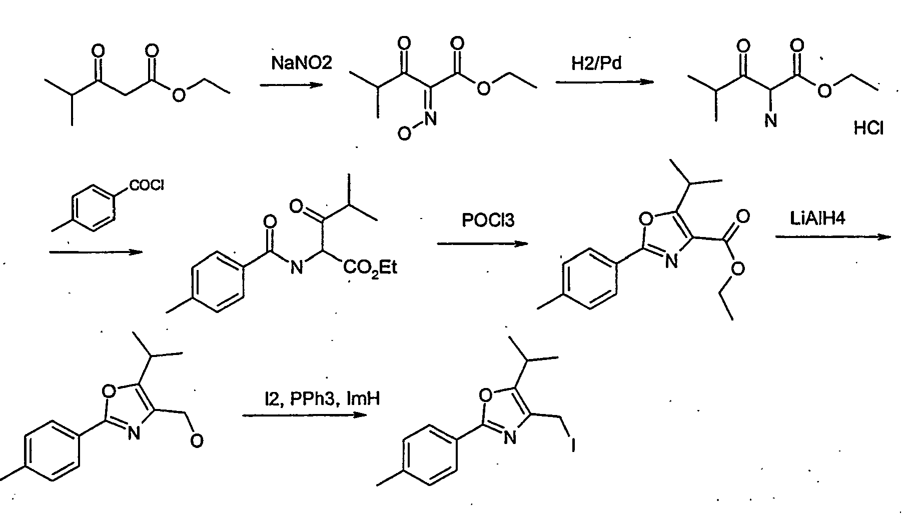

### 2-Hydroxyimino-4-methyl-3-oxo-pentansäureethylester

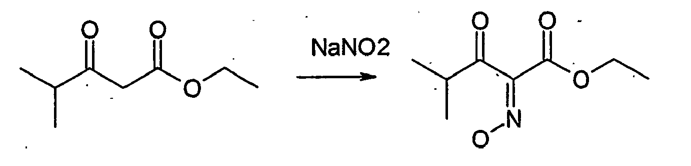

42.4 g 4-Methyl-3-oxo-pentansäureethylester werden in 100 ml Eisessig gelöst und bei 5°C mit 21 g Natriumnitrit, gelöst in 100 ml Wasser, versetzt. Man lässt innerhalb einer Stunde auf Raumtemperatur erwärmen, fügt sodann 100 ml Wasser hinzu und rührt eine weiter Stunde bei Raumtemperatur nach. Man extrahiert dreimal mit je 150 ml Methyl-tert-butylether, die vereinigten organischen Phasen werden mit 200 ml Wasser versetzt und durch Zugabe von festem NaHCO3 neutralisiert. Die organische Phase wird abgetrennt, mit gesättigter NaCl-Lösung gewaschen, über MgSO4 getrocknet und anschließend das Lösungsmittel im Vakuum entfernt. Man erhält 46 g 2-Hydroxyimino-4-methyl-3-oxo-pentansäure-ethylester als Öl. C8H13NO4 (187.20), MS(ESI) = 188 (M+H⁺).

### 2-Amino-4-methyl-3-oxo-pentansäureethylesterrhydrochlorid

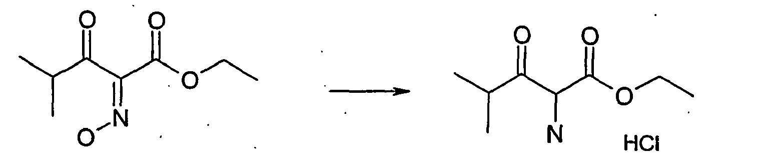

In 200 ml Ethanol werden 10 g HCl eingeleitet. 46 g 2-Hydroxyimino-4-methyl-3-oxo-pentansäureethylester werden darin gelöst und mit 5 g Pd(10% auf Kohle) versetzt und 8 Stunden unter einer Wasserstoffatmosphäre (5 bar) gerührt. Das Reaktionsgemisch wird über Celite filtriert und das Lösungsmittel im Vakuum entfernt. Man erhält 45 g 2-Amino-4-methyl-3-oxo-pentansäureethylester hydrochlorid als weißen Feststoff. C8H15NO3*HCl (209.5), MS(ESI) = 188 (M+H⁺).

### 4-Methyl-2-(4-methyl-benzoylamino)-3-oxo-pentansäureethylester

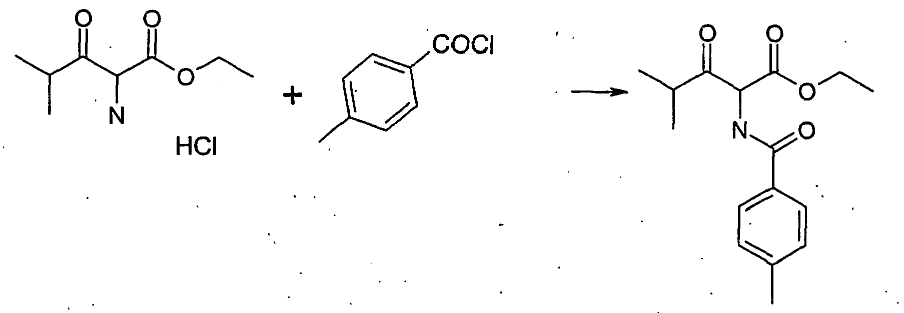

10 g 2-Amino-4-methyl-3-oxo-pentansäureethylesterhydrochlorid und 7.4 g 4-Methyl-benzoylchlorid werden in 250 ml Dichlormethan gelöst und bei 0°C langsam und tropfenweise mit 13.3 ml Triethylamin versetzt. Man rührt eine Stunde bei Raumtemperatur nach, dann wird mit Wasser gewaschen, die organische Phase abgetrennt, über MgSO4 getrocknet und anschließend das Lösungsmittel im Vakuum entfernt. Man erhält 13 g 4-Methyl-2-(4-methyl-benzoylamino)-3-oxo-pentansäureethylester als Öl. C16H21 N04 (291.35), MS(ESI) = 292 (M+H⁺).

### 5-Isopropyl-2-p-tolyl-oxazol-4-carbonsäureethylester

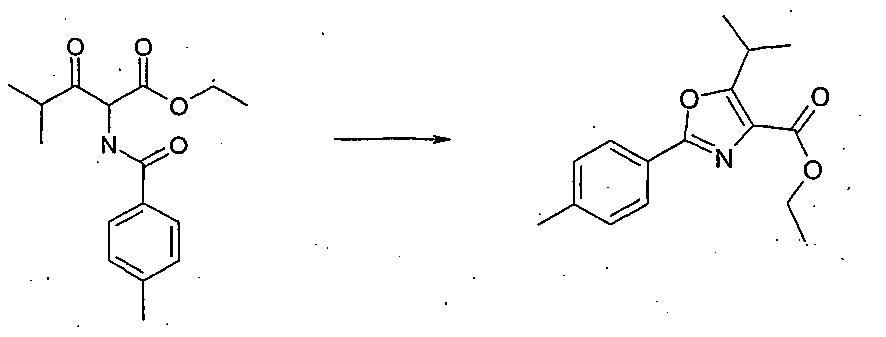

13 g 4-Methyl-2-(4-methyl-benzoylamino)-3-oxo-pentansäureethylester werden in 80 ml Phosphoroxychlorid 2h unter Rückfluss zum Sieden erhitzt. Das Phosphoroxychlorid wird im Vakuum entfernt und der resultierende Rückstand in 200 ml Dichlormethan gelöst , dreimal mit gesättigter NaHCO₃-Lösung gewaschen, über MgSO4 getrocknet und anschließend das Lösungsmittel im Vakuum entfernt. Man erhält 11 g 5-lsopropyl-2-p-tolyl-oxazol-4-carbonsäureethylester als bräunlichen Feststoff.C16H19NO3 (273.33), MS(ESI) = 292 (M+H⁺), Rf(n-Heptan:Ethylacetat) = 2:1) = 0.43.

### (5-Isopropyl-2-p-tolyl-oxazol-4-yl)-methanol

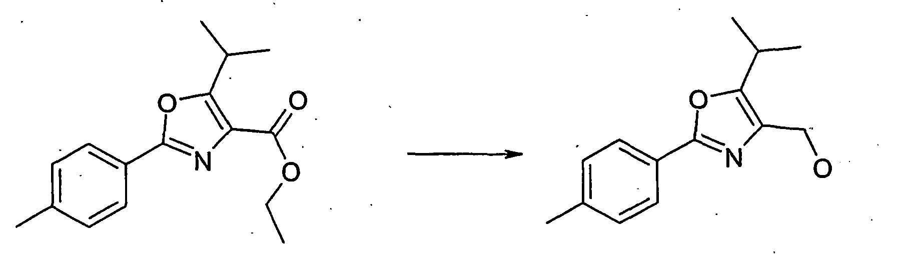

11g 5-Isopropyl-2-p-tolyl-oxazol-4-carbonsäureethylester werden in 100 ml Tetrahydrofuran gelöst und bei 0°C mit 40 ml einer 1 molaren Lösung von Lithiumaluminiumhydrid in Tetrahydrofuran versetzt. Nach 30 min wird das Reaktionsgemisch mit 50 ml 1 N HCl versetzt und fünfmal mit Ethylacetat extrahiert. Die vereinigten organischen Phasen werden über MgSO4 getrocknet und anschließend das Lösungsmittel im Vakuum entfernt. Der Rückstand wird an Kieselgel mit dem Laufmittel n-Heptan:Ethylacetat = 6:1 => 1:1 gereinigt. Man erhält 4.3 g (5-Isopropyl-2-p-tolyl-oxazol-4-yl)-methanol als hellgelben Feststoff. C14H17NO2 (231.30), MS(ESI) = 232 (M+H⁺), Rf(n-Heptan:Ethylacetat) = 1:1) = 0.17.

### 4-Iodmethyl-5-isopropyl-2-p-tolyloxazol

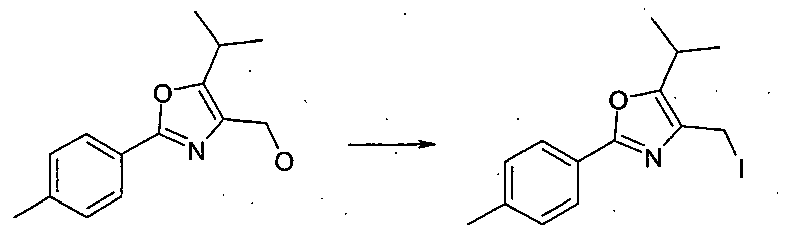

500 mg (5-Isopropyl-2-p-tolyl-oxazol-4-yl)-methanol werden zusammen mit 690 mg Triphenylphosphin und 600 mg Imidazol in 20 ml Toluol gelöst. Man gibt 715 mg Iod hinzu und rührt 1 Stunde bei Raumtemperatur nach. Dann wird 10 ml gesättigte Natriumcarbonat-Lösung und 500 mg Iod nachgegeben. Nach 10 Minuten wird die organische Phase abgetrennt und zweimal mit gesättigter Na2S2O3-Lösung gewaschen, über MgSO4 getrocknet und anschließend die lösungsmittel im Vakuum entfernt. Der Rückstand wird an Kieselgel mit dem Laufmittel n-Heptan:Ethylacetat = 10:1 gereinigt. Man erhält 400 mg 4-lodmethyl-5-isopropyl-2-p-tolyl-oxazol als weißen Feststoff. C14H161NO (341.19), MS(ESI): 342 (M+H⁺), Rf(n-Heptan:Ethylacetat = 1:1) = 0.75.

Analog zur Bausteinsynthese nach Verfahren K wurde aus 2-Amino-4-methyl-3-oxo-pentansäureethylester hydrochlorid und 3-Methoxy-benzoylchlorid 4-lodmethyl-2-(3-methoxy-phenyl)-5-isopropyl-oxazol erhalten. 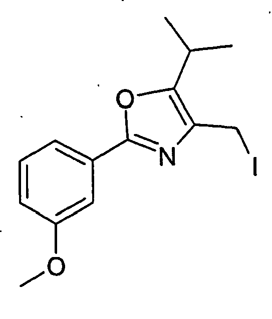 C14H161NO2 (357.19), MS(ESI): 358 (M+H⁺), Rf(n-Heptan:Ethylacetat = 1:1) = 0.60.

Analog zur Bausteinsynthese von 4-Iodmethyl-5-isopropyl-2-p-tolyl-oxazol wurde aus 4,4,4-Trifluoro-3-oxo-buttersäureethylester und 3-Methoxy-benzoylchlorid 4-lodmethyl-2-(3-methoxy-phenyl)-5-trifluormethyl-oxazol erhalten. 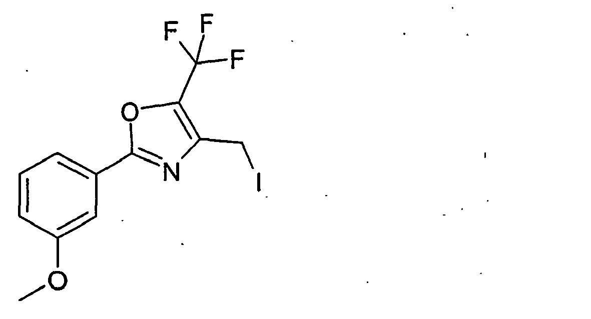 C12H9F3INO2 (383.11), MS(ESI): 384 (M+h⁺).

Analog zur Bausteinsynthese von 4-Iodmethyl-5-isopropyl-2-p-tolyl-oxazol wurde aus 4,4,4-Trifluoro-3-oxo-buttersäureethylester und 3-Trifluormethylbenzoylchlorid 4-lodmethyl-2-(3-trifluormethyl -phenyl)-5-trifluormethyl-oxazol erhalten. 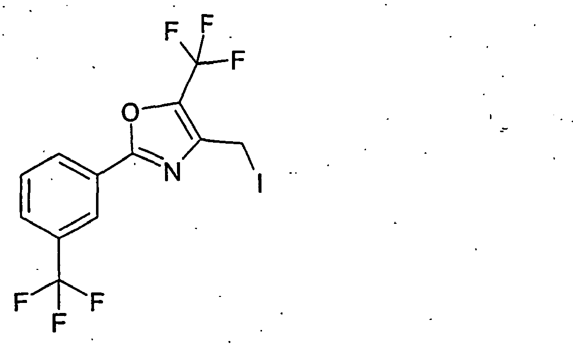 C12H6F6INO (421.08), MS(ESI): 422(M+h⁺).

Analog zur Bausteinsynthese von 4-lodmethyl-5-isopropyl-2-p-tolyl-oxazol wurde aus 4,4,4-Trifluoro-3-oxo-buttersäureethylester und 4-Methyl-benzoylchlorid 4-lodmethyl-5-trifluormethyl-2-p-tolyl-oxazol erhalten. 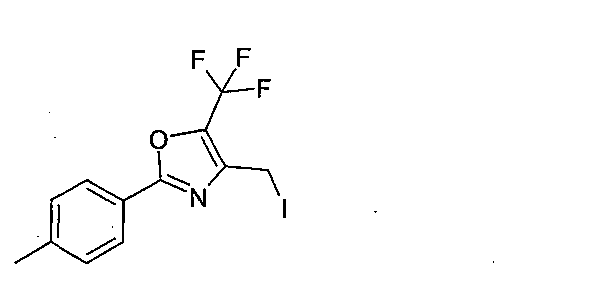 C12H9F3INO (367.11), MS(ESI): 368 (M+h⁺).

**Bausteinsynthese nach Verfahren J:**

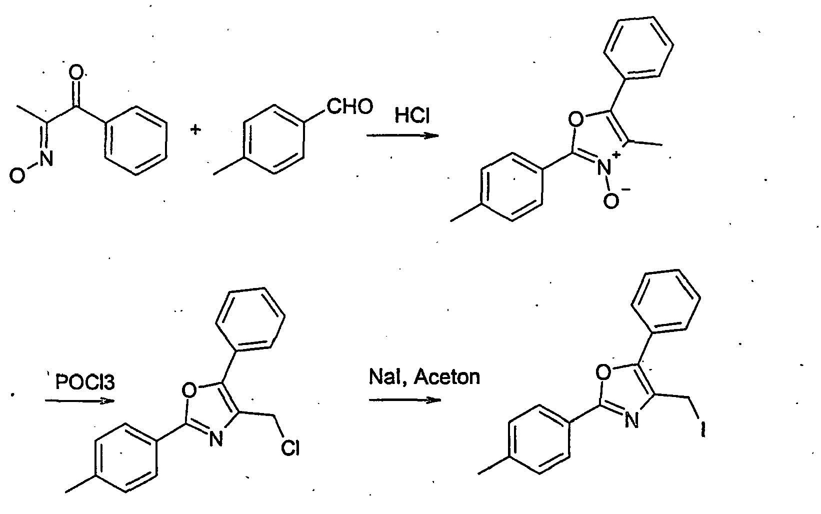

### 4-Methyl-5-phenyl-2-p-tolyl-oxazol 3-oxid

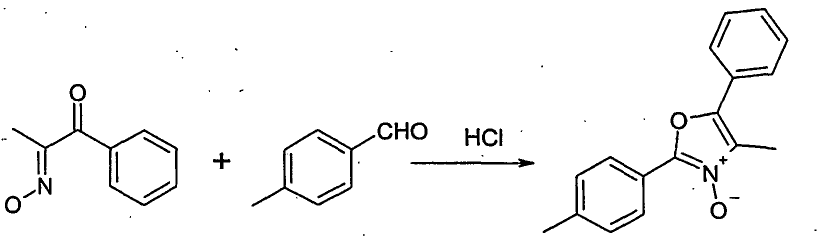

12.5 g 1-Phenyl-1,2-propandion-2-oxim und 10ml p-Toluolaldehyd werden in 50 ml Eisessig gegeben und 30 Minuten unter Eiskühlung HCl Gas durchgeleitet. Durch Zugabe von Methyl-tert-butylether wird das Produkt als Hydrochlorid ausgefällt, abgesaugt und der Niederschlag mit Methyl-tert-butylether gewaschen. Man suspendiert den Niederschlag in Wasser und stellt mit Ammoniak einen basischen pH-Wert ein. Es wird dreimal mit je 200 ml Dichlormethan extrahiert, die vereinigten organischen Phasen werden über MgSO4 getrocknet und anschließend das Lösungsmittel im Vakuum entfernt. Man erhält 6.4 g 4-Methyl-5-phenyl-2-p-tolyl-oxazol 3-oxid als weißen Feststoff. C17H15NO2 (265.31), MS(ESI) = 266 (M+H⁺).

### 4-Chlomethyl-5-phenyl-2-p-tolyl-oxazol

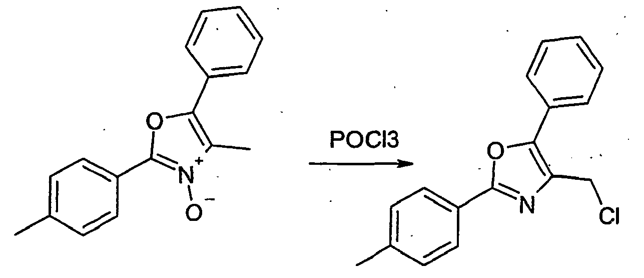

6.4 g 4-Methyl-5-phenyl-2-p-tolyl-oxazol 3-oxid werden in 50 ml Chloroform gelöst, mit 2.4 ml Phosphoroxychlorid versetzt und 30 Minuten unter Rückfluss zum Sieden erhitzt. Das Reaktionsgemisch'wird auf 0°C abgekühlt, mit Ammoniak ein schwach alkalischer pH-Wert eingestellt und dreimal mit je 100 ml Ethylacetat extrahiert. Die vereinigten organischen Phasen werden mit Wasser gewaschen, über MgSO4 getrocknet und anschließend das Lösungsmittel im Vakuum entfernt. Man erhält 5.4 g 4-Chlormethyl-5-phenyl-2-p-tolyl-oxazol als gelben Feststoff. C17H14CINO (283.76), MS(ESI) = 284 (M+H⁺), Rf(n-Heptan:Ethylacetat) = 7:1) = 0.41.

### 4-Iodmethyl-5-phenyl-2-p-tolyl-oxazol

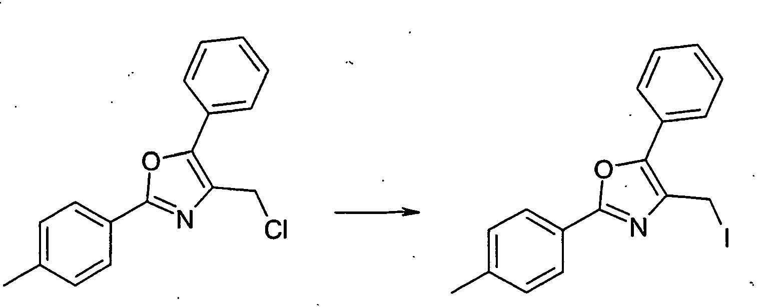

1.8 g 4-Chlormethyl-5-phenyl-2-p-tolyl-oxazol werden zusammen mit 3 g Natriumiodid in 150 ml Aceton 2 Stunden unter Rückfluss zum Sieden erhitzt. Nach dem Abkühlen des Reaktionsgemischs wird 300 ml Methyl-tert-butylether zugefügt, das Gemisch dreimal mit gesättigter Na2S2O3-Lösung gewaschen, über MgSO4 getrocknet und anschließend die Lösungsmittel im Vakuum entfernt: Man erhält 2.7 g 4-lodmethyl-5-phenyl-2-p-tolyl-oxazol als hellgelben Feststoff. C17H141NO (375.21), MS(ESI): 376 (M+H⁺).

Analog zur Bausteinsynthese von 4-Iodmethyl-5-phenyl-2-p-tolyl-oxazol wurde aus 1-Phenyl-1,2-propandion-2-oxim und m-Anisaldehyd 4-lodmethyl-2-(3-methoxy-phenyl)-5-phenyl-oxazol erhalten. 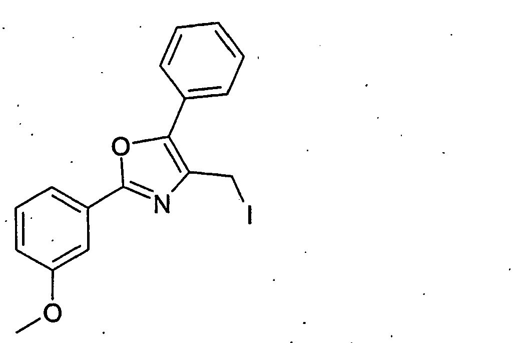 C17H14INO2 (391.21), MS(ESI): 392 (M+h⁺).

Analog zur Bausteinsynthese von 4-lodmethyl-5-phenyl-2-p-tolyl-oxazol wurde aus 1-Ethyl-1,2-propandion-2-oxim und m-Anisaldehyd 4-lodmethyl-5-ethyl-2-(3-methoxyphenyl)-oxazol erhalten. 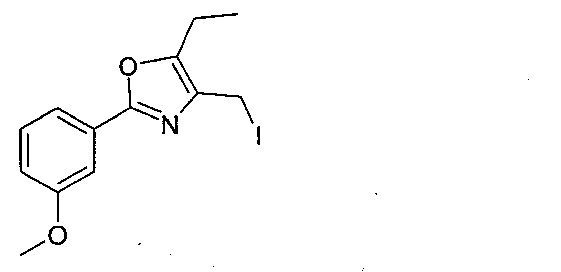 C13H14INO2 (343.17), MS(ESI): 344 (M+h⁺).

Analog zur Bausteinsynthese von 4-lodmethyl-5-phenyl-2-p-tolyl-oxazol wurde aus 1-Ethyl-1,2-propandion-2-oxim und p-Toluolaldehyd 4-lodmethyl-5-ethyl-2-p-tolyl-oxazol erhalten. 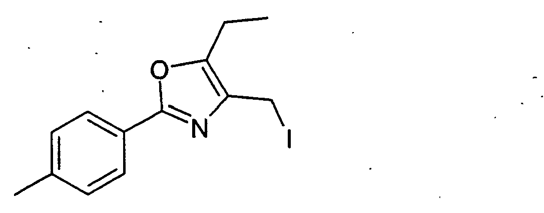 C13H14INO (327.17), MS(ESI): 328 (M+h⁺).

Analog zur Bausteinsynthese von 4-lodmethyl-5-phenyl-2-p-tolyl-oxazol wurde aus 1-Cyclohexyl-1,2-propandion-2-oxim und m-Anisaldehyd 4-lodmethyl-5-cyclohexyl-2-(3-methoxy-phenyl)-oxazol erhalten. 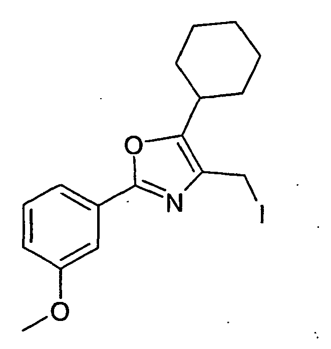 C17H20INO2 (397.26), MS(ESI): 398 (M+h⁺).

Analog zur Bausteinsynthese von 4-lodmethyl-5-phenyl-2-p-tolyl-oxazol wurde aus 1-Cyclohexyl-1,2-propandion-2-oxim und p-Toluolaldehyd 4-lodmethyl-5-cyclohexyl-2-p-tolyl-oxazol erhalten. 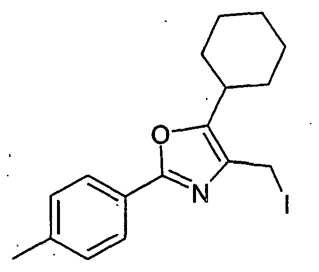 C17H20INO (381.26), MS(ESI): 382 (M+h⁺).

Analog zur Bausteinsynthese von 4-lodmethyl-5-phenyl-2-p-tolyl-oxazol wurde aus Diacetylmonoxim und p-Toluolaldehyd 4-lodmethyl-5-methyl-2-p-tolyl-oxazol erhalten. 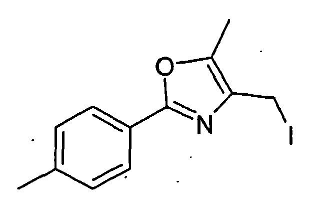 C12H12INO (313.14), MS(ESI): 314 (M+h⁺).

Analog zur Bausteinsynthese von 4-lodmethyl-5-phenyl-2-p-tolyl-oxazol wurde aus Diacetylmonoxim und m-Anisaldehyd 4-lodmethyl-2-(3-methoxy-phenyl)-5-methyl-oxazol erhalten. 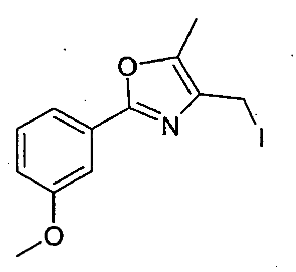 C12H12INO2 (329.14), MS(ESI): 330 (M+h⁺).

Analog zur Bausteinsynthese von 4-lodmethyl-5-phenyl-2-p-tolyl-oxazol wurde aus Diacetylmonoxim und 3-Bromo-benzaldehyd 2-(3-Bromo-phenyl)-4-iodmethyl-5-methyl-oxazol erhalten. 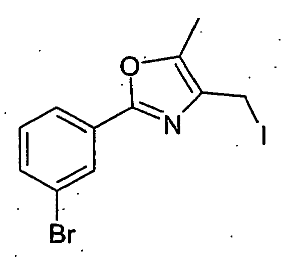 C11H9BrINO (377.01/379.01), MS(ESI): 378/380 (M+h⁺).

Analog zur Bausteinsynthese von 4-lodmethyl-5-phenyl-2-p-tolyl-oxazol wurde aus Diacetylmonoxim und 3-Trifluormethylbenzaldehyd 4-lodmethyl-5-methyl-2-(3-trifluoromethyl-phenyl)-oxazol erhalten. 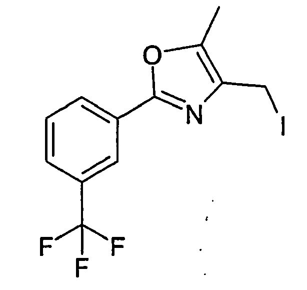 C12H9F3INO (367.11), MS(ESI): 368 (M+h⁺).

Analog zur Bausteinsynthese von 4-lodmethyl-5-phenyl-2-p-tolyl-oxazol wurde aus Diacetylmonoxim und 4-Fluorbenzaldehyd 2-(4-Fluoro-phenyl)-4-iodmethyl-5-methyl-oxazol erhalten. 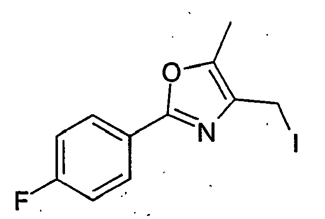 C11H9FINO (317.10), MS(ESI):318 (M+h+).

Analog zur Bausteinsynthese von 4-lodmethyl-5-phenyl-2-p-tolyl-oxazol wurde aus Diacetylmonoxim und 4-Methoxybenzaldehyd 4-lodmethyl-2-(4-methoxy-phenyl)-5-methyl-oxazol erhalten. 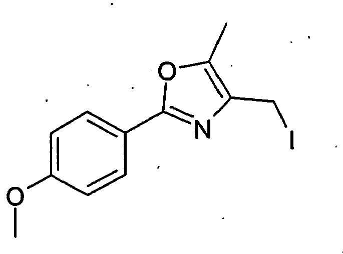 C12H12INO2 (329.14), MS(ESI):330 (M+h+).

Analog zur Bausteinsynthese von 4-lodmethyl-5-phenyl-2-p-tolyl-oxazol wurde aus Diacetylmonoxim und 4-Trifluormethylbenzaldehyd 4-lodmethyl-5-methyl-2-(4-trifluoromethyl-phenyl)-oxazol erhalten. 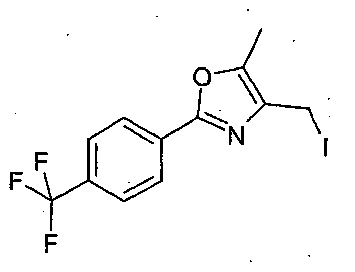 C12H9F3INO (367.11), MS(ESI):368 (M+h+).

Analog zur Bausteinsynthese von 4-lodmethyl-5-phenyl-2-p-tolyl-oxazol wurde aus Diacetylmonoxim und m-Toluolaldehyd 4-lodmethyl-5-methyl-2-m-tolyl-oxazol erhalten. 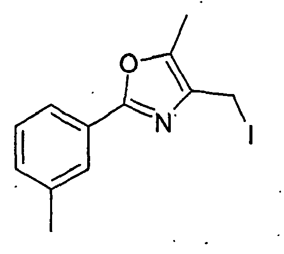 C12H12INO (313.14), MS(ESI):314 (M+h+).

Analog zur Bausteinsynthese von 4-lodmethyl-5-phenyl-2-p-tolyl-oxazol wurde aus Diacetylmonoxim und Benzaldehyd 4-Chlormethyl-5-methyl-2-phenyl-oxazol erhalten. 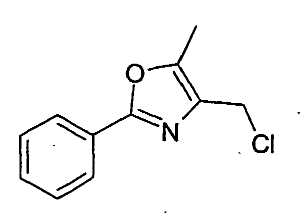 C11H10CINO (299.15), MS(ESI): 300 (M+h+).

Analog zur Bausteinsynthese von 4-lodmethyl-5-phenyl-2-p-tolyl-oxazol wurde aus Diacetylmonoxim und p-Phenylbenzaldehyd 4-lodmethyl-5-methyl-2-p-biphenyl-oxazol erhalten. 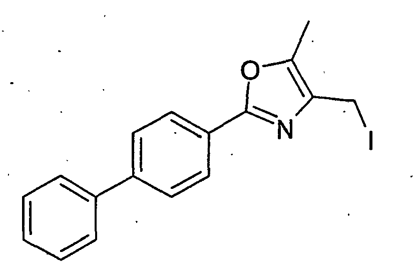 C18H13INO (375,21), MS(ESI):376 (M+h+).

Analog zur Bausteinsynthese von 4-lodmethyl-5-phenyl-2-p-tolyl-oxazol wurde aus Diacetylmonoxim und 3-Trifluormethoxybenzaldehyd 4-lodmethyl-5-methyl-2-(3-trifluoromethoxy-phenyl)-oxazol erhalten. 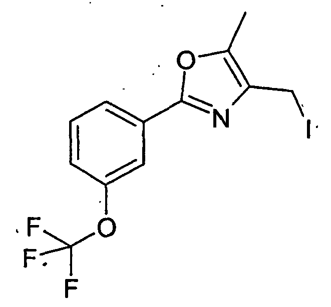 C12H9F3INO2 (383.11), MS(ESI):384 (M+h+).

Analog zur Bausteinsynthese von 4-lodmethyl-5-phenyl-2-p-tolyl-oxazol wurde aus Diacetylmonoxim und 5-Methylfuran-2-carbaldehyd 4-lodmethyl-5-methyl-2-(5-methylfuran-2-yl)-oxazol erhalten. 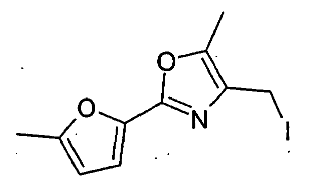 C10H10INO2 (303.11), MS(ESI):304 (M+h+).

Analog zur Bausteinsynthese von 4-lodmethyl-5-phenyl-2-p-tolyl-oxazol wurde aus Diacetylmonoxim und Thiophen-2-carbaldehyd 4-Iodmethyl-5-methyl-2-thiophen-2-yl-oxazol erhalten. 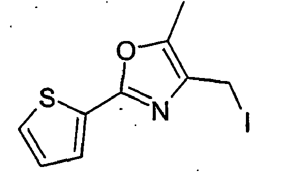 C9H8INOS (305.14), MS(ESI):306 (M+h+).

Analog zur Bausteinsynthese von 4-lodmethyl-5-phenyl-2-p-tolyl-oxazol wurde aus Diacetylmonoxim und 4-lsopropylbenzaldehyd 4-lodmethyl-2-(4-isopropyl-phenyl)-5-methyl-oxazol erhalten. 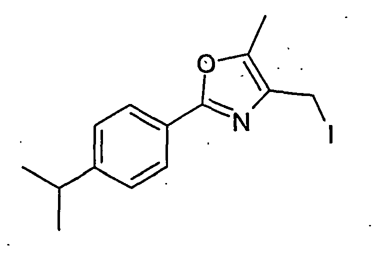 C14H16INO (341.19), MS(ESI):342 (M+h+).

Analog zur Bausteinsynthese von 4-Iodmethyl-5-phenyl-2-p-tolyl-oxazol wurde aus Pentan-2,3-dion 2-oxim und 3- Trifluoromethyl-benzaldehyd 5-Ethyl-4-iodomethyl-2-(3-trifluoromethyl-phenyl)-oxazol erhalten. 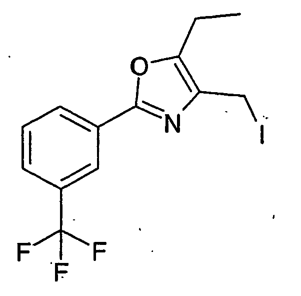 C13H11F3INO (381.14), MS(ESI):382 (M+h+).

Analog zur Bausteinsynthese von 4-lodmethyl-5-phenyl-2-p-tolyl-oxazol wurde aus Pentan-2,3-dion 2-oxim und Naphthalen-2-carbaldehyd 5-Ethyl-4-iodomethyl-2-naphthale-2-yl-oxazol erhalten. 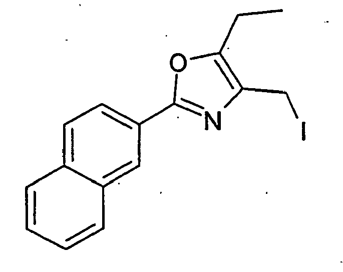 C16H14INO (363.20), MS(ESI):364 (M+h+).

Analog zur Bausteinsynthese von 4-lodmethyl-5-phenyl-2-p-tolyl-oxazol wurde aus Butan-2,3-dion 2-oxim und Naphthalin-2-carbaldehyd 5-Methyl-4-iodomethyl-2-naphthale-2-yl-oxazol erhalten. 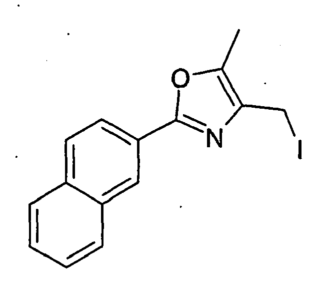 C15H12INO (349.20), MS(ESI):350 (M+h+).

Analog zur Bausteinsynthese von 4-lodmethyl-5-phenyl-2-p-tolyl-oxazol wurde aus Pentan-2,3-dion 2-oxim und 4-Isopropylbenzaldehyd 5-Ethyl-4-iodomethyl-2-(4-isopropyl-phenyl)-oxazol erhalten. 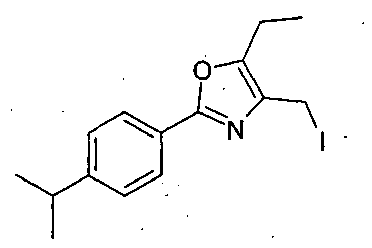 C15H18INO (355.22), MS(ESI):356 (M+h+).

Analog zur Bausteinsynthese von 4-lodmethyl-5-phenyl-2-p-tolyl-oxazol wurde aus Diacetylmonoxim und 3,4-Dimethoxybenzaldehyd 4-lodmethyl-5-methyl-2-(3,4-dimethoxyphenyl)-oxazol erhalten. 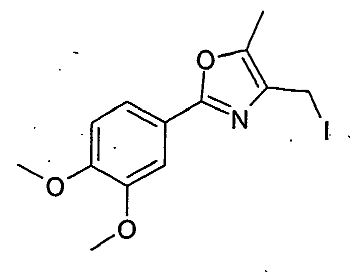 C13H14INO3 (359,17), MS(ESI): 360 (M+h+).

Alle im folgenden beschriebenen Substanzen sind am Cyclohex-1,3-ylen cis-konfiguriert.

### Beispiel 1:

### 2-[cis-3-(5-Methyl-2-p-tolyl-oxazol-4-ylmethoxy)-cyclohexylmethoxy]-pentansäure:

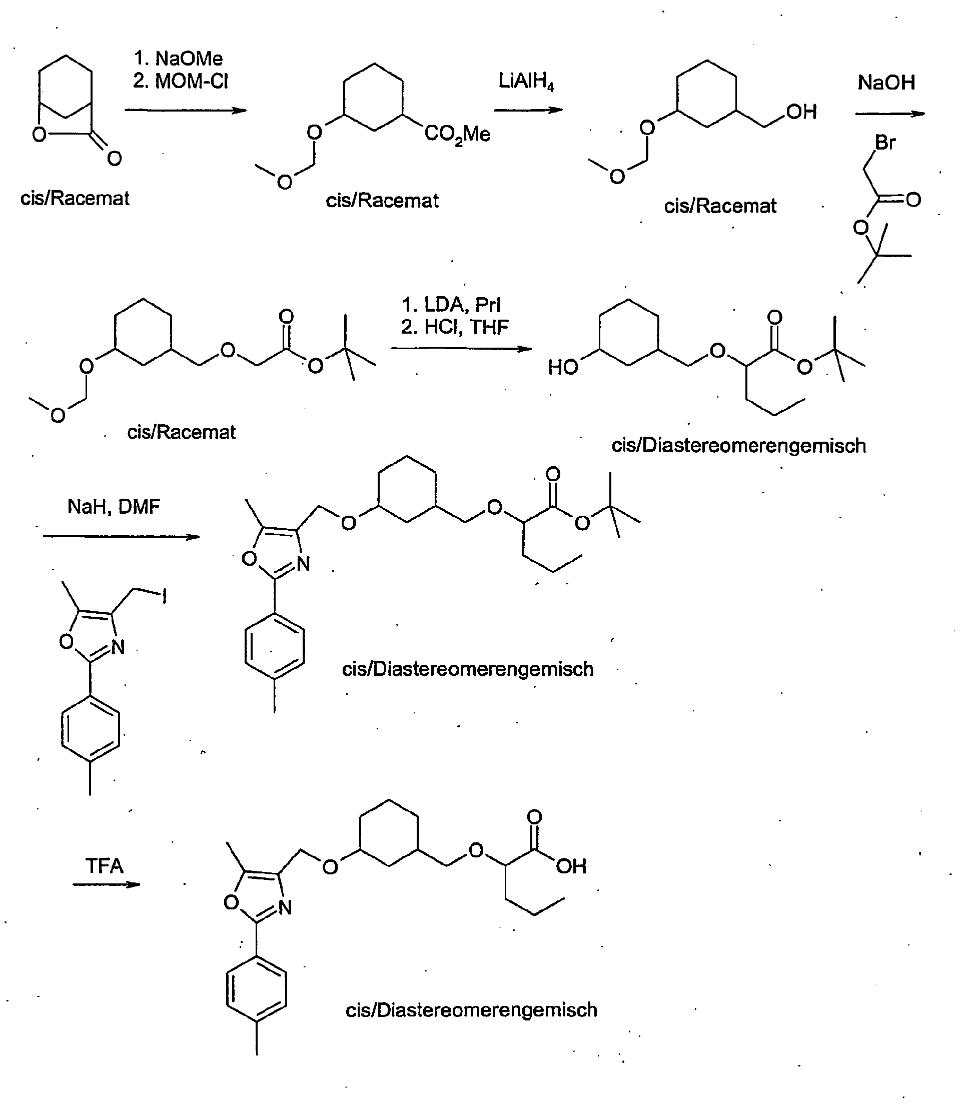

### cis-3-(Methoxymethoxy)-cyclohexancarbonsäuremethylester:

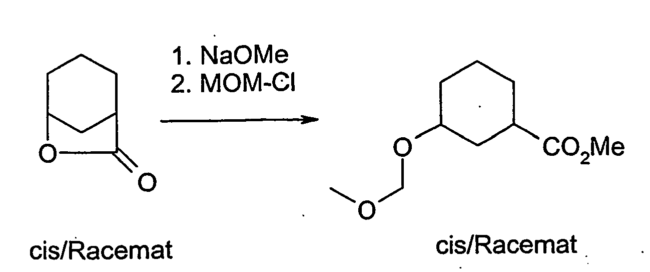

15 g 6-Oxa-bicyclo[3.2.1]octan-7-on werden in 150 ml Methanol gelöst, mit 13 g Natriummethanolat versetzt und 2 h bei Raumtemperatur gerührt. Dann werden 13,7 ml Eisessig zugegeben, und das Lösungsmittel wird im Vakuum weitgehend abdestilliert. Der Rückstand wird in Wasser aufgenommen und dreimal mit je 100 ml Ethylacetat extrahiert. Die organischen Phasen werden über MgSO4 getrocknet und anschließend im Vakuum eingeengt. Man erhält 18,8 g des Methylesters als farbloses Öl. Dieser wird in 150 ml Dichlormethan gelöst und mit 19,2 g Methoxymethylchlorid und 23,2 g Diisopropylethylamin versetzt und 15 h bei Raumtemperatur gerührt. Die Lösung wird mit 250 ml gesättigter NH4Cl-Lösung und 200 ml Wasser vesetzt, die organische Phase wird abgetrennt. Die wäßrige Phase wird mit Dichlormethan extrahiert, die vereinigten organischen Phasen werden über Magnesiumsulfat getrocknet und eingeengt. Man erhält 22,2 g cis-,3-(Methoxymethoxy)-cyclohexancarbonsäuremethylester als gelbes Öl. C10H18O4 (202), MS(ESI): 203 (MH⁺).

### cis-(3-Methoxymethoxycyclohexyl)-methanol:

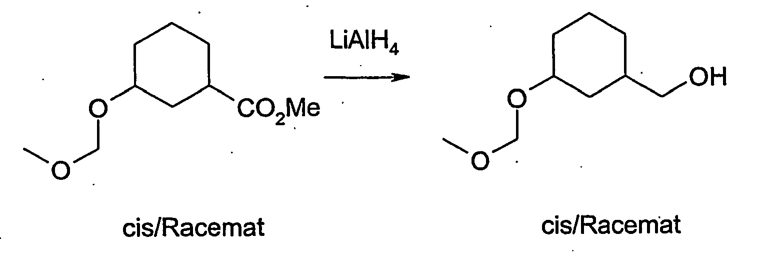

9,0 g cis-3-Methoxymethoxy-cyclohexancarbonsäuremethylester werden in 280 ml Diethylether gelöst und mit 2,2 g LiAlH4 versetzt und bei Raumtemperatur gerührt. Nach 4 h werden bei 0°C 10 ml Ethylacetat und anschließend 15 ml 10N NaOH zugetropft. Die Suspension wird 1 h gerührt und mit MgSO4 versetzt, über Celite filtriert, und das Filtrat eingeengt, wobei 7,0 g (cis-3-Methoxymethoxy-cyclohexyl)-methanol als farbloses Öl erhalten werden. C9H18O3 (174), MS(ESI): 175 (MH⁺).

### (cis-3-Methoxymethoxycyclohexylmethoxy)-essigsäure-tert-butylester:

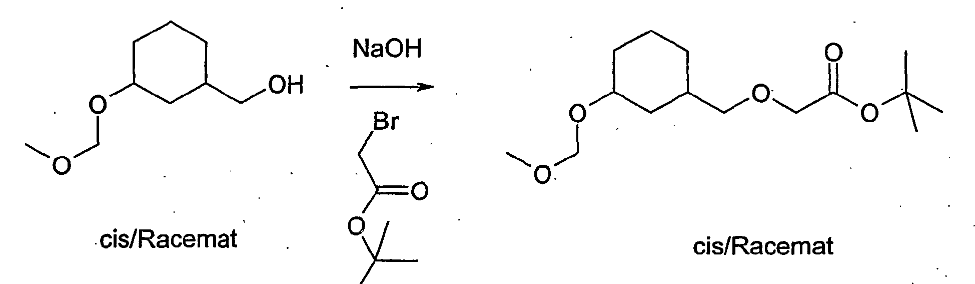

1,0 g (cis-3-Methoxymethoxy-cyclohexyl)-methanol und 3,3 g Bromessigsäure-tert-butylester werden in 30 ml Toluol gelöst und mit 0,50 g Tetrabutylammoniumhydrogensulfat versetzt. Die Suspension wird auf 10°C gekühlt. 10 ml 50% NaOH werden zur Suspension gegeben. Die Mischung wird auf Raumtemperatur kommen lassen und nach 3 h wird die wäßrige Phase abgetrennt und mit Methyl-tert-butylether extrahiert. Die vereinigten organischen Phasen werden über MgSO4 getrocknet und eingeengt. Nach Flash-Säulenchromatographie an Kieselgel (Heptan/Ethytacetat 10/1 -> 2/1) werden 1,10 g (cis-3-Methoxymethoxycyclohexylmethoxy)-essigsäure-tert-butylester als farbloses Öl erhalten. C15H28O5 (288), LCMS(ESI): 306 (M⁺+H2O).

### 2-(cis-3-Hydroxycyclohexylmethoxy)-pentansäure-tert-butytester:

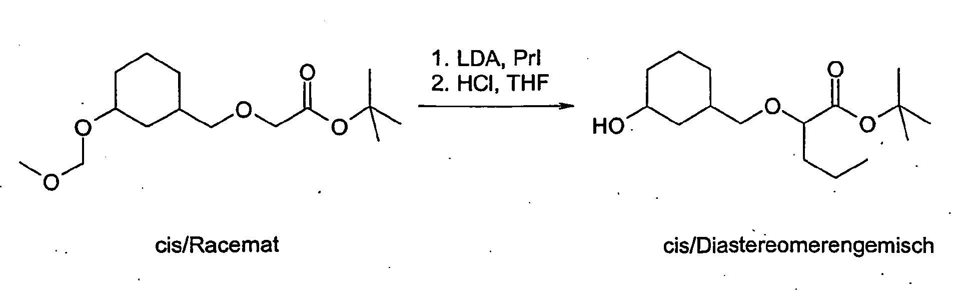

200 mg (cis-3-Methoxymethoxycyclohexylmethoxy)-essigsäure-tert-butylester werden in 5 ml abs. Tetrahydrofuran gelöst und auf -78°C (Trockeneis/Aceton-Bad) gekühlt. Anschließend werden 0,7 ml 2M Lithiumdiisopropylamid-Lösung in Tetrahydrofuran/Hexanfraktion zugetropft. Die Lösung wird zunächst bei -78 °C gerührt, dann auf 0°C erwärmt (Eisbad) und 20 min bei dieser Temperatur gerührt. Dann werden 600 mg Propyliodid in 2 ml Tetrahydrofuran zugegeben, und die Lösung wird weitere 2,5 h bei 0°C gerührt. 15 ml gesättigte Ammoniumchloridlösung werden zugesetzt und die Phasen getrennt. Die wäßrige Phase wird mit Methyl-tert-butylether extrahiert. Die vereinigten organischen Phasen werden über MgSO4 getrocknet und eingeengt (Ausbeute: 240 mg Rohprodukt). Der Rückstand wird in 2 ml Tetrahydrofuran aufgenommen, mit 0,5 ml konz. HCl versetzt und 18 h bei Raumtemperatur gerührt. Die Mischung wird mit Wasser und Methyl-tert-butylether verdünnt, die Phasen werden getrennt, und die wäßrige Phase wird mit Methyl-tert-butylether extrahiert. Die vereinigten organischen Phasen werden mit gesättigter NaCl-Lösung gewaschen, über MgSO4 getrocknet und eingeengt. Dabei werden 130 mg 2-(cis-3-Hydroxycyclohexylmethoxy)-pentansäure-tert-butylester als gelbes Öl erhalten. NMR(CDCl3) Diastereomerengemisch: 3.55-3.67 (m, 2H), 3.41-3.48 (m, 1H), 3.07-3.18 (m, 1H), 1.91-2.13 (m, 2H), 1.11-1.82 (m, 14H), 1.48 (s, 9H).

### 2-[cis-3-(5-Methyl-2-p-tolyl-oxazol-4-ylmethoxy)-cyclohexylmethoxy]-pentansäure-tert-butylester:

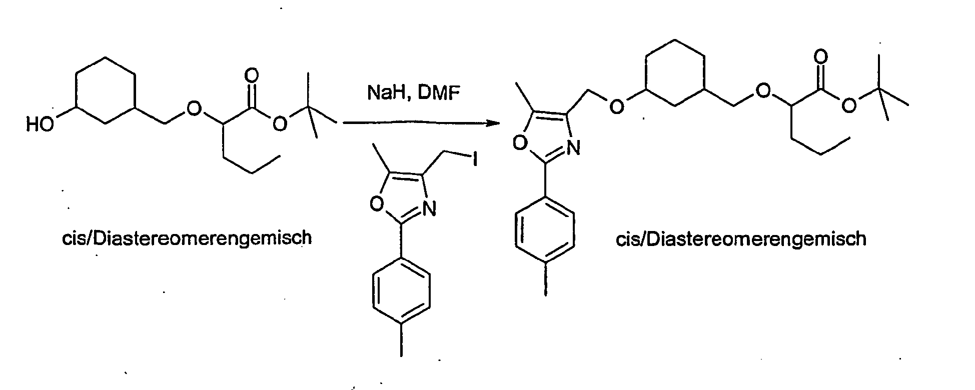

130 mg 2-(cis-3-Hydroxycyclohexylmethoxy)-pentansäure-tert-butylester werden in 3 ml Dimethylformamid gelöst und mit 20 mg NaH (95%) versetzt. Nach 60 min Rühren werden bei 0°C 350 mg 5-Methyl-2-p-tolyl-oxazol-4-ylmethyliodid in 1 ml Dimethylformamid zugegeben. Die Mischung wird bei Raumtemperatur 2 h gerührt. Anschließend werden 10 ml Methyl-tert-butylether, 5 ml Wasser und 10 ml gesättigte NaCl-Lösung zugegeben. Die Phasen werden getrennt, die wäßrige Phase wird einmal mit Methyl-tert-butylether extrahiert, die organsichen Phasen werden über MgSO4 getrocknet und eingeengt. Der Rückstand wird an Kieselgel chromatographiert (Heptan/Ethylacetat 99/1 -> 10/1). 20 mg des rohen 2-[cis-3-(5-Methyl-2-p-tolyl-oxazol-4-ylmethoxy)-cyclohexylmethoxy]-pentansäure-tert-butylesters werden als gelbes Öl erhalten. C28H41NO5 (471), LCMS (ESI): 472 (MH⁺).

### 2-[cis-3-(5-Methyl-2-p-tolyl-oxazol-4-ylmethoxy)-cyclohexylmethoxy]-pentansäure:

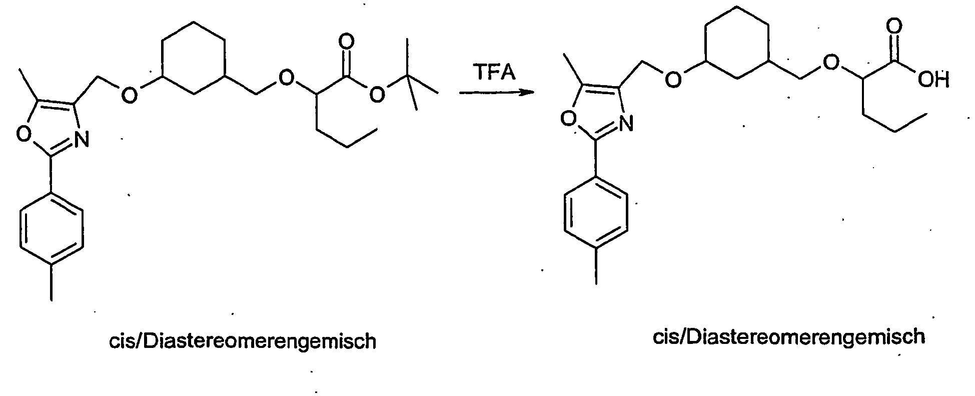

20 mg 2-[cis-3-(5-Methyl-2-p-tolyl-oxazol-4-ylmethoxy)-cyclohexylmethoxy]-pentansäure-tert-butylesters werden in 1 ml Trifluoressigsäure über Nacht gerührt. Die Lösung wird vollständig eingeengt und per HPLC gereinigt, wobei 15 mg 2-[cis-3-(5-Methyl-2-p-tolyl-oxazol-4-ylmethoxy)-cyclohexylmethoxy]-pentansäure 7 erhalten werden. C24H33NO5 (415), MS(ES+) 416 (MH⁺).

### Beispiel 2

Analog zu Beispiel 1 erhält man aus (cis-3-Methoxymethoxycyclohexylmethoxy)-essigsäure-tert-butylester, Methyliodid und 5-Methyl-2-p-tolyl-oxazol-4-ylmethyliodid 2-[cis-3-(5-Methyl-2-p-tolyl-oxazol-4-ylmethoxy)-cyclohexylmethoxy]-propionsäure. C22H29NO5 (387), LCMS(ES+) 388 (MH⁺).

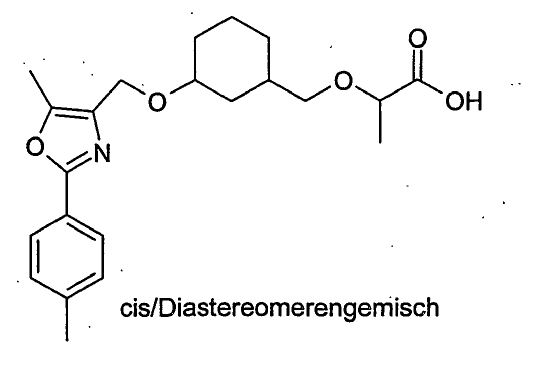

### Beispiel 3

Analog zu Beispiel 1 erhält man aus (cis-3-Methoxymethoxycyclohexylmethoxy)-essigsäure-tert-butylester, Ethyliodid und 5-Methyl-2-p-tolyl-oxazol-4-ylmethyliodid 2-[3-(5-Methyl-2-p-tolyl-oxazol-4-ylmethoxy)-cyclohexylmethoxy]-buttersäure. C23H31NO5 (401), LCMS(ES+) 402 (MH⁺).

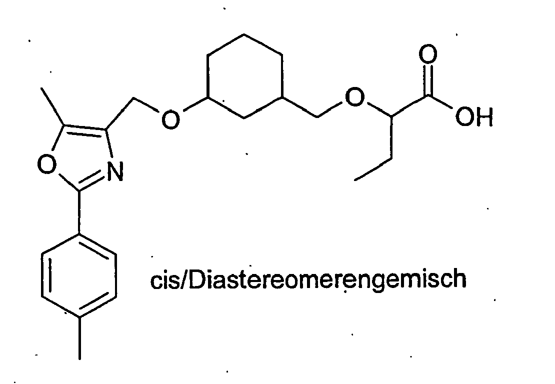

### Beispiel 4

Analog zu Beispiel 1 erhält man aus (cis-3-Methoxymethoxycydohexytmethoxy)-essigsäure-tert-butylester, Benzylbromid und 5-Methyl-2-p-tolyl-oxazol-4-ylmethyliodid 2-[cis-3-(5-Methyl-2-p-tolyl-oxazol-4-ylmethoxy)-cyclohexylmethoxy]-3-phenylpropionäure. C28H33NO5 (463), LCMS(ES+) 464(MH⁺).

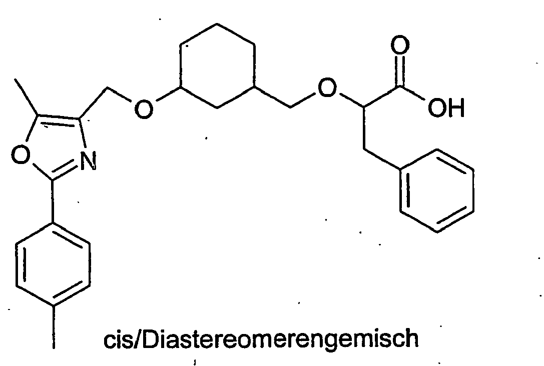

### Beispiel 5

Analog zu Beispiel 1 erhält man aus (cis-3-Methoxymethoxycyclohexylmethoxy)-essigsäure-tert-butylester, Methyliodid und 5-Methyl-2-(3-methoxyphenyl)-oxazol-4-ylmethyliodid 2-[cis-3-(5-Methyl-2-(3-methoxyphenyl)-oxazol-4-ylmethoxy)-cyclohexylmethoxy]-propionsäure. C22H29NO6 (403), LCMS(ES+) 404(MH⁺).

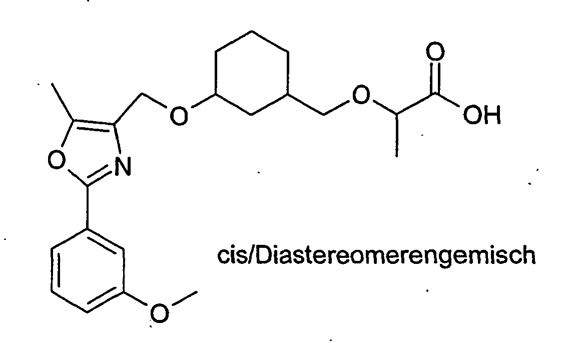

### Beispiel 6

Analog zu Beispiel 1 erhält man aus (cis-3-Methoxymethoxycyclohexylmethoxy)-essigsäure-tert-butylester, Methyliodid und 5-Methyl-2-m-tolyl-oxazol-4-ylmethyliodid 2-[cis-3-(5-Methyl-2-m-tolyl-oxazol-4-ylmethoxy)-cyclohexylmethoxy]-propionsäure. C23H31NO5 (401), LCMS(ES+) 402 (MH⁺).

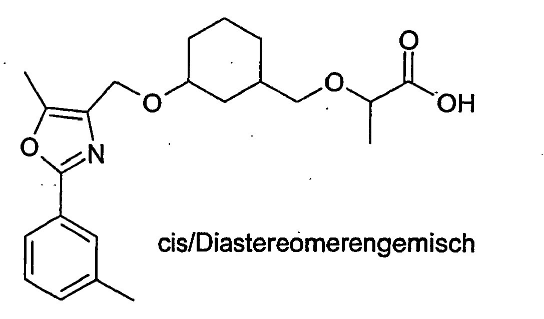

### Beispiel 7

Analog zu Beispiel 1 erhält man aus (cis-3-Methoxymethgoxycyclohexylmethoxy)-essigsäure-tert-butylester, Methyliodid und 5-methyl-2-(3-trifluormethylphenyl)-oxazol-4-ylmethyliodid 2-[cis-3-(5-Methyl-2-(3-trifluormethylphenyl)-oxazol-4-ylmethoxy)-cyclohexylmethoxy]-propionsäure. C22H26F3NO5 (441), LCMS(ES+) 442 (MH⁺).

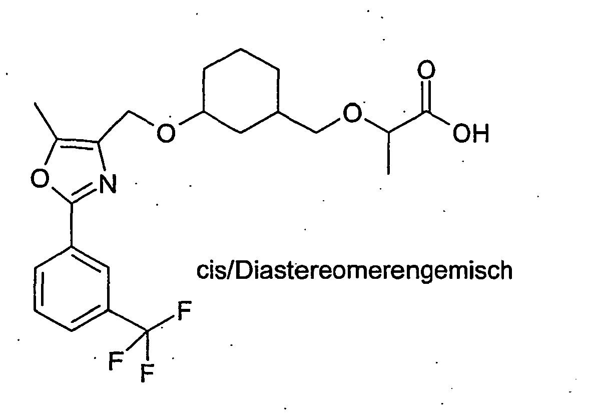

### Beispiel 8

2-[cis-3-(5-Methyl-2-p-tolyl-oxazol-4-ylmethoxy)-cyclohexylmethoxy]-2-methylpropionsäure:

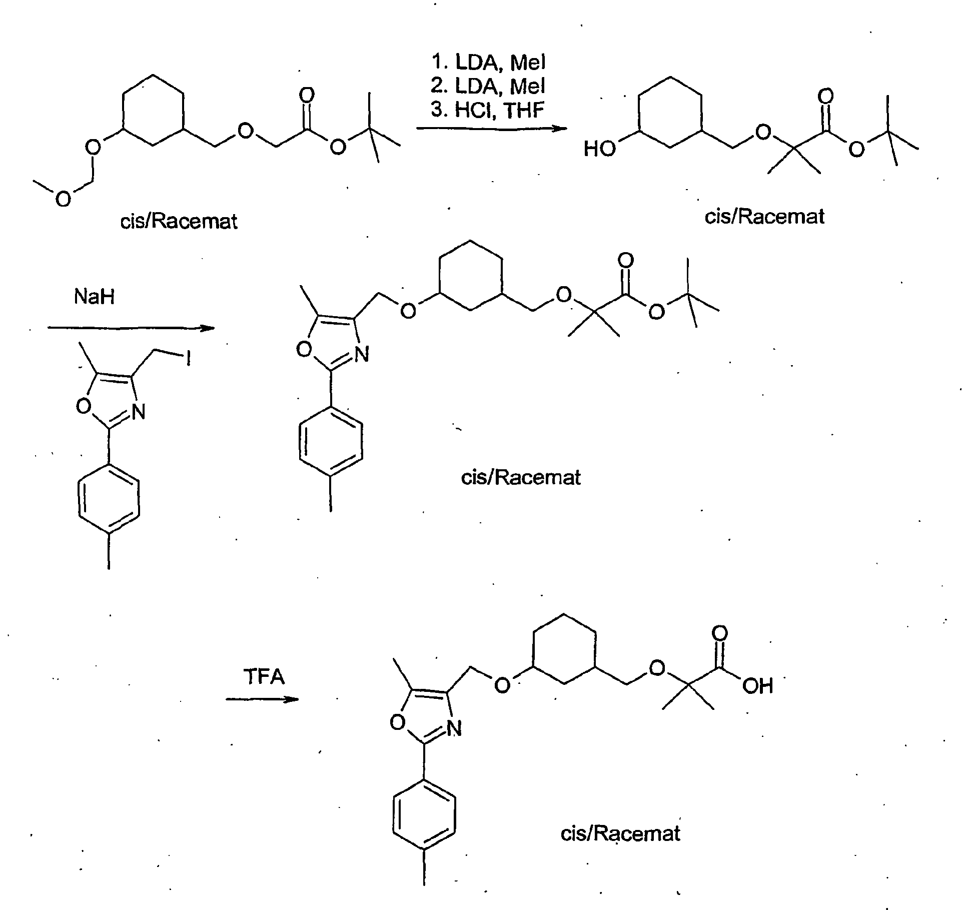

### 2-(cis-3-Hydroxycyclohexylmethoxy)-2-methylpropionsäure-tert-butylester:

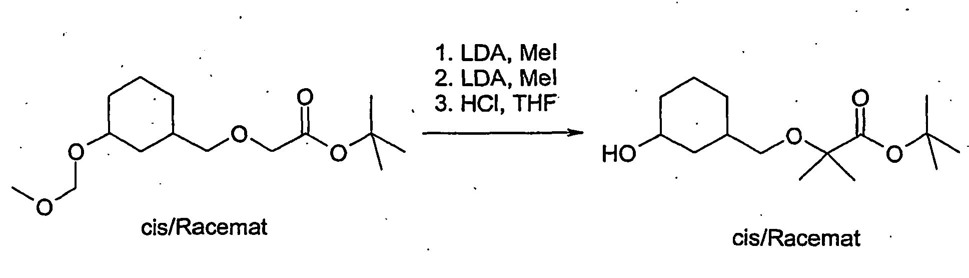

300 mg (cis-3-Methoxymethoxycyclohexylmethoxy)-essigsäure-tert-butylester werden in 5 ml abs. Tetrahydrofuran gelöst und auf-78°C (Trockeneis/Aceton-Bad) gekühlt. Anschließend werden 1,5 ml 2M Lithiumdiisopropylamid-Lösung in Tetrahydrofuran/Hexanfraktion zugetropft. Die Lösung wird zunächst bei -78 °C 90 min gerührt, dann auf 0°C erwärmt (Eisbad) und mit 1,41 g Methyliodid in 1,5 ml Tetrahydrofuran zugegeben, und die Lösung wird 1 h bei 0°C gerührt. 1 ml HCl (konz.) wird zugesetzt und die Phasen werden getrennt. Die wäßrige Phase wird mit Ethylacetat extrahiert. Die vereinigten organischen Phasen werden über MgSO4 getrocknet und eingeengt (Ausbeute: 320 mg Rohprodukt). Das Rohprodukt wird in 5 ml abs. Tetrahydrofuran gelöst und auf -78°C (Trockeneis/Aceton-Bad) gekühlt. Anschließend werden 1,5 m12M Lithiumdiisopropylamid-Lösung in Tetrahydrofuran/Hexanfraktion zugetropft. Die Lösung wird zunächst bei -78 °C 90 min gerührt, dann auf 0°C erwärmt (Eisbad) und mit 1,41 g Methyliodid in 1,5 ml Tetrahydrofuran zugegeben, und die Lösung wird 1 h bei 0°C gerührt. 1 ml HCl (konz.) wird zugesetzt und die Phasen werden getrennt. Die wäßrige Phase wird mit Ethylacetat extrahiert. Die vereinigten organischen Phasen werden über MgSO4 getrocknet und eingeengt (Ausbeute: 350 mg Rohprodukt). Der Rückstand wird in 1 ml Tetrahydrofuran aufgenommen, mit 1 ml konz. HCl versetzt und 3 d bei Raumtemperatur gerührt. Die Mischung wird mit Wasser und Ethylacetat verdünnt, die Phasen werden getrennt, und die wäßrige Phase wird mit Ethylacetat extrahiert. Die vereinigten organischen Phasen werden mit gesättigter NaCl-Lösung gewaschen, über MgSO4 getrocknet und eingeengt. Der Rückstand wird an Kieselgel (Heptan/Ethylacetat 2/1) chromatographiert, wobei 200 mg 2-(cis-3-Hydroxycyclohexylmethoxy)-2-methylpropionsäure-tert-butylester als gelbes Öl erhalten werden. C15H28O4 (272,20), MS(ESI): 273,4 (MH⁺).

### 2-[cis-3-(5-Methyl-2-p-tolyl-oxazol-4-ylmethoxy)-cyclohexylmethoxy]-2-methylpropionsäure-tert-butylester:

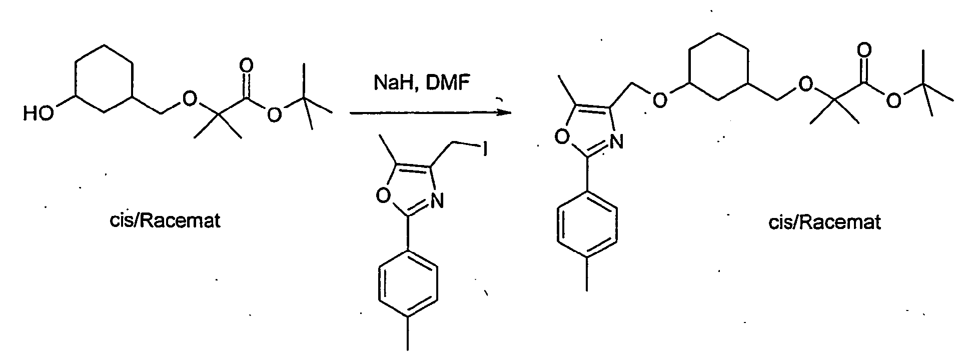

200 mg 2-(cis-3-Hydroxycyclohexylmethoxy)-2-methylpropionsäure-tert-butylester werden in 5 ml Dimethylformamid gelöst und mit 20 mg NaH (95%) versetzt. Nach 60 min Rühren bei Raumtemperatur werden bei 0°C 460 mg 5-Methyl-2-p-tolyl-oxazol-4-ylmethyliodid in 1,5 ml Dimethylformamid zugegeben. Die Mischung wird bei Raumtemperatur 2 h gerührt. Anschließend werden 10 ml Methyl-tert-butylether und 10 ml gesättigte NH4Cl-Lösung zugegeben. Die Phasen werden getrennt, die wäßrige Phase wird einmal mit Methyl-tert-butylether extrahiert, die organsichen Phasen werden über MgSO4 getrocknet und eingeengt. Der Rückstand wird an Kieselgel chromatographiert (Heptan/Ethylacetat 5/1 -> 1/1). 200 mg des rohen 2-[cis-3-(5-Methyl-2-p-tolyl-oxazol-4-ylmethoxy)-cyclohexylmethoxy]-2-methylpropionsäure-tert-butylesters werden als gelbes Öl erhalten. C27H39NO5 (457), LCMS(ESI): 458 (MH⁺).

### Verbesserte Synthese von 2-[cis-3-(5-Methyl-2-p-tolyl-oxazol-4-ylmethoxy)-cyclohexylmethoxy]-2-methylpropionsäure-tert-butylester:

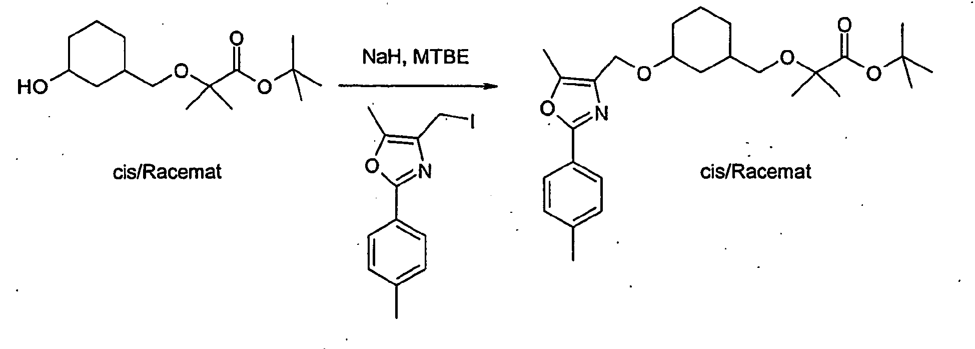

50 mg 2-(cis-3-Hydroxycyclohexylmethoxy)-2-methylpropionsäure-tert-butylester werden in 0,5 ml Dimethylformamid gelöst und mit 22 mg NaH (60%) versetzt. Nach 30 min Rühren werden bei Raumtemperatur 112 mg 5-Methyl-2-p-tolyl-oxazol-4-ylmethyliodid zugegeben. Die Mischung wird 10 min ins Ultraschallbad gegeben und anschließend 3 h bei Raumtemperatur gerührt. Anschließend werden 10 ml Methyl-tert-butylether und 10 ml Wasser zugegeben. Die Phasen werden getrennt, die wäßrige Phase wird einmal mit Methyl-tert-butylether extrahiert, die organsichen Phasen werden über MgSO4 getrocknet und eingeengt. Der Rückstand wird an Kieselgel chromatographiert (Heptan/Ethylacetat 5/1 -> 1/1). 60 mg des rohen 2-[cis-3-(5-Methyl-2-p-tolyl-oxazol-4-ylmethoxy)-cyclohexylmethoxy]-2-methylpropionsäure-tert-butylesters werden als gelbes Öl erhalten. C27H39NO5 (457), LCMS(ESI): 458 (MH⁺).

### 2-[cis-3-(5-Methyl-2-p-tolyl-oxazol-4-ylmethoxy)-cyclohexyimethoxy]-2-methylpropionsäure:

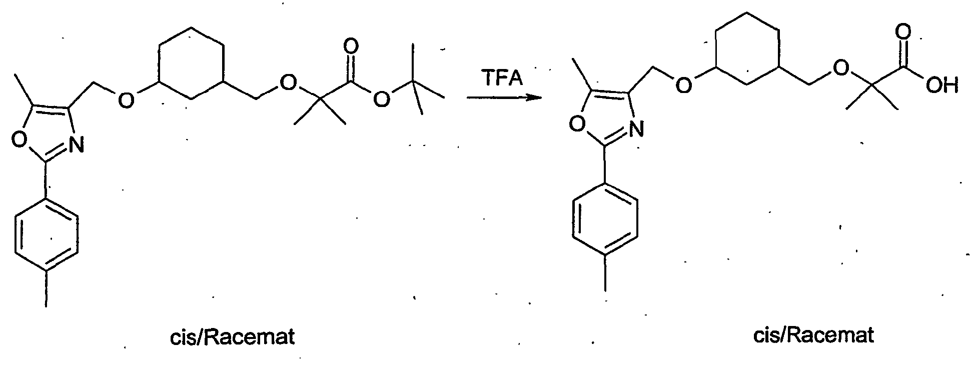

200 mg 2-[cis-3-(5-Methyl-2-p-tolyl-oxazol-4-ylmethoxy)-cyclohexylmethoxy]-2-methylpropionsäure-tert-butylesters werden in 2 ml Trifluoressigsäure 1 h gerührt. Die Lösung wird vollständig eingeengt und flash-chromatographisch gereinigt (Heptan/Ethylacetat 5/1), wobei 66 mg 2-[cis-3-(5-Methyl-2-p-tolyl-oxazol-4-ylmethoxy)-cyclohexylmethoxy]-2-methylpropionsäure erhalten werden. C23H31NO5 (401, 51), MS(ES+) 402,29 (MH⁺).

### Beispiel 8a:

### 2-[(1R,3S)-3-(5-Methyl-2-p-tolyl-oxazol-4-ylmethoxy)-cyclohexyl methoxy]-2-methylpropionsäure

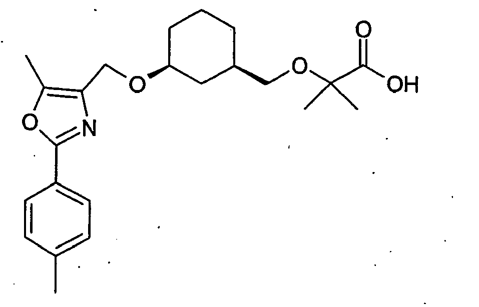

Ausgehend von enantiomerenreinem 2-[(1R,3S)-cis-3-Hydroxycyclohexylmethoxy]-2-methylpropionsäure-tert-butylester erhält man 2-[(1R,3S)-cis-3-(5-Methyl-2-p-tolyl-oxazol-4-ylmethoxy)-cyclohexylmethoxy]-2-methylpropionsäure. C23H31NO5 (401,51), MS(ES+) 402,29 (MH⁺).

### Beispiel 9:

### 1-[cis-3-(5-Methyl-2-p-tolyl-oxazol-4-ylmethoxy)-cyclohexylmethoxy]-cyclopentancärbonsäure:

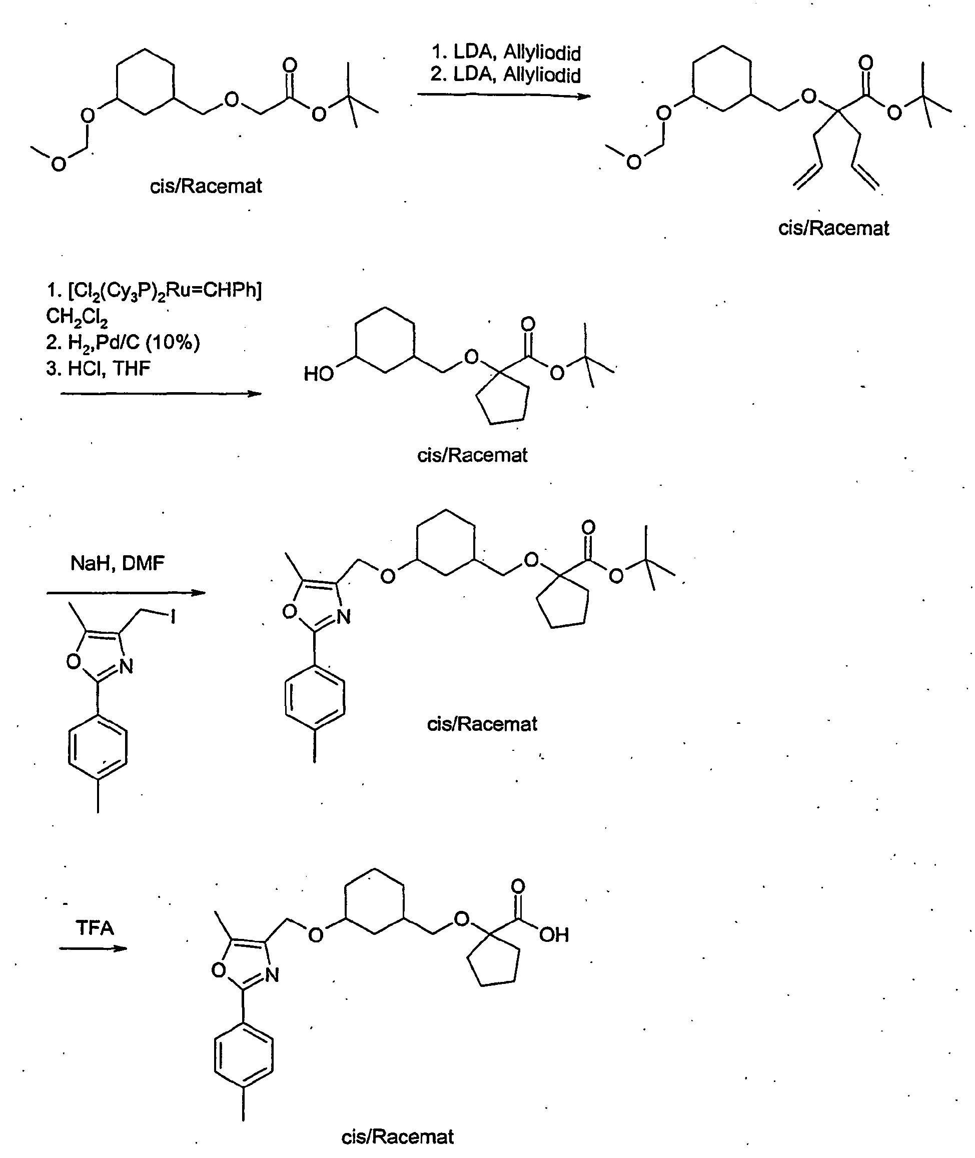

### 2-Allyl-2-(cis-3-methoxymethoxycyclohexylmethoxy)-pent-4-ensäure-tert-butylester:

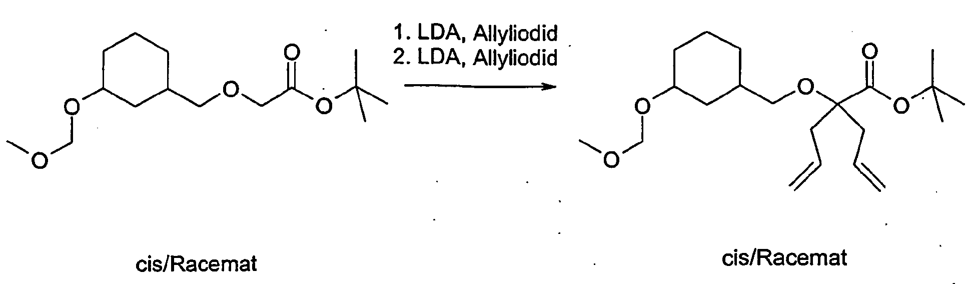

200 mg (cis-3-Methoxymethoxycyclohexylmethoxy)-essigsäure-tert-butylester werden in 6 ml abs. Tetrahydrofuran gelöst und auf -78°C (Trockeneis/Aceton-Bad) gekühlt. Anschließend werden 1,05 ml 2M Lithiumdiisopropylamid-Lösung in Tetrahydrofuran/Hexanfraktion zugetropft. Die Lösung wird zunächst bei -78 °C, dann bei 0 °C jeweils 20 min gerührt und bei 0 °C mit 0,85 g Allylbromid in 1,5 ml Tetrahydrofuran versetzt. Die Lösung wird 30 min bei 0°C gerührt. 1 ml gesättigte NH4Cl-Lösung und 5 ml Ethylacetat werden zugesetzt und die Phasen werden getrennt. Die wäßrige Phase wird mit Ethylacetat extrahiert. Die vereinigten organischen Phasen werden über MgSO4 getrocknet und eingeengt. Der Rückstand wird an Kieselgel chromatographiert (Heptan/Ethylacetat 10/1), wobei 160 mg monoallyliertes Produkt erhalten werden. Dieses wird in 6 ml abs. Tetrahydrofuran gelöst und auf -78°C (Trockeneis/Aceton-Bad) gekühlt. Anschließend werden 1,05 ml 2M Lithiumdiisopropylamid-Lösung in Tetrahydrofuran/Hexanfraktion zugetropft. Die Lösung wird zunächst bei -78 °C, dann bei 0 °C jeweils 20 min gerührt und bei 0 °C mit 0,85 g Allylbromid in 1,5 ml Tetrahydrofuranversetzt. Die Lösung wird 2 h bei 0°C gerührt. 1 ml gesättigte NH4Cl-Lösung und 5 ml Ethylacetat werden zugesetzt und die Phasen werden getrennt. Die wäßrige Phase wird mit Ethylacetat extrahiert. Die vereinigten organischen Phasen werden über MgSO4 getrocknet und eingeengt. Der Rückstand wird an Kieselgel chromatographiert (Heptan/Ethylacetat 5/1), wobei 140 mg 2-Allyl-2-(cis-3-methoxymethoxycyclohexylmethoxy)-pent-4-ensäure-tert-butylester als gelbes Öl erhalten werden. C21H36O5 (368,52), MS(ESI): 296,25 (MH⁺-C4H9O).

### (cis-3-Hydroxycyclohexylmethoxy)-cyclopentancarbonsäure-tert-butylester:

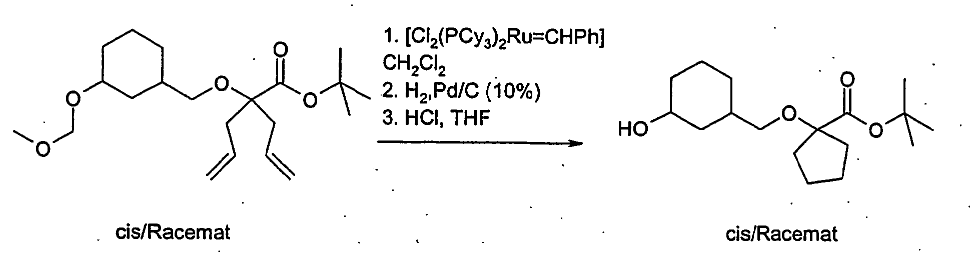

140 mg 2-Allyl-2-(cis-3-methoxymethoxycyclohexylmethoxy)-pent-4-ensäure-tert-butylester werden in 5 ml Dichlormethan gelöst, unter einer Ar-Atmosphäre mit 10 mg Grubbs-Katalysator (Cl₂(Cy₃P)₂Ru=CHPh) versetzt und bei 40 °C 48 h gerührt. 10 ml Heptan/Ethylacetat (3/1) werden zugegeben und die Lösung wird über Kieselgel filtriert. 100 mg 1-(cis-3-Methoxymethoxycyclohexylmethoxy)-cyclopent-3-encarbonsäure-tert-butylester werden als braunes Öl erhalten. Dieses wird in 2 ml MeOH gelöst, entgast und mit Ar gesättigt. Dann werden 30 mg Pd/C (10%) zugegeben und erneut entgast. Die Lösung wird mit Wasserstoff gesättigt und bei Raumtemperatur über Nacht gerührt. Verdünnen mit 20 ml Ethylacetat und Filtration über Celite liefern 100 mg rohen 1-(cis-3-Methoxymethoxycyclohexylmethoxy)-cyclopentancarbonsäure-tert-butylester. Dieser wird in 2 ml Tetrahydrofuran aufgenommen, mit 0,5 ml HCl (konz.) versetzt und über Nacht bei Raumtemperatur gerührt. Die Lösung wird mit gesättigter NaHCO3-Lösung neutralisiert und dreimal mit Ethylacetat extrahiert. Die organischen Phasen werden über MgSO4 getrocknet und eingeengt. Chromatographie des Rückstandes an Kieselgel (Heptan/Ethylacetat 10/1 -> 1/1) liefert 57 mg 1-(cis-3-Hydroxycyclohexylmethoxy)-cyclopentancarbonsäure-tert-butylester als gelbes Öl. Dieses wird roh für die nächste Stufe eingesetzt.

### 1-[cis-3-(5-Methyl-2-p-tolyl-oxazol-4-ylmethoxy)-cyclohexylmethoxy]-cyclopentancarbonsäure-tert-butylester:

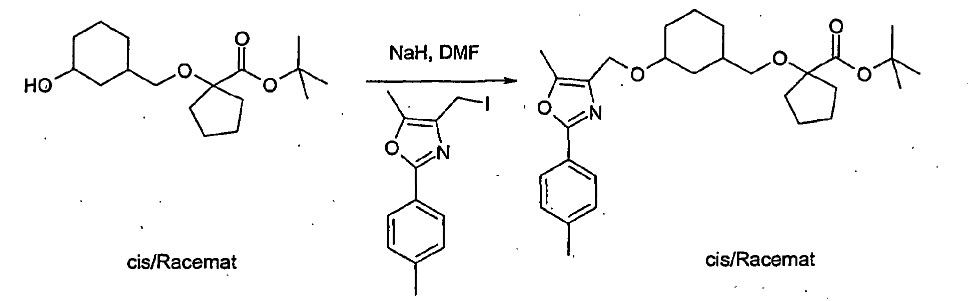

57 mg 1-(cis-3-Hydroxycyclohexylmethoxy)-cyclopentancarbonsäure-tert-butylester werden'in 3 ml Dimethylformamid gelöst und mit 10 mg NaH versetzt. Die Suspension wird 30 min bei Raumtemperatur gerührt, dann auf 0 °C gekühlt und tropfenweise mit 150 mg Methyl-2-p-tolyl-oxazol-4-ylmethyliodid in 1 ml Dimethylformamid versetzt. Die Suspension wird 2 h bei Raumtemperatur gerührt und mit Methyl-tert-butylether und gesättigter Nacl-Lösung verdünnt. Die wäßrige Phase wird abgetrennt und mit Methyl-tert-butylether extrahiert. Die vereinigten organischen Phasen werden mit gesättigter NaCl-Lösung gewaschen, über MgSO4 getrocknet und eingeengt. Chromatographie des Rückstandes an Kieselgel (Heptan/Ethylacetat 99/ -> 10/1) liefert 20 mg eines Produktgemisches, das den gewünschten 1-[cis-3-(5-Methyl-2-p-tolyl-oxazol-4-ylmethoxy)-cyclohexylmethoxy]-cyclopentancarbonsäure-tert-butylester It. LCMS enthält. Dieses Gemisch wird ohne weitere Aufreinigung in der nächsten Stufe eingesetzt. C29H41NO5 (483,65), LCMS (ESI): 484,2 (MH⁺).

### 1-[cis-3-(5-Methyl-2-p-tolyl-oxazol-4-ylmethoxy)-cyclohexylmethoxy]-cyclopentancarbonsäure:

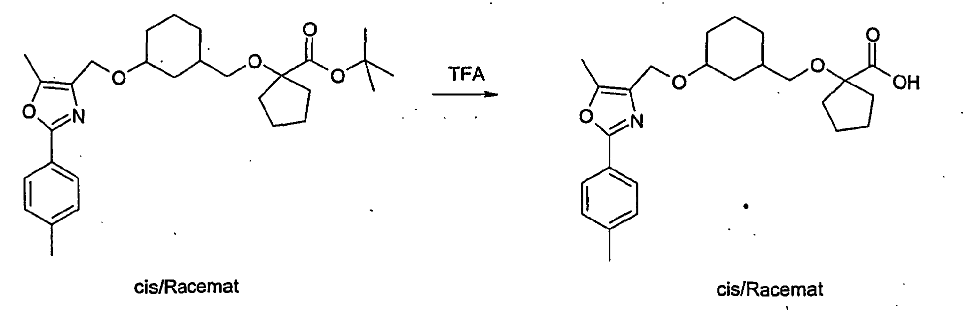

20 mg verunreinigter 1-[cis-3-(5-Methyl-2-p-tolyl-oxazol-4-ylmethoxy)-cyclohexylmethoxy]-cyclopentancarbonsäure-tert-butylester wird in 1 ml Trifluoressigsäure bei Raumtemperatur über Nacht gerührt. Die Lösung wird vollständig eingeengt und der Rückstand an Kieselgel chromatographiert (Heptan/Ethylacetat 10/1 -> 1/1 -> Methyl-tert-butylether), wobei 7,5 mg 1-[cis-3-(5-Methyl-2-p-tolyl-oxazol-4-ylmethoxy)-cyclohexylmethoxy]-cyclopentancarbonsäure erhalten werden. C25H33NO5 (427,55); LCMS 428,2 (MH+).

### Beispiel 10:

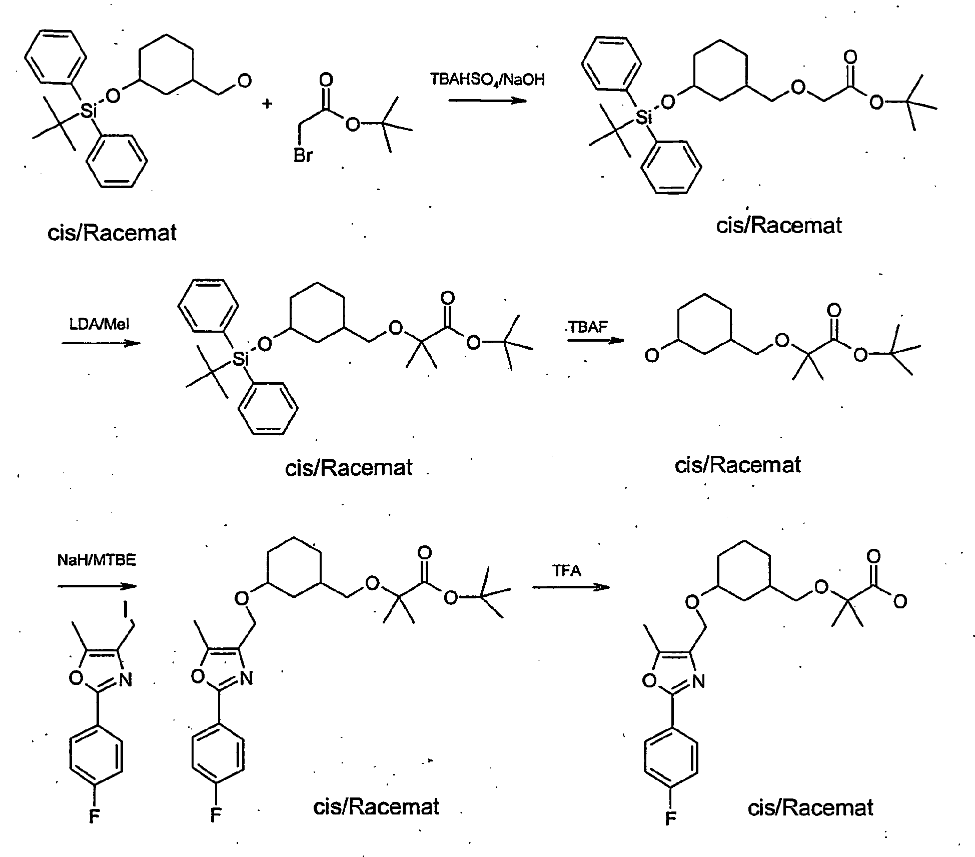

### cis-3-(tert-Butyl-diphenyl-silanyloxy)-cyclohexylmethoxy]-essigsäure-tert-butylester

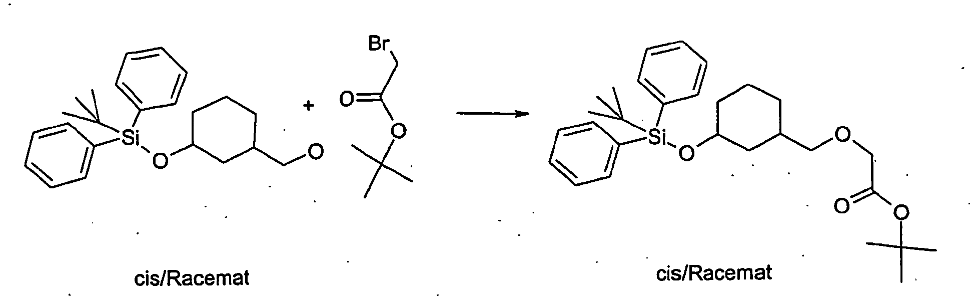

25 g [cis-3-(tert-Butyl-diphenyl-silanyloxy)-cyclohexyl]-methanol werden zusammen mit 40 g Bromessigsäure-tert-butylester und 6,9 g Tetrabutylammoniumhydrogensulfat in 300 ml Toluol gelöst und anschließend bei 0 °C 200 ml NaOH (50%ig) zugetropft. Bei 0 °C wird 1 h gerührt und dann auf Raumtemperatur erwärmt. Das Lösungsmittel wird entfernt und mit 3 x 100 ml Methyl-tert-butylether extrahiert. Nach der dritten Extraktion wird die wässrige Phase angesäuert und nochmals mit 200 ml Methyl-tert-butylether extrahiert. Die vereinigten organischen Phasen werden mit gesättigter Natriumclorid-Lösung ausgeschüttelt, über Magnesiumsulfat getrocknet und das Lösungsmittel im Vakuum entfernt. Man erhält 27,8 g des cis-3-(tert-Butyl-diphenyl-silanyloxy)-cyclohexylmethoxy]-essigsäure-tert-butylesters als gelbes Öl.
C₂₉H₄₂O₄Si (482.74), MS(ESI): 483 (M + H⁺)

### 2-[cis-3-(tert-Butyl-diphenyl-silanyloxy)-cyclohexylmethoxy]-2-methyl-propionsäure-tert-butylester

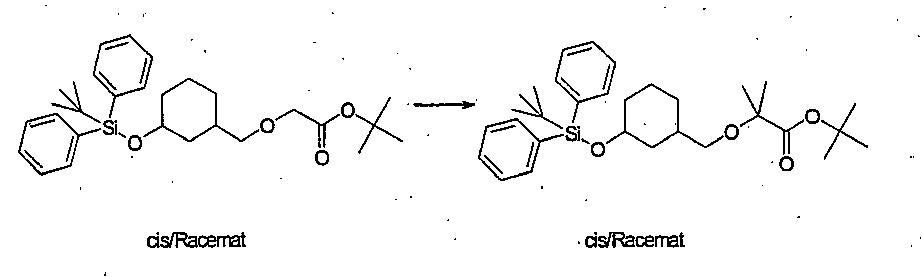

20,0 g des cis-3-(tert-Butyl-diphenyl-silanyloxy)-cyclohexylmethoxy]-essigsäure-tert-butylesters werden in einem ausgeheizten 1L-Dreihals-Kolben vorgelegt, in 200 ml trockenem Tetrahydrofuran gelöst, auf - 78 °C gekühlt und 83 ml Lithiumdiisopropylamid (2N in Tetrahydrofuran) langsam zugetropft, so dass die Innentemperatur nicht über - 65 °C ansteigt. Anschließend wird auf 0 °C erwärmt und 1 h gerührt, wobei sich die Lösung gelb färbt. Nach erneutem Abkühlen auf - 70 °C werden 35,27 g Methyliodid zugetropft und 3 h bei 0 °C gerührt. Reaktionskontrolle (DC und LCMS) ergibt die Bildung eines neuen Produktes (Monomethyl Verbindung). Das Reaktionsgemisch wird mit 200 ml gesättigter Ammoniumchlorid-Lösung versetzt und mit Wasser/Methyl-tert-butylether extrahiert. Man erhält das Rohprodukt als dunkelrotes Öl, welches ohne Aufreinigung in der gleichen Reaktionssequenz zur geminalen Dimethylverbindung umgesetzt wird. Das Rohprodukt wird an Kieselgel (Heptan/Ethylacetat 50:1 → 10:1) gereinigt. Man erhält 16 g des 2-[cis-3-(tert-Butyldiphenyl-silanyloxy)-cyclohexylmethoxy]-2-methyl-propionsäure-tert-butylesters als hellgelbes Öl.
C₃₁H₄₆O₄Si (510.80), MS(ESI): 511 (M+H⁺)

### 2-(cis-3-Hydroxy-cyclohexylmethoxy)-2-methyl-propionsäure-tert-butylester

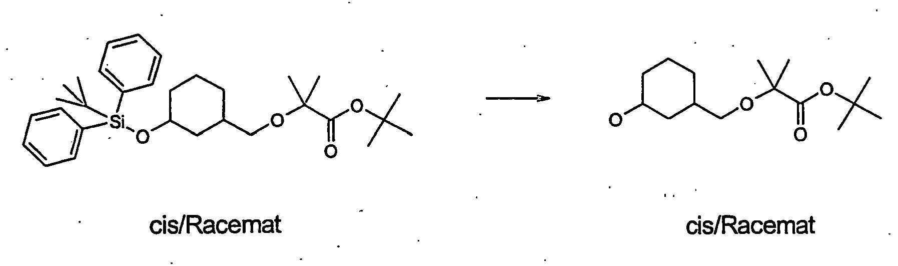

16 g des 2-[cis-3-(tert-Butyl-diphenyl-silanyloxy)-cyclohexylmethoxy]-2-methylpropionsäure-tert-butylesters werden in 100 ml Acetonitril gelöst und mit 62 ml Tetrabutylammoniumfluorid (1N Lösung in Tetrahydrofuran) versetzt. Nach 2 h Rühren bei 60 °C ist die Reaktion beendet und es wird im Vakuum eingeengt. Der Rückstand wird aus Wasser/Ethylacetat extrahiert. Die vereinigten org. Phasen werden mit gesättigter Natriumchlorid-Lösung ausgeschüttelt, über Magnesiumsulfat getrocknet und das Lösungsmittel im Vakuum entfernt. Das Rohprodukt wird an Kieselgel (Heptan/Ethylacetat 15:1 → 1:1) gereinigt. Man erhält 16 g des Produktes 2-(cis-3-Hydroxy-cyclohexylmethoxy)-2-methyl-propionsäure-tert-butylester als farbloses Öl. C₁₅H₂₈O₄ (272.39), MS(ESI): 273 (M + H⁺)

### 2-{cis-3-[2-(4-Fluoro-phenyl)-5-methyl-oxazol-4-ylmethoxy]-cyclohexylmethoxy}-2-methyl-propionsäure-tert-butylester

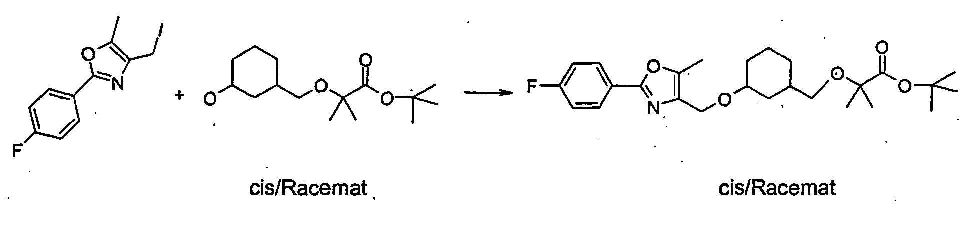

0,05 g des Alkohols 2-(cis-3-Hydroxy-cyclohexylmethoxy)-2-methyl-propionsäure-tert-butylester wird in Methyl-tert-butylether gelöst und mit 15 mg Natriumhydrid versetzt. Nach 15 Minuten Rühren bei Raumtemperatur werden 0.12 g 2-(4-Fluoro-phenyl)-4-iodmethyl-5-methyl-oxazol zugegeben und 12 h bei Raumtemperatur gerührt. Nach Zugabe von 2 ml 1N HCl wird mit Ethylacetat (2 x 5 ml) extrahiert, das Lösungsmittel im Vakuum entfernt und das Rohprodukt anschließend über HPLC gereinigt. Man erhält 0.08 g der Verbindung 2-{cis-3-[2-(4-Fluoro-phenyl)-5-methyl-oxazol-4-ylmethoxy]-cyclohexylmethoxy}-2-methyl-propionsäure-tert-butylester als farbloses Öl C₂₆H₃₆FNO₅ (461.58), MS(ESI): 462 (M + H⁺)

### 2-{cis-3-[2-(4-Fluoro-pheny)-5-methy-oxazol-4-ylmethoxy]-cyclohexylmethoxy}-2-methyl-propionsäure

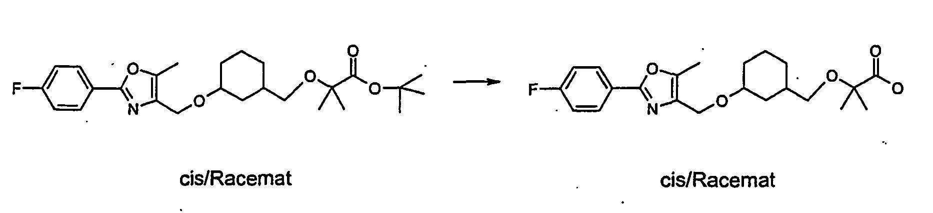

0,07 g des 2-{cis-3-[2-(4-Fluoro-phenyl)-5-methyl-oxazol-4-ylmethoxy]-cyclohexylmethoxy}-2-methyl-propionsäure-tert-butylesters wird in 1 ml Dichlormethan gelöst, mit 1 ml Trifluoressigsäure versetzt und bei Raumtemperatur gerührt. Reaktionskontrolle (LCMS) ergibt nach 30 Minuten einen vollständigen Umsatz. Das Reaktionsgemisch wird mit Wasser / Dichlormethan ausgeschüttelt und nach Entfernen des Lösungsmittels im Vakuum mittels präparativer HPLC gereinigt. Man erhält 0,06 g der Carbonsäure 2-{cis-3-[2-(4-Fluoro-phenyl)-5-methyl-oxazol-4-ylmethoxy]-cyclohexylmethoxy}-2-methyl-propionsäure als farbloses Öl.
C₂₂H₂₈FNO₅ (405.47), MS(ESI): 406 (M + H⁺)

### Beispiel 11:

Analog zu Beispiel 10 erhält man aus 2-(cis-3-Hydroxycyclohexylmethoxy)-2-methylpropionsäure-tert-butylester und 2-(3-Methoxy-phemyl)-5-methyl-oxazol-4-ylmethyliodid 2-{(1 S,3R)-cis-3-[2-(3-Methoxy-phenyl)-5-methyl-oxazol-4-ylmethoxy]-cyclohexylmethoxy}-2-methyl-propionsäure.

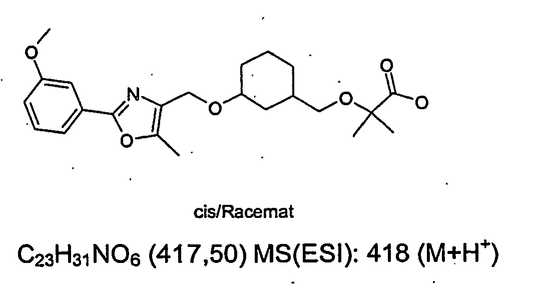

### Beispiel 12:

Analog zu Beispiel 10 erhält man aus 2-(cis-3-Hydroxycyclohexylmethoxy)-2-methylpropionsäüre-tert-butylester und 2-(3-Triflüormethyl-phenyl)-5-methyl-oxazol-4-ylmethyliodid 2-{(1S,3R)-cis-3-[2-(3-Trifluormethyl-phenyl)-5-methyl-oxazol-4-ylmethoxy]-cyclohexylmethoxy}-2-methyl-propionsäure. 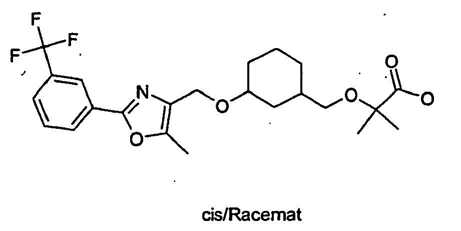 C₂₃H₂₈F₃NO₅ (455,47), MS(ESI): 456 (M + H⁺).

### Beispiel 13:

Analog zu Beispiel 10 erhält man aus 2-(cis-3-Hydroxycyclohexylmethoxy)-2-methylpropionsäure-tert-butylester und 5-methyl-2-(5-methyl-furan-2-yl)-oxazol-4-ylmethyliodid 2-Methyl-2-{(1R,3S)-cis-3-[5-methyl-2-(5-methyl-furan-2-yl)-oxazol-4-ylmethoxy]-cyclohexylmethoxy}-propionsäure. 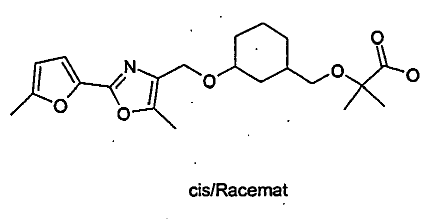 C₂₁H₂₉NO₅ (391,46), MS(ESI): 392 (M + H⁺)

### Beispiel 14:

Analog zu Beispiel 10 erhält man aus 2-(cis-3-Hydroxycyclohexylmethoxy)-2-methylpropionsäure-tert-butylester und 2-(3,4-Dimethoxy-phenyl)-5-methyl-oxazol-4-ylmeth yliodid 2-{(1 R,3S)-cis-3-[2-(3,4-Dimethoxy-phenyl)-5-methyl-oxazol-4-ylmethoxy]-cyclohexylmethoxy}-2-methyl-propionsäure. 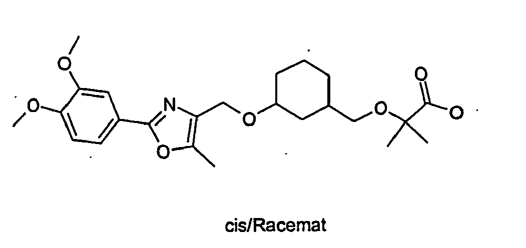 C₂₄H₃₃NO₇ (447,53), MS(ESI): 448 (M + H⁺)

### Beispiel 15:

Analog zu Beispiel 10 erhält man aus 2-(cis-3-Hydroxycyclohexylmethoxy)-2-methylpropionsäure-tert-butylester und 5-Phenyl-2-p-tolyl-oxazol-4-ylmethyliodid 2-Methyl-2-[(1R,3S)-cis-3-(5-phenyl-2-p-tolyl-oxazol-4-ylmethoxy)-cyclohexylmethoxy]-propionsäure 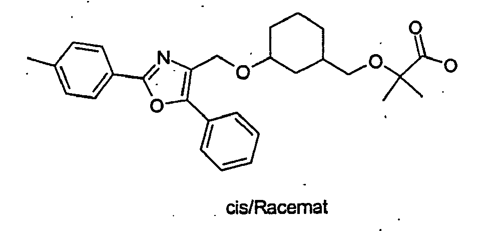 C₂₈H₃₃NO₅ (463,57), MS(ESI): 464 (M + H⁺)

### Beispiel 16:

Analog zu Beispiel 10 erhält man aus 2-(cis-3-Hydroxycyclohexylmethoxy)-2-methylpropionsäure-tert-butylester und 5-Methyl-2-(4-trifluoromethyl-phenyl)-oxazol-4-ylmethyliodid 2-Methyl-2-{(1R,3S)-cis-3-[5-methyl-2-(4-trifluoromethyl-phenyl)-oxazol-4-ylmethoxy]-cyclohexylmethoxy}-propionsäure 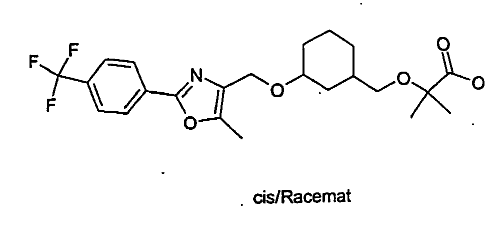 C₂₃H₂₈F₃NO₅ (455,47), MS(ESI): 456 (M + H⁺)

### Beispiel 17:

Analog zu Beispiel 10 erhält man aus 2-(cis-3-Hydroxycyclohexylmethoxy)-2-methylpropionsäure-tert-butylester und 2-(4-Methoxy-phenyl)-5-methyl-oxazol-4-ylmethyliodid 2-{(1R,3S)-cis-3-[2-(4-Methoxy-phenyl)-5-methyl-oxazol-4-ylmethoxy]-cyclohexylmethoxy}-2-methyl-propionsäure 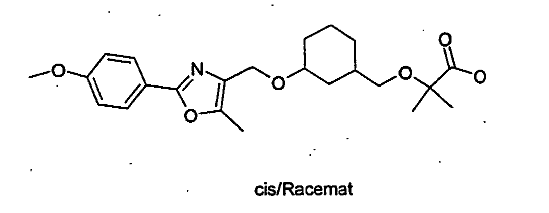 C₂₃H₃₁NO₆ (417,50), MS(ESI): 418 (M + H⁺)

### Beispiel 18:

Analog zu Beispiel 10 erhält man aus 2-(cis-3-Hydroxycyclohexylmethoxy)-2-methylpropionsäure-tert-butylester und 5-Methyl-2-thiophen-2-yl-oxazol-4-ylmethyliodid 2-Methyl-2-[(1R,3S)-cis-3-(5-methyl-2-thiophen-2-yl-oxazol-4-ylmethoxy)-cyclohexylmethoxy]-propionsäure 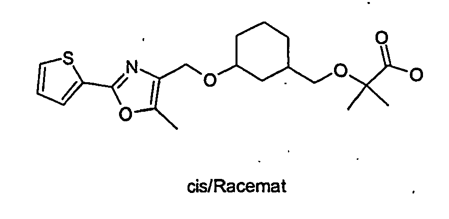 C₂₀H₂₇NO₅S (393,50), MS(ESI): 394 (M + H⁺)

### Beispiel 19:

Analog zu Beispiel 10 erhält man aus 2-(cis-3-Hydroxycyclohexylmethoxy)-2-methylpropionsäure-tert-butylester und -methyl-2-(3-trifluoromethoxy-phenyl)-oxazol-4-ylmethyliodid 2-Methyl-2-[(1R,3S)-cis-3-[5-methyl-2-(3-trifluoromethoxy-phenyl)-oxazol-4-ylmethoxy]-cyclohexylmethoxy]-propionsäure 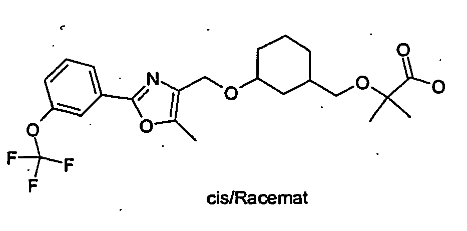 C₂₃H₂₈F₃NO₆ (471,47), MS(ESI): 472 (M + H⁺)

### Beispiel 20:

Analog zu Beispiel 10 erhält man aus 2-(cis-3-Hydroxycyclohexylmethoxy)-2-methylpropionsäure-tert-butylester und 5-methyl-2-(4-Isopropyl-phenyl)-oxazol-4-ylmethyliodid 2-Methyl-2-[(1R,3S)-cis-3-(5-methyl-2-(4-Isopropyl-phenyl)-oxazol-4-ylmethoxy)-cyclohexylmethoxy]-propionsäure. 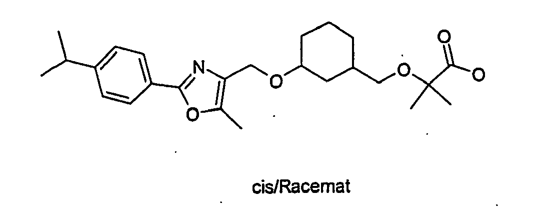 C₂₅H₃₅NO₅ (429,56), MS(ESI): 430 (M + H⁺)

### Beispiel 21:

Analog zu Beispiel 10 erhält man aus 2-(cis-3-Hydroxycyclohexylmethoxy)-2-methylpropionsäure-tert-butylester und 5-methyl-2-m-toluyl-oxazol-4-ylmethyliodid 2-Methyl-2-[(1R,3S)-cis-3-(5-methyl-2-m-toluyl-oxazol-4-ylmethoxy)-cyclohexylmethoxy]-propionsäure. 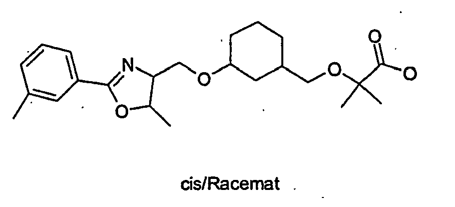 C₂₃H₃₁NO₅ (401,50), MS(ESI): 402 (M + H⁺)

### Beispiel 22:

### cis-3-(5-Methyl-2-p-tolyloxazol-4-ylmethoxy)-cyclohexylmethylsulfanyl]-essigsäure:

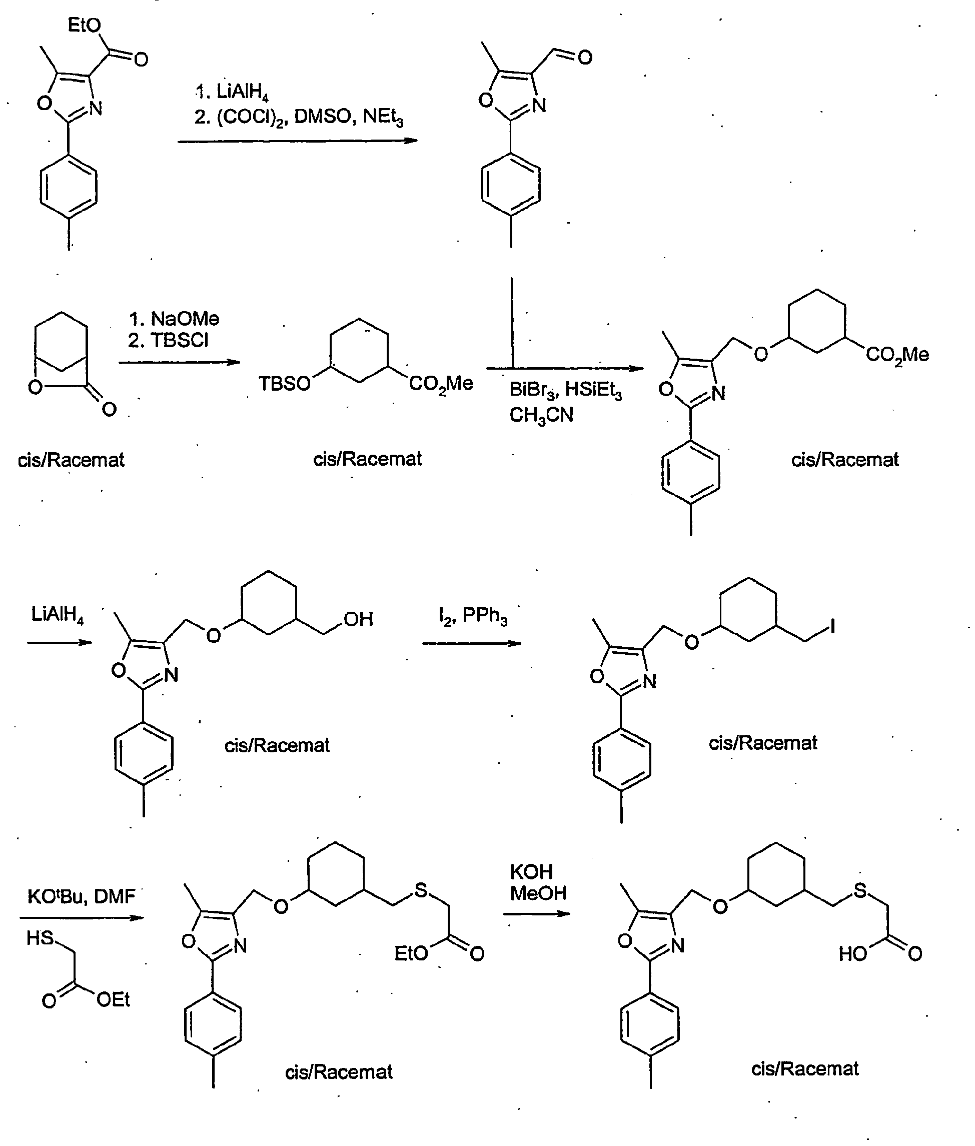

### 5-Methyl-2-p-tolyl-oxazol-4-carboxaldehyd:

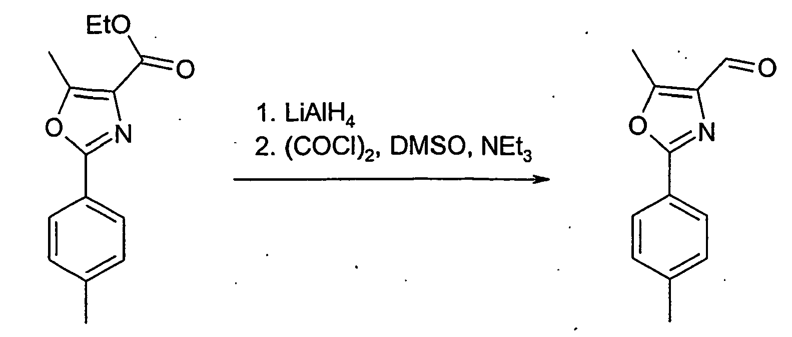

9,3 g LiAlH4 werden in einem trockenen Vierhalskolben mit Rührmotor, Innenthermometer, Tropftrichter mit Druckausgleich und Rückflusskühler mit Argonzuleitung (Absaugstück mit Hahn) mit 600 ml Diethylether überschichtet. Die Suspension wird auf 0°C gekühlt. 30 g 5-Methyl-2-p-tolyl-oxazol-4-carbonsäüreethylester werden in 100 ml Diethylether gelöst und der Suspension tropfenweise zugegeben. Nach einstündigem Rühren bei Raumtemperatur ist die Reaktion beendet (DC(Heptan/Ethylacetat 1/1): R_{f,Edukt} = 0.66, R_{f,Produkt} = 0.18). 80 g MgSO₄, 300 ml Methyl-tert-butylether und 30 ml Ethylacetat werden nacheinander zugegeben, und die Suspension wird bei Raumtemperatur gerührt. Dann wird die Mischung auf 0°C gekühlt, tropfenweise mit 90 ml 10N KOH versetzt und weitere 60 min gerührt. Die Feststoffe werden abfiltriert, der Rückstand dreimal mit Ethylacetat gewaschen, und das Filtrat wird eingeengt, wobei 24 g 5-Methyl-2-p-tolyl-oxazol-4-methanol als gelber Feststoff erhalten werden. C12H13NO2 (203,24), LCMS(ESI): 204.1 (MH⁺).
Zu einer Lösung von 12 ml Oxalylchlorid in 150 ml Dichlormethan werden bei -78 °C 22.2 ml DMSO in 30 ml Dichlormethan so zugetropft, dass die Temperatur nicht über-70 °C steigt. Anschließend wird die Lösung 30 min bei dieser Temperatur gerührt. Dann werden 24 g 5-Methyl-2-p-tolyl-oxazol-4-methanol in 120 ml Dichlormethan/Chloroform (2/1) zugetropft, wobei die Temperatur nicht über -70 °C steigt. Die Lösung wird 30 min bei dieser Temperatur gerührt. Anschließend werden 80 ml NEt₃ so zugetropft, dass die Temperatur nicht über-70 °C steigt. Nach beendeter Zugabe wird das Kühlbad entfernt und die Lösung unter Rühren auf 0 °C gebracht. Bei dieser Temperatur werden 100 ml Wasser zugegeben, und die Mischung wird heftig bei Raumtemperatur gerührt. Die wäßrige Phase wird abgetrennt und mit Chloroform extrahiert. Die vereinigten organischen Phasen werden mit gesättigter NH₄Cl-Lösung gewaschen, über MgSO₄ getrocknet und eingeengt, wobei 23,8 g 5-Methyl-2-p-tolyl-oxazol-4.-carbaldehyd als gelber Feststoff erhalten werden. C12H11NO2 (201,23), LCMS(ESI): 202,1 (MH⁺).

### [cis-3-(tert-Butyldimethylsilanyloxy)-cyclohexyl]-carbonsäuremethylester:

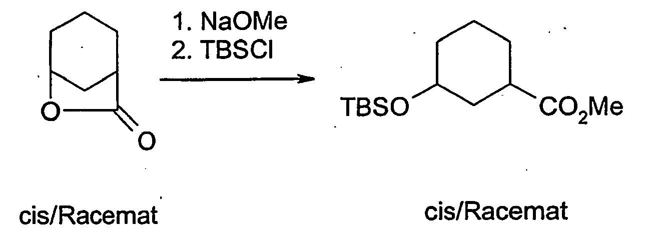

47 g 6-Oxa-bicyclo[3.2.1]octan-7-on werden in 500 ml MeOH gelöst und mit 40,5 g NaOMe versetzt. Nach 2,5-stündigem Rühren bei Raumtemperatur werden 135 ml Essigsäure zugegeben und das Methanol weitgehend abdestilliert. Der Rückstand wird mit Ethylacetat/Wasser aufgenommen, und die Phasen werden getrennt. Die wäßrige Phase wird mit Ethylacetat extrahiert, die vereinigten organischen Phasen werden über MgSO4 getrocknet und eingeengt, wobei der Methylester quantitativ als Rückstand erhalten wird.
10,7 g des Rückstandes werden in 100 ml Dimethylformamid gelöst und mit 11,2 g tert-Butyldimethylsilylchlorid versetzt. Bei 0 °C werden 11,5 g Imidazol zugegeben und die Lösung wird bei Raumtemperatur über Nacht gerührt. 200 ml gesättigte NaCl-Lösung werden zugesetzt und die Lösung wird dreimal mit Methyl-tert-butylether extrahiert. Die vereinigten organischen Phasen werden mit gesättigter NaCl-Lösung gewaschen, über MgSO4 getrocknet und eingeengt. 16,4 g [cis-3-(tert-Butyldimethylsilanyloxy)-cyclohexyl]-carbonsäuremethylester werden als farbloses Öl erhalten. C14H28O3Si (272,46), MS(ESI): 273,13 (MH⁺).

### cis-3-(5-Methyl-2-p-tolyloxazol-4-ylmethoxy)-cyclohexancarbonsäuremethylester:

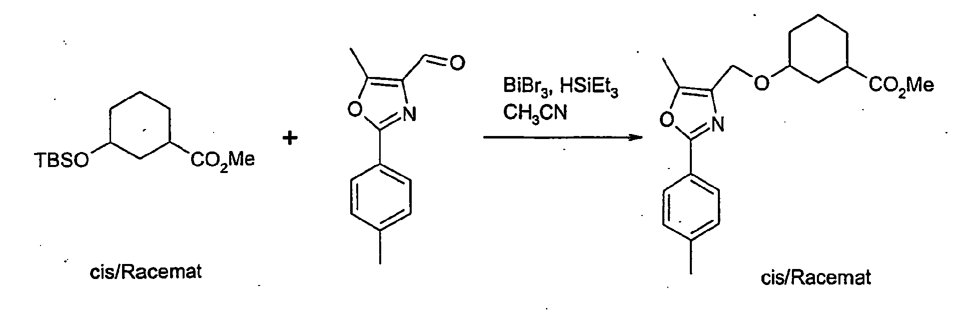

1,35 g [cis-3-(tert-Butyldimethylsilanyloxy)-cyclohexyl]-essigsäuremethylester werden bei Raumtemperatur zu einer Mischung von 4,0 ml HSiEt3 und 1,50 g BiBr3 in 20 ml Acetonitril getropft. Anschließend werden 1,51 g 5-Methyl-2-p-tolyl-oxazol-4-carbaldehyd in 5 ml Acetonitril zugegeben und die Mischung 4 h bei Raumtemperatur gerührt. Die Suspension wird filtriert und eingeengt. Der Rückstand wird an Kieselgel chromatographiert (Dichlormethan/Methanol 100/1), wobei 1,20 g cis-3-(5-Methyl-2-p-tolyloxazol-4-ylmethoxy)-cyclohexancarbonsäuremethylester als hellgelbes Öl erhalten werden. C20H25NO4 (343,43), LCMS(ESI): 344,1 (MH⁺).

### cis-3-(5-Methyl-2-p-tolyl-oxazol-4-ylmethoxy)-cyclohexyl]-methanol:

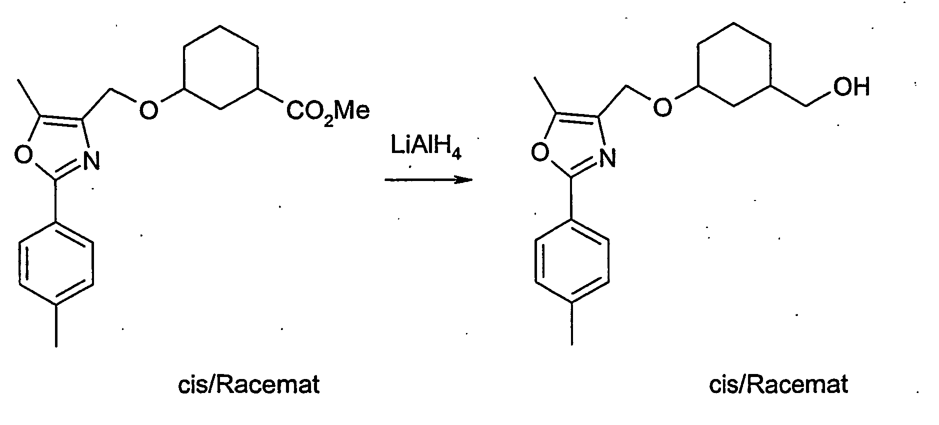

1,70 g cis-3-(5-Methyl-2-p-tolyloxazol-4-ylmethoxy)-cyclohexancarbonsäuremethylester in 5 ml Tetrahydrofuranwerden bei 0 °C zu einer Suspension von 380 mg LiAlH4 in 50 ml Diethylether getropft und 2 h bei Raumtemperatur gerührt. 3 g MgSO₄, 30 ml Methyl-tert-butylether und 3 ml Ethylacetat werden nacheinander zugegeben und die Suspension bei Raumtemperatur gerührt. Dann wird die Mischung auf 0°C gekühlt, tropfenweise mit 1 ml 10N KOH versetzt und weitere 60 min gerührt. Die Feststoffe werden abfiltriert, der Rückstand dreimal mit Ethylacetat gewaschen, und das Filtrat wird eingeengt, wobei 1,55 g cis-3-(5-Methyl-2-p-tolyl-oxazol-4-ylmethoxy)-cyclohexyl]-methanol als gelbes Öl erhalten werden.
C19H25NO3 (315,42), MS(EI): 315.4 (M⁺).

### 4-(cis-3-lodmethylcyclohexyloxymethyl)-5-methyl-2-p-tolyl-oxazol:

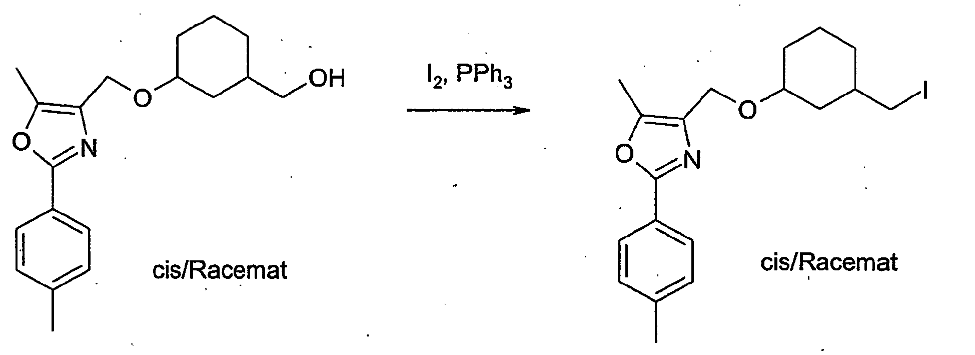

Zu 1,55 g cis-3-(5-Methyl-2-p-tolyl-oxazol-4-ylmethoxy)-cyclohexyl]-methanol in 20 ml Toluol werden 1,56 g PPh3, 0,87 g Imidazol und 1,64 g Iod gegeben und die Mischung 2 h bei Raumtemperatur gerührt. Dann werden 10 ml Dichlormethan zugesetzt und weitere 60 min gerührt. Die Lösung wird mit 50 ml Wasser
und 50 ml Methyl-tert-butylether verdünnt, die Phasen werden getrennt, die organische Phase wird über MgSO4 getrocknet und eingeengt. Filtration des Rückstandes über Kieselgel mir Dichlormethan liefert 1,12 g 4-(cis-3-lodmethylcyclohexyloxymethyl)-5-methyl-2-p-tolyl-oxazol als gelben Feststoff. C19H24INO2 (425,31); LCMS (ESI): 426.0 (MH⁺).

### cis-3-(5-Methyl-2-p-tolyloxazol-4-ylmethoxy)-cyclohexylmethylsulfanyl]-essigsäureethylester:

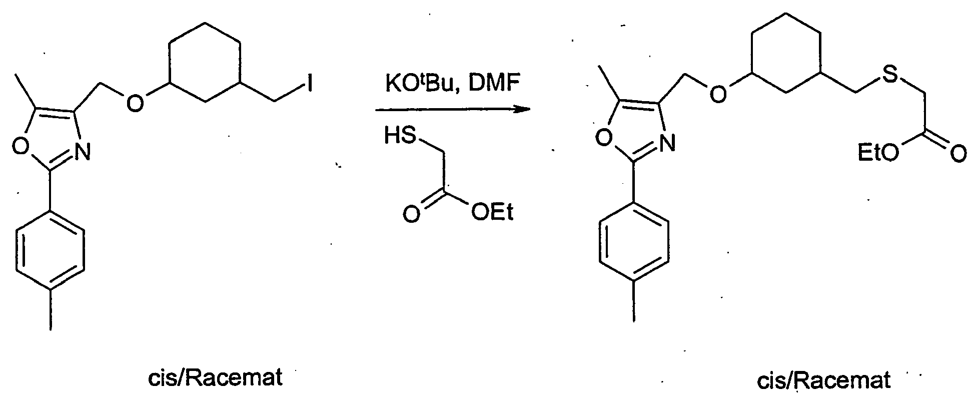

68 mg Mercaptoessigsäureethylester in 1,5 ml Dimethylformamid werden mit 50 mg KO^{t}Bu versetzt und 1 h bei Raumtemperatur gerührt. Anschließend werden 120 mg 4-(cis-3-lodmethylcyclohexyloxymethyl)-5-methyl-2-p-tolyt-oxazol zugegeben und die Lösung wird bei Raumtemperatur gerührt. Nach 1 h werden 20 ml Methyl-tert-butylether, 15 ml gesättigte NaCl-Lösung und 15 ml Wasser zugegeben und die Phasen getrennt. Die wäßrige Phase wird mit Methyl-tert-butylether extrahiert, die vereinigten organischen Phasen werden mit gesättigter NaCl-Lösung gewaschen, über MgSO4 getrocknet und eingeengt, wobei 117 mg cis-3-(5-Methyl-2-p-tolyloxazol-4-ylmethoxy)-cyclohexylmethylsulfanyl]-essigsäureethylester erhalten werden.
C23H31NO4S (417,57); LCMS (ESI): 418,1 (MH⁺).

### cis-3-(5-Methyl-2-p-tolyloxazol-4-ylmethoxy)-cyclohexylmethylsulfanyl]-essigsäure:

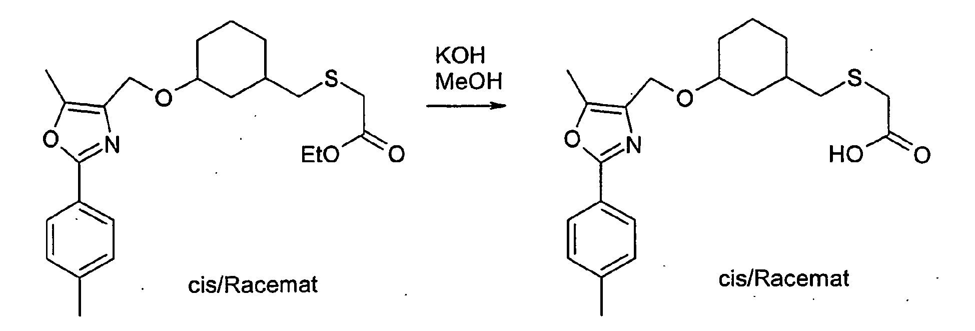

117 mg cis-3-(5-Methyl-2-p-tolyloxazol-4-ylmethoxy)-cyclohexylmethylsulfanyl]-essigsäureethylester in 3 ml Methanol gelöst werden mit 1 ml 2N KOH versetzt und bei Raumtemperatur über Nacht gerührt. Dann werden 2 ml 2N HCl, 10 ml gesättigte Nacl-Lösung, 5 ml Wasser und 20 ml Dichlormethan zugesetzt und die Phasen getrennt. Die organische Phase wird über MgSO4 getrocknet und eingeengt, wobei 100 mg cis-3-(5-Methyl-2-p-tolyloxazol-4-yimethoxy)-cyclohexylmethylsulfanyl]-essigsäure erhalten werden. C21H27NO4S (389,52); LCMS(ESI): 390,1 (MH⁺).

### Beispiel 23:

Analog zu Beispiel 22 erhält man aus 4-(cis-3-lodmethylcyclohexyloxymethyl)-5-methyl-2-p-tolyl-oxazol und 2-Mercaptopropionsäureethylester 2-[cis-3-(5-Methyl-2-p-tolyloxazol-4-ylmethoxy)-cyclohexylmethylsulfanyl]-propionsäure als Diastereomerengemisch. C22H29NO4S (403,54), LCMS(ESI): 404,1 (MH⁺).

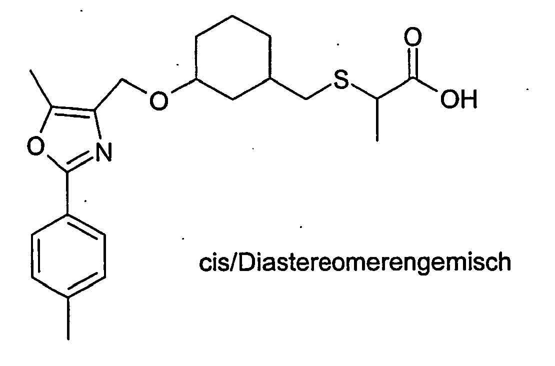

### Beispiel 24:

Analog zu Beispiel 22 erhält man aus 4-(cis-3-lodmethylcyclohexyloxymethyl)-5-methyl-2-p-tolyl-oxazol und 2-Mercaptobuttersäuremethylester 2-[cis-3-(5-Methyl-2-p-tolyloxazol-4-ylmethoxy)-cyclohexylmethylsulfanyl]-buttersäure als Diastereomerengemisch. C23H31NO4S (417,57), LCMS(ESI): 418,1 (MH⁺).

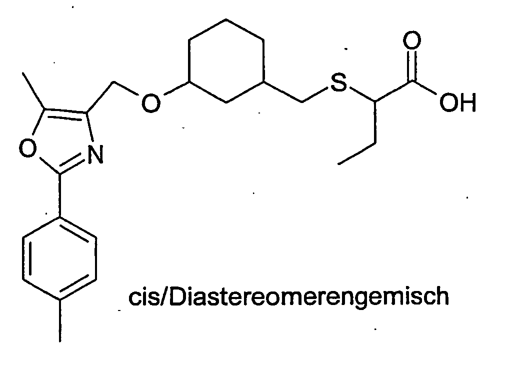

### Beispiel 25:

Analog zu Beispiel 22 erhält man aus 4-(cis-3-iodmethylcyclohexyloxymethyl)-5-methyl-2-p-tolyl-oxazol und 2-Mercaptoheptansäureethylester 2-[cis-3-(5-Methyl-2-p-tolyloxazol-4-ylmethoxy)-cyclohexylmethylsulfanyl]-heptansäure als Diastereomerengemisch. C26H37NO4S (459,65), MS(ESI): 460,41 (MH⁺).

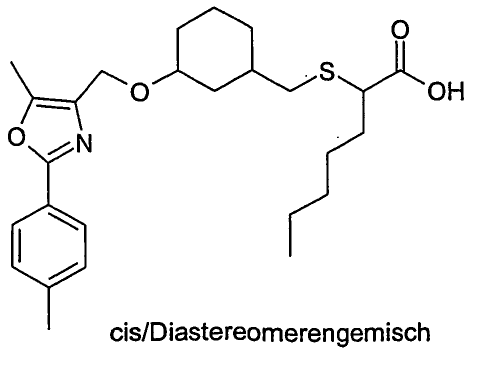

### Beispiel 26:

Analog zu Beispiel 22 erhält man aus 4-(cis-3-lodmethylcyclohexyloxymethyl)-5-methyl-2-p-tolyl-oxazol und 2-Mercapto-3-methylbuttersäuresäureethylester 2-[cis-3-(5-Methyl-2-p-tolyloxazol-4-ylmethoxy)-cyclohexylmethylsulfanyl]-3-methylbuttersäure als Diastereomerengemisch. C24H33NO4S (431,60), LCMS(ESI): 432.2 (MH⁺).

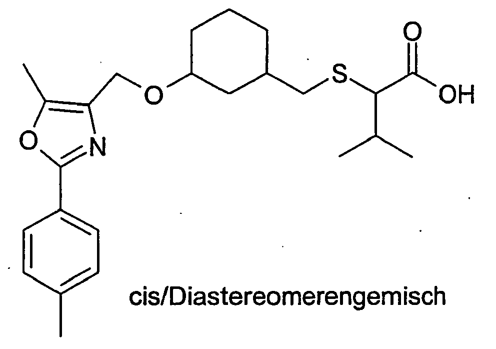

### Beispiel 27:

Analog zu Beispiel 22 erhält man aus 4-(cis-3-lodmethylcyclohexyloA-ymethyl)-5-methyl-2-p-tolyl-oxazol und 2-Mercapto-2-methylpropionsäuresäureethylester 2-[cis-3-(5-Methyl-2-p-tolyloxazol-4-ylmethoxy)-cyclohexylmethylsulfanyl]-2-methylpropionsäure. C23H31NO4S (417,57), LCMS(ESI): 418,1 (MH⁺).

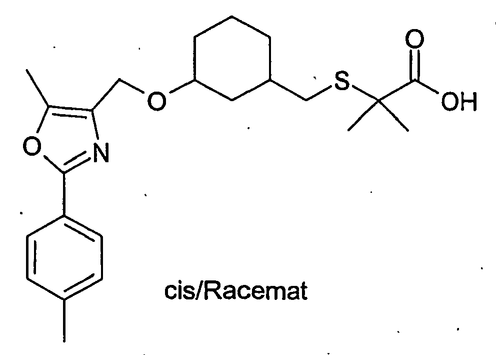

### Beispiel 28:

Analog zu Beispiel 22 erhält man aus 4-(cis-3-lodmethylcyclohexyloxymethyl)-5-methyl-2-p-tolyl-oxazol und 2-Mercapto-2-phenylessigsäureethylester 2-[cis-3-(5-Methyl-2-p-tolyloxazol-4-ylmethoxy)-cyclohexylmethylsulfanyl]-2-phenylessigsäure als Diastereomerengemisch. C27H31NO4S (465,62), MS(ESI): 466,39 (MH⁺).

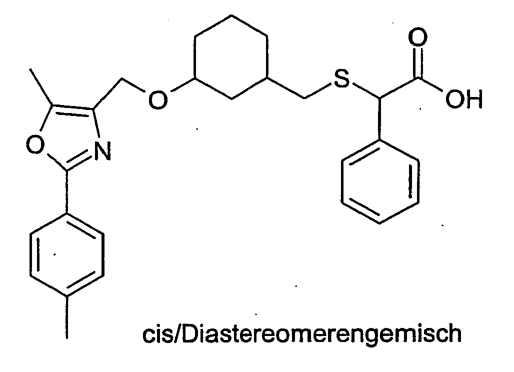

### Beispiel 29:

Analog zu Beispiel 22 erhält man aus 4-(cis-3-lodmethylcyclohexyloxymethyl)-5-methyl-2-p-tolyl-oxazol und 2-Mercapto-2-cyclohexylessigsäureethylester 2-[cis-3-(5-Methyl-2-p-tolyloxazol-4-ylmethoxy-cyclohexylmethylsulfanyl]-2-cyclohexylessigsäure als Diastereomerengemisch. C27H37NO4S (471,66), LCMS(ESI): 472,2 (MH⁺).

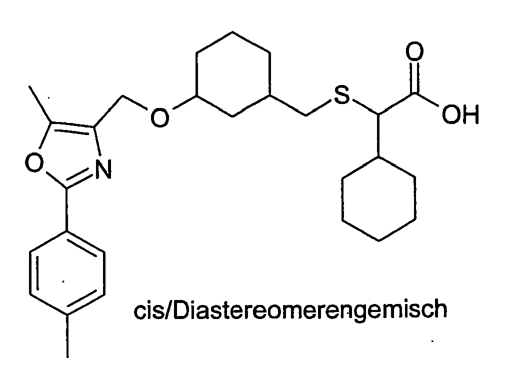

### Beispiel 30:

Analog zu Beispiel 22 erhält man aus 4-(cis-3-lodmethylcyclohexyloxymethyl)-5-methyl-2-p-tolyl-oxazol und 2-Mercaptovaleriansäureethylester 2-[cis-3-(5-Methyl-2-p-tolyloxazol-4-ylmethoxy)-cyclohexylmethylsulfanyl]-valeriansäure als Diastereomerengemisch. C24H33NO4S (431,60), MS(ESI): 432,39 (MH⁺).

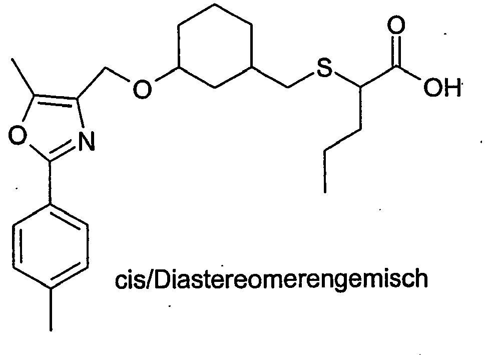

### Beispiel 31:

Analog zu Beispiel 22 erhält man aus 4-(cis-3-Iodmethylcyclohexyloxymethyl)-5-methyl-2-p-tolyl-oxazol und 1-Mercaptocyclobutancarbonsäureethylester 1-[cis-3-(5-Methyl-2-p-tolyloxazol-4-ylmethoxy)-cyclohexylmethylsulfanyl]-cyclobutancarbonsäure. C24H31NO4S (429,58), MS(ESI): 430,35
(MH⁺).

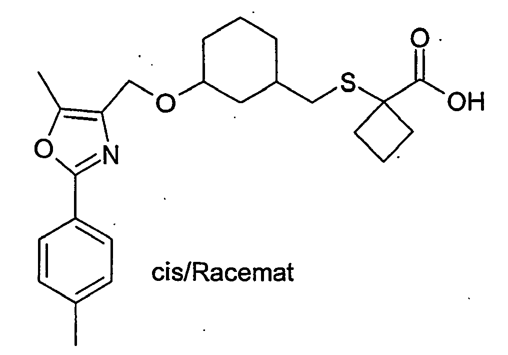

### Bausteinsynthese der 2-Mercaptobuttersäuremethylester

### 2-Mercaptobuttersäuremethylester

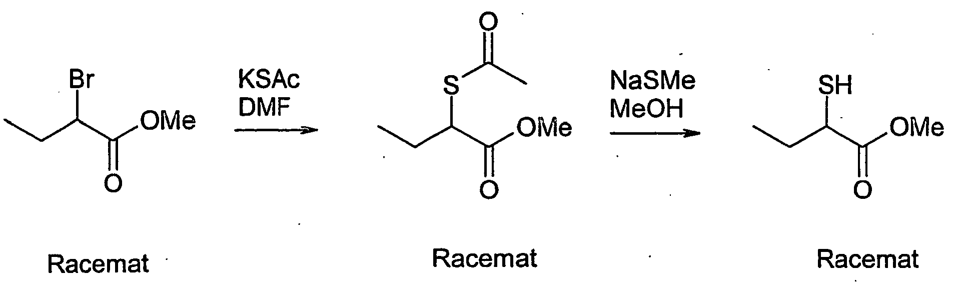

Zu 1,81 g 2-Brombuttersäuremethylester in 5 ml Dimethylformamid werden 1,43 g KSAc gegeben und die Mischung 12 h bei Raumtemperatur gerührt. Dann werden 25 ml Methyl-tert-butylether, 10 ml Wasser und 15 ml gesättigte NaCl-Lösung zugesetzt und die Phasen getrennt. Die wäßrige Phase wird mit Methyl-tert-butylether extrahiert, die vereinigten organischen Phasen werden mit gesättigter NaCl-Lösung gewaschen, über MgSO4 getrocknet und eingeengt, wobei 2-Acetylsulfanylbuttersäuremethylester als gelbes Öl erhalten werden. Dieses wird in 10 ml Methanol aufgenommen und mit 11 ml einer 1M NaSMe Lösung in Methanol versetzt und über Nacht bei Raumtemperatur gerührt. Das Lösungsmittel wird im Vakuum vollständig abdestilliert, der Rückstand mit 15 ml Methyl-tert-butylether und 20 ml Wasser aufgenommen, die Phasen werden getrennt, die organische Phase mit gesättigter Nacl-Lösung gewaschen und über MgSO4 getrocknet. Nach Einengen der Lösung im Vakuum werden 1,30 g 2-Mercaptobuttersäuremethylester als gelbes Öl erhalten.

Analog zu Bausteinsynthese der 2-Mercaptobuttersäuremethylester erhält man aus 2-Bromheptansäureethylester 2-Mercaptoheptansäureethylester.

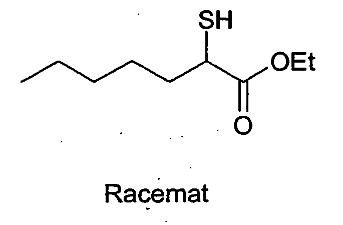

Analog zu Bausteinsynthese der 2-Mercaptobuttersäuremethylester erhält man aus 2-Brom-3-methylbuttersäuresäureethylester 2-Mercapto-3-methylbuttersäuresäureethylester.

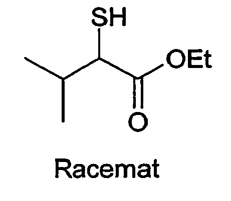

Analog zur Bausteinsynthese der 2-Mercaptobuttersäuremethylester erhält man aus 2-Brom-2-methylpropionsäuresäureethylester 2-Mercapto-2-methylpropionsäuresäureethylester.

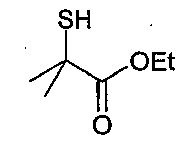

Analog zu Bausteinsynthese der 2-Mercaptobuttersäuremethylester erhält man aus 2-Brom-2-phenylessigsäureethylester 2-Mercapto-2-phenylessigsäureethylester.

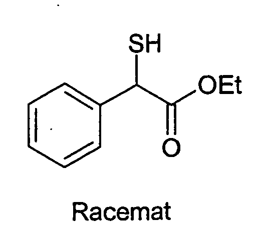

Analog zu Bausteinsynthese der 2-Mercaptobuttersäuremethylester erhält man aus 2-Brom-2-cyclohexylessigsäuremethylester 2-Mercapto-2-cyclohexylessigsäuremethylester.

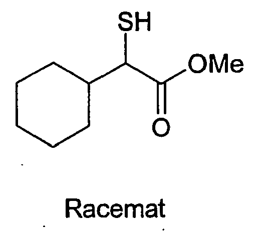

Analog zu Bausteinsynthese der 2-Mercaptobuttersäuremethylester erhält man aus 2-Bromvaleriansäureethylester 2-Mercaptovaleriansäureethylester.

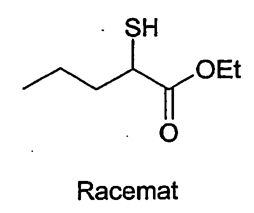

Analog zu Bausteinsynthese der 2-Mercaptobuttersäuremethylester erhält man aus 1-Bromcyclobutancarbonsäureethylester 1-Mercaptocyclobutancarbonsäureethylester.

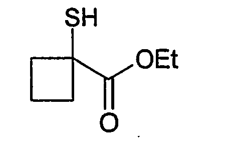

### Beispiel 32:

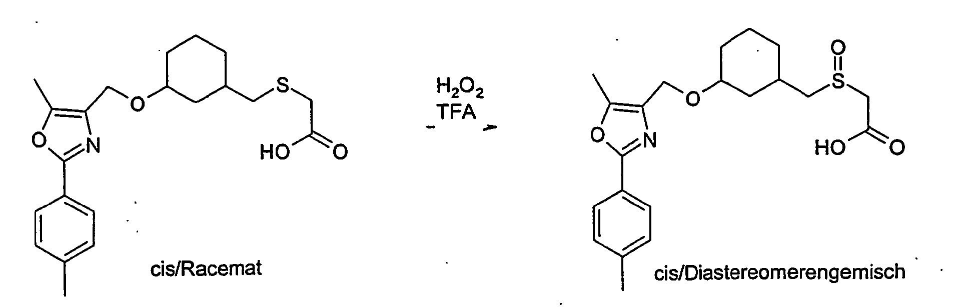

cis-3-(5-Methyl-2-p-tolyloxazol-4-ylmethoxy)-cyclohexylmethylsulfinyl]-essigsäure:

65 mg cis-3-(5-Methy)-2-p-tolytoxazol-4-ylmethoxy)-cyclohexylmethylsulfanyl]-essigsäure werden in 1,5 ml Trifluoressigsäure gelöst, bei 0 °C mit 6,3 µl 35% H2O2 versetzt und über Nacht bei Raumtemperatur gerührt. Gesättigte NH4Cl-Lösung und Methyl-tert-butylether werden zugegeben, die Phasen getrennt, die wäßrige Phase mit Methyl-tert-butylether extrahiert, die vereinigten organischen Phasen mit gesättigter NaCl-Lösung gewaschen, über MgSO4 getrocknet und eingeengt. Der Rückstand wird per HPLC gereinigt, wobei 6,6 mg eines farblosen Feststoffs erhalten wurden. C21H27NO5S (405,52), LCMS(ESI): 406,1 (MH⁺).

### Beispiel 33:

Analog zu Beispiel 32 erhält man aus 2-[cis-3-(5-Methyl-2-p-tolyloxazol-4-ylmethoxy)-cyclohexylmethylsulfanyl]-heptansäure 2-[cis-3-(5-Methyl-2-p-tolyloxazol-4-ylmethoxy)-cyclohexylmethylsulfinyl]-heptansäure als Diastereomerengemisch. C26H37NO5S (475,65), MS(ESI): 476,18 (MH⁺).

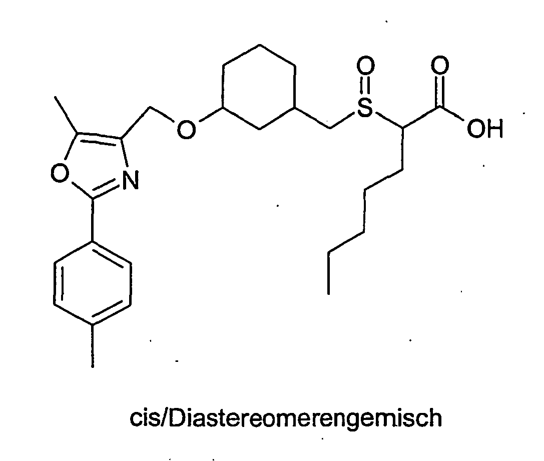

### Beispiel 34:

Analog zu Beispiel 32 erhält man aus 2-[cis-3-(5-Methyl-2-p-tolyloxazol-4-ylmethoxyl)-cyclohexylmethylsulfanyl]-2-methylpropionsäure 2-[cis-3-(5-Methyl-2-p-tolyloxazol-4-ylmethoxy)-cyclohexylmethylsulfinyl]-2-methylpropionsäure. C23H31NO5S (433,57), LCMS(ESI): 434,1 (MH⁺).

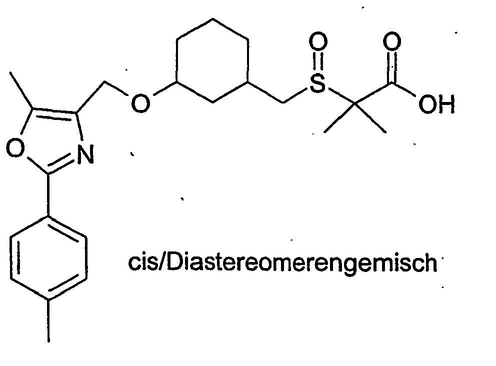

### Beispiel 35:

Analog zu Beispiel 32 erhält man aus 2-[cis-3-(5-Methyl-2-p-tolyloxazol-4-ylmethoxy)-cyclohexylmethylsulfanyl]-3-methylbuttersäure 2-[cis-3-(5-Methyl-2-p-tolyloxazol-4-ylmethoxy)-cyclohexylmethylsulfinyl]-3-methylbuttersäure als Diastereomerengemisch. C24H33NO5S (447,60), MS(ESI): 448,43 (MH⁺).

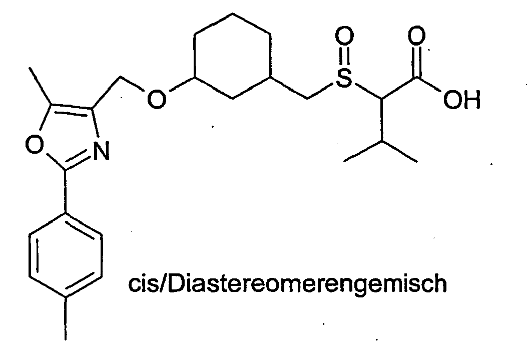

### Beispiel 36:

Analog zu Beispiel 32 erhält man aus 2-[cis-3-(5-Methyl-2-p-tolyloxazol-4-ylmethoxy)-cyclohexylmethylsulfanyl]-valeriansäure 2-[cis-3-(5-Methyl-2-p-tolyloxazol-4-ylmethoxy)-cyclohexylmethylsulfinyl]-valeriansäure als Diastereomerengemisch.
C24H33NO5S (447,60), MS(ESI): 448,14 (MH⁺).

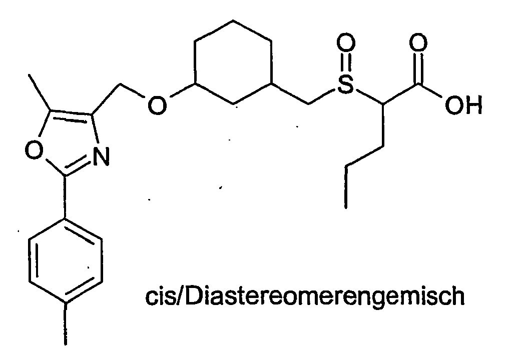

### Beispiel 37:

cis-3-(5-Methyl-2-p-tolyloxazol-4-ylmethoxy)-cyclohexylmethylsulfonyl]-essigsäure:

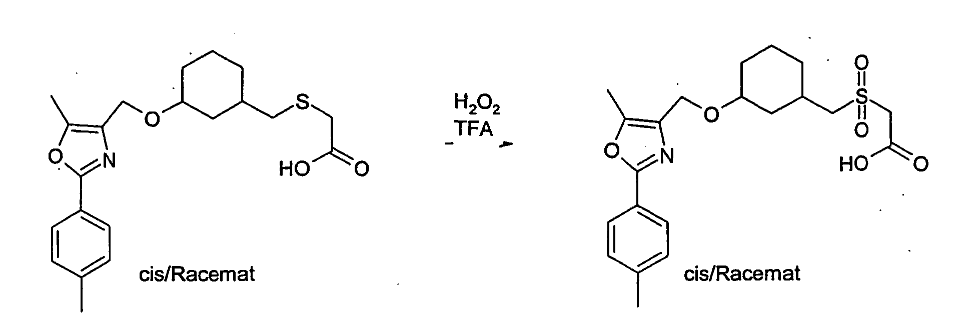

65 mg cis-3-(5-Methyl-2-p-tolyloxazol-4-ylmethoxy)-cyclohexylmethylsulfanyl]-essigsäure werden in 1,5 ml Trifluoressigsäure gelöst, bei 0 °C mit 21,5 µl 35% H2O2 versetzt und über Nacht bei Raumtemperatur gerührt. Gesättigte NH4Cl-Lösung und Methyl-tert-butylether werden zugegeben, die Phasen getrennt, die wäßrige Phase mit Methyl-tert-butylether extrahiert, die vereinigten organischen Phasen mit gesättigter NaCl-Lösung gewaschen, über MgSO4 getrocknet und eingeengt. Der Rückstand wird per HPLC gereinigt, wobei 6,6 mg eines farblosen Feststoffs erhalten wurden.
C21H27NO6S (421,52), LCMS(ESI):422,1 (MH⁺).

### Beispiel 38:

Analog zu Beispiel 37 erhält man aus 2-[cis-3-(5-Methyl-2-p-tolyloxazol-4-ylmethoxy)-cyclohexylmethylsulfanyl]-heptansäure 2-[cis-3-(5-Methyl-2-p-tolyloxazol-4-ylmethoxy)-cyclohexylmethylsulfonyl]-heptansäure als Diastereomerengemisch. C26H37NO6S (491,65), MS(ESI): 492,42 (MH⁺).

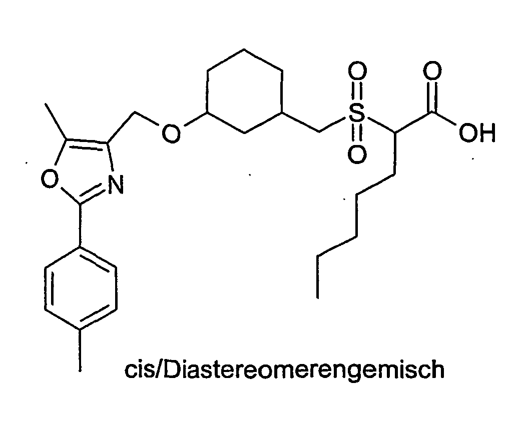

### Beispiel 39:

Analog zu Beispiel 37 erhält man aus 2-[cis-3-(5-Methyl-2-p-tolyloxazol-4-ylmethoxy)-cyclohexylmethylsulfanyl]-2-methylbuttersäure 2-[cis-3-(5-Methyl-2-p-tolyloxazol-4-ylmethoxy)-cyclohexylmethylsulfonyl]-2-methylbuttersäure. C23H31NO6S (449,57), LCMS(ESI): 450,1 (MH⁺).

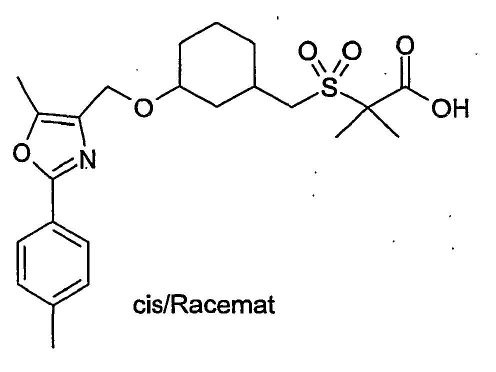

### Beispiel 40:

Analog zu Beispiel 37 erhält man aus 2-[cis-3-(5-Methyl-2-p-tolyloxazol-4-ylmethoxy)-cyclohexylmethylsulfanyl]-3-methylbuttersäure 2-[cis-3-( 5-Methyl-2-p-tolyloxazol-4-ylmethoxy)-cyclohexylmethylsulfonyl]-3-methylbuttersäure als Diastereomerengemisch. C24H33NO6S (463,60), LCMS(ESI): 464,1 (MH⁺).

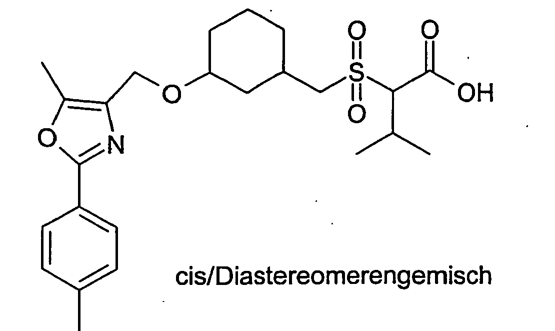

### Beispiel 41:

Analog zu Beispiel 37 erhält man aus 2-[cis-3-(5-Methyl-2-p-tolyloxazol-4-ylmethoxy)-cyclohexylmethylsulfanyl]-valeriansäure 2-[cis-3-(5-Methyl-2-p-tolyloxazol-4-ylmethoxy)-cyclohexylmethylsulfonyl]-valeriansäure als Diastereomerengemisch. C24H33NO6S (463,60), MS(ESI): 464,14 (MH⁺).

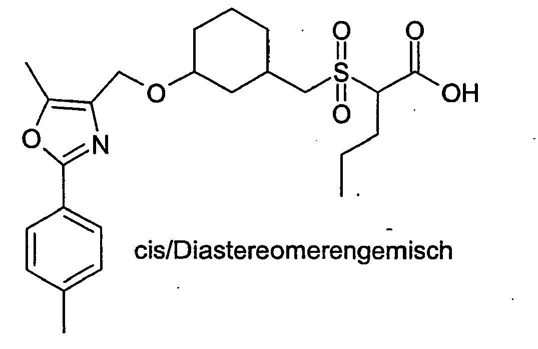

### Beispiel 42:

### (S)-3-Methyl-2-{[cis-3-(5-methyl-2-p-tolyl-oxazol-4-ylmethoxy)-cyclohexylmethyl]-amino}-buttersäure

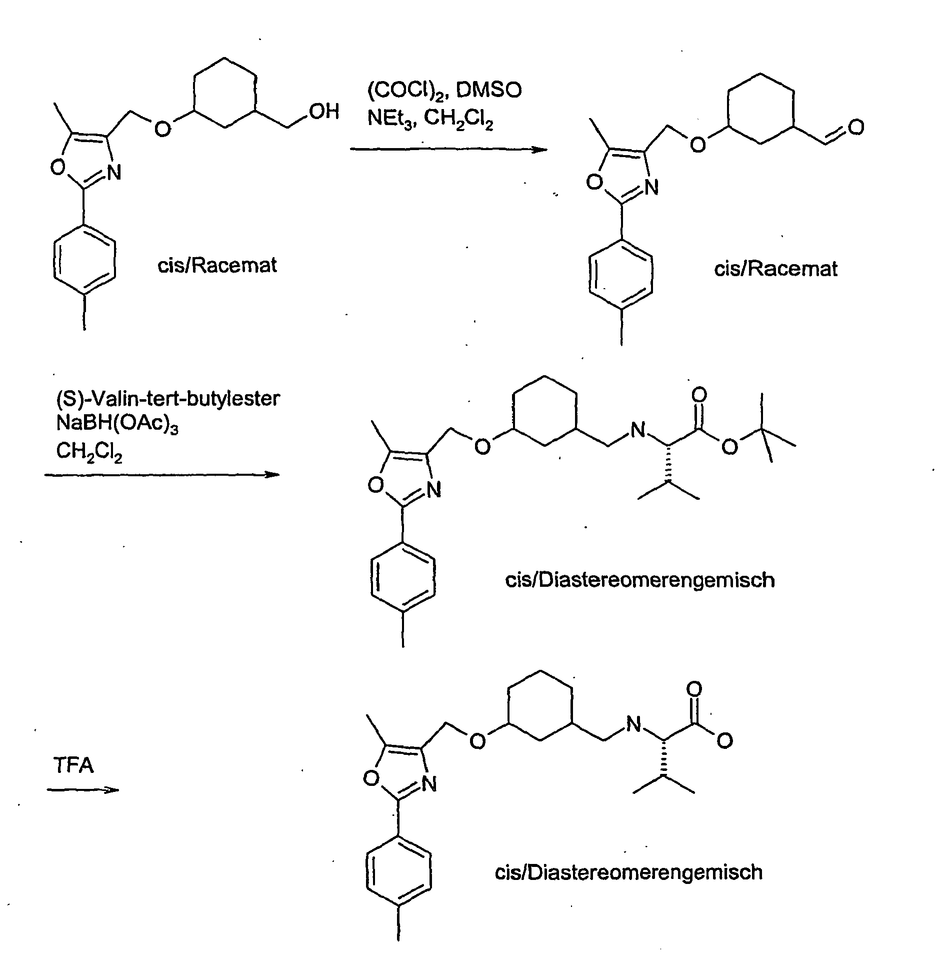

### cis-3-(5-Methyl-2-p-tolyl-oxazol-4-ylmethoxy)-cyclohexancarbaldehyd

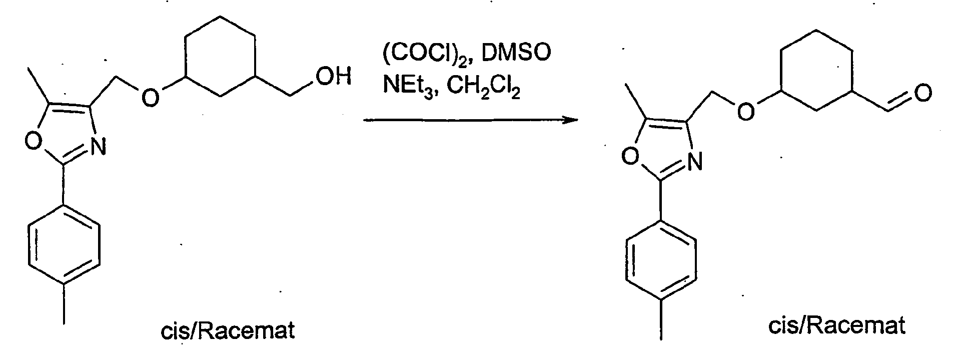

Zu 0,48 ml Oxalylchlorid in 15 ml Dichlormethan werden bei -78 °C 089 ml DMSO in 1 ml Dichlormethan so zugetropft, dass die Temperatur -70 °C nicht übersteigt. Nach beendeter Zugabe wird die Lösung 30 min bei dieser Temperatur gerührt. Dann werden 1,5 g cis-3-(5-Methyl-2-p-tolyl-oxazol-4-ylmethoxy)-cyclohexyl]-methanol in 2 ml Dichlormethan so zugetropft, dass die Temperatur unter-78 °C bleibt. Die Lösung wird 30 min bei dieser Temperatur gerührt. Dann werden 3,2 ml NEt3 zugetropft, das Kühlbad wird entfernt und die Lösung auf 0 °C erwärmt. Bei dieser Temperatur werden 10 ml Wasser zugegeben, und die Mischung wird heftig bei Raumtemperatur gerührt. Die wäßrige Phase wird abgetrennt und mit Dichlormethan extrahiert. Die vereinigten organischen Phasen werden mit gesättigter NH₄Cl-Lösung gewaschen, über MgSO₄ getrocknet und eingeengt, wobei 1,50 g cis-3-(5-Methyl-2-p-tolyl-oxazol-4-ylmethoxy)-cyclohexancarbaldehyd erhalten werden. C19H23NO3 (313,40); LCMS (ESI): 314,1 (MH⁺).

### (S)-3-Methyl-2-{[cis-3-(5-methyl-2-p-tolyl-oxazol-4-ylmethoxy)-cyclohexylmethyl] amino}-buttersäure-tert-butylester

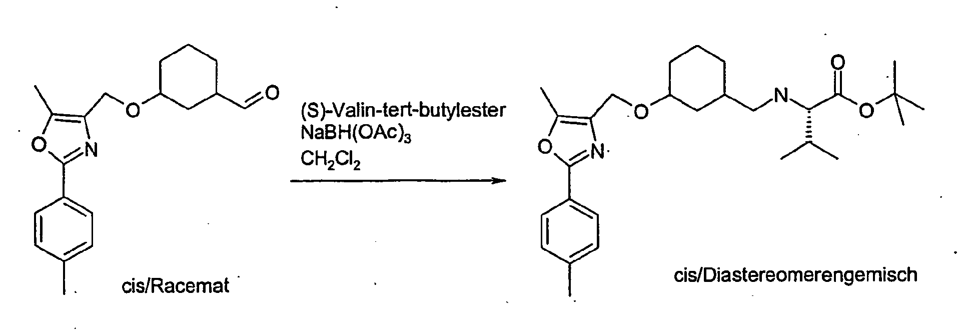

511 mg cis-3-(5-Methyl-2-p-tolyl-oxazol-4-ylmethoxy)-cyclohexancarbaldehyd, 0,9 ml HOAc und 310 mg (S)-Valin-tert-butylester werden in 5 ml abs. Dichlormethan gelöst. Dann werden 500 mg Molsieb 4 Å zugegeben und die Suspension wird auf 0°C gekühlt. 414 mg Natriumtriacetoxyborhydrid werden portionsweise zugesetzt. Dies Suspension wird 2 h bei 0°C gerührt, dann mit 3 ml gesättigter NH4Cl-Lösung versetzt und weitere 10 min gerührt. Jeweils 10 ml Wasser und Dichlormethan werden zugegeben, die Phasen werden getrennt, die wäßrige Phase wird mit Dichlormethan extrahiert, und die vereinigten organischen Phasen werden über MgSO4 getrocknet und eingeengt, wobei 760 mg (S)-3-Methyl-2-{[cis-3-(5-methyl-2-p-tolyl-oxazol-4-ylmethoxy)-cyclohexylmethyl]-amino}-buttersäure-tert-butylester erhalten werden.
C28H42N2O4 (470,66); MS (ESI): 471.50 (MH⁺).

### (S)-3-Methyl-2-{[cis-3-(5-methyl-2-p-tolyl-oxazol-4-ylmethoxy)-cyclohexylmethyl]-amino}-buttersäure

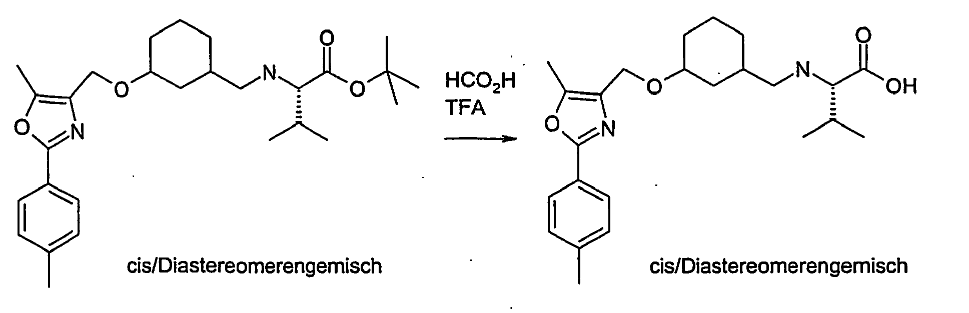

40 mg (S)-3-Methyl-2-{[cis-3-(5-methyl-2-p-tolyl-oxazol-4-ylmethoxy)-cyclohexylmethyl]-amino}-buttersäure-tert-butylester werden in 1 ml Ameisensäure gelöst und mit 0,5 ml Trifluoressigsäure versetzt. Die Lösung wird 18 bei Raumtemperatur gerührt, dann vollständig eingeengt. Der Rückstand wird per HPLC gereinigt, wobei 28,2 mg (S)-3-Methyl-2-{[cis-3-(5-methyl-2-p-tolyl-oxazol-4-ylmethoxy)-cyclohexylmethyl]-amino}-buttersäure Trifluoressigsäuresalz als farbloser Feststoff erhalten werden. C24H34N2O2.C2HF3O2 (414,55); MS(ES-): 413,28 (M⁺-H).

### Beispiel 43:

### (S)-2-{Acetyl-[cis-3-(5-methyl-2-p-tolyloxazol-4-ylmethoxy)-cyclohexylmethyl]-amino}-3-methylbuttersäure

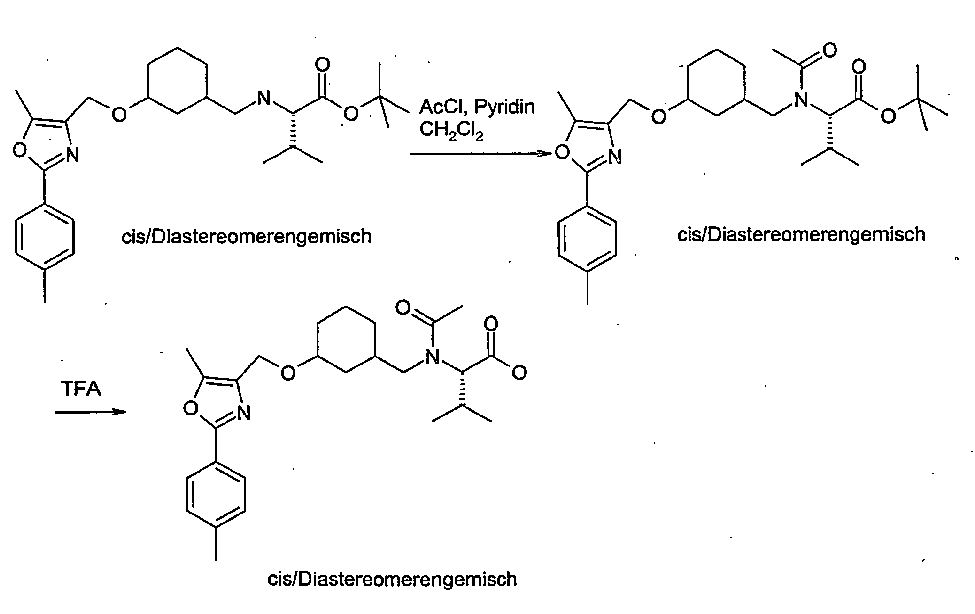

### (S)-2-{Acetyl-[cis-3-(5-methyl-2-p-tolyloxazol-4-ylmethoxy)-cyclohexylmethyl]-amino}-3-methylbuttersäure

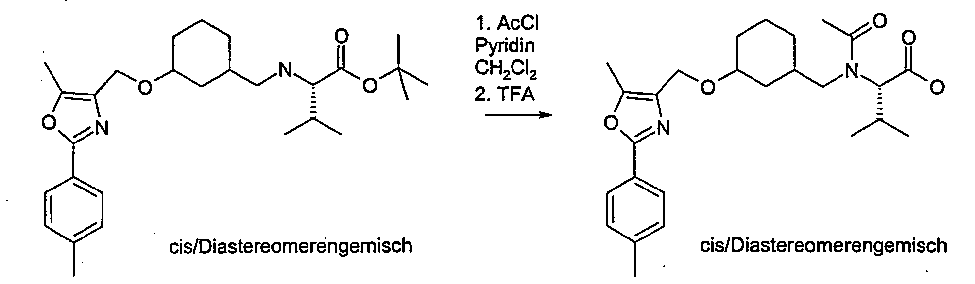

40 mg (S)-3-Methyl-2-{[cis-3-(5-methyl-2-p-tolyl-oxazol-4-ylmethoxy)-cyclohexylmethyl]-amino}-buttersäure-tert-butylester in 0,5 ml Dichlormethan werden mit 12 µl Acetylchlorid und 22 µl Pyridin versetzt und 18 h bei Raumtemperatur gerührt. Dann wird die Lösung mit Wasser und Dichlormethan verdünnt, die wäßrige Phase wird abgetrennt und mit Dichlormethan extrahiert, die vereinigten Phasen werden über MgSO4 getrocknet und eingeengt. Der Rückstand wird in 0,5 ml Trifluoressigsäure aufgenommen und bei Raumtemperatur über Nacht stehen gelassen. Das Lösungsmittel wird vollständig abdestilliert, der Rückstand per HPLC gereinigt, wobei 17 mg (S)-2-{Acetyl-[cis-3-(5-methyl-2-p-tolyloxazol-4-ylmethoxy)-cydohexylmethyl]-amino}-3-methylbuttersäure erhalten werden. C26H36N2O5 (456,59); LCMS (ESI):457,36 (MH⁺).

### Beispiel 44:

Analog zu Beispiel 43 erhält man aus (S)-3-Methyl-2-{[cis-3-(5-methyl-2-p-tolyl-oxazol-4-ylmethoxy)-cyclohexylmethyl]-amino}-buttersäure-tert-butylester und Benzoylchlorid (S)-2-{Benzoyl-[cis-3-(5-methyl-2-p-tolyloxazol-4-ylmethoxy)-cyclohexylmethyl]-amino}-3-methylbuttersäure. C31H38N2O5 (518,29); LCMS (ESI):519,54 (MH⁺).

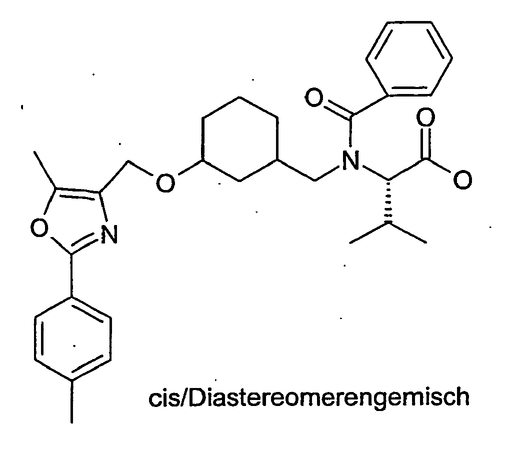

### Beispiel 45:

Analog zu Beispiel 43 erhält man aus (S)-3-Methyl-2-{[cis-3-(5-methyl-2-p-tolyl-oxazol-4-ylmethoxy)-cyclohexylmethyl]-amino}-buttersäure-tert-butylester und Methylsulfonylchlorid (S)-2-(Methylsulfonyl-[cis-3-(5-methyl-2-p-tolyloxazol-4-ylmethoxy)-cyclohexylmethyl]-amino)-3-methylbuttersäure. C25H36N2O6S (492,23); LCMS (ESI): 493,26 (MH⁺).

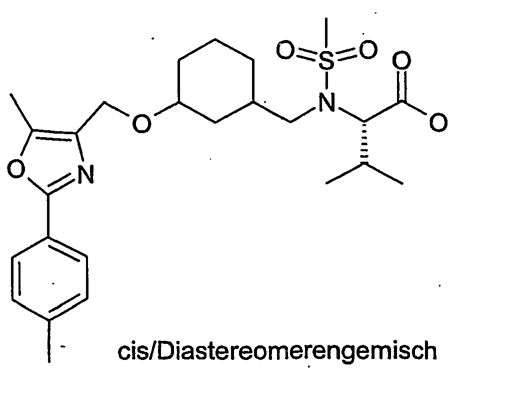

### Beispiel 46:

Analog zu Beispiel 43 erhält man aus (S)-3-Methyl-2-{[cis-3-(5-methyl-2-p-tolyl-oxazol-4-ylmethoxy)-cyclohexylmethyl]-amino}-buttersäure-tert-butylester und Methylsulfonylchlorid in Triethylamin (S)-2-{Methylsulfonylmethylsulfonyl-[cis-3-(5-methyl-2-p-tolyloxazol-4-ylmethoxy)-cyclohexylmethyl]-amino}-3-methylbuttersäure. C26H38N2O8S2 (570,21); LCMS (ES-): 569,23 (M⁺-H).

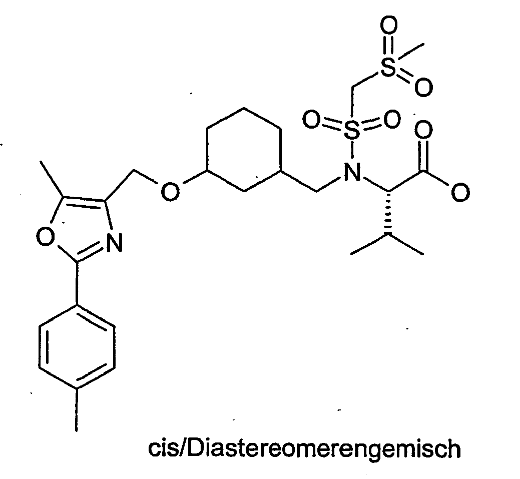

### Beispiel 47:

Analog zu Beispiel 43 erhält man aus (S)-3-Methyl-2-{[cis-3-(5-methyl-2-p-tolyl-oxazol-4-ylmethoxy)-cyclohexylmethyl]-amino}-buttersäure-tert-butylester und p-Toluolsulfonylchlorid (S)-2-{p-Toluolsulfonyl-[cis-3-(5-methyl-2-p-tolyloxazol-4-ylmethoxy)-cyclohexylmethyl]-amino}-3-methylbuttersäure. C31H40N2O6S (568,26); LCMS (ESI): 569,35 (MH⁺).

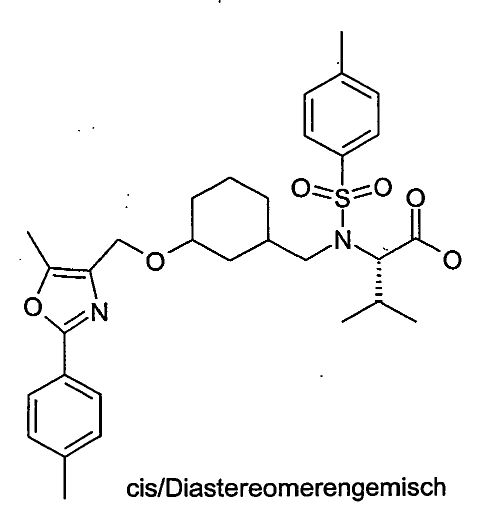

### Beispiel 48:

Analog zu Beispiel 43 erhält man aus (S)-3-Methyl-2-{[cis-3-(5-methyl-2-p-tolyl-oxazol-4-ylmethoxy)-cyclohexylmethyl]-amino}-buttersäure-tert-butylester und Chlorameisensäuremethylester (S)-2-{Methoxycarbonyl-[cis-3-(5-methyl-2-p-tolyloxazol-4-ylmethoxy)-cyclohexylmethyl]-amino}-3-methylbuttersäure. C26H36N2O6 (472,26), LCMS (ESI): 473,37 (MH⁺).

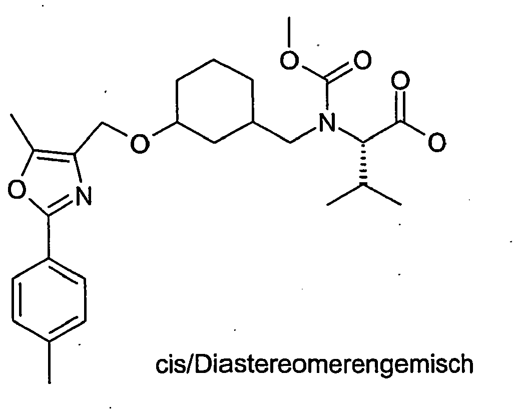

### Beispiel 49:

Analog zu Beispiel 42 erhält man aus cis-3-(5-Methyl-2-p-tolyl-oxazol-4-ylmethoxy)-cyclohexancarbaldehyd und Glycinisopropylester-Hydrochlorid 2-{[cis-3-(5-Methyl-2-p-tolyl-oxazol-4-ylmethoxy)-cyclohexylmethyl]-amino}-essigsäure Trifluoressigsäuresalz. C21H28N2O4 (486,49), MS (ESI): 487 (MH⁺).

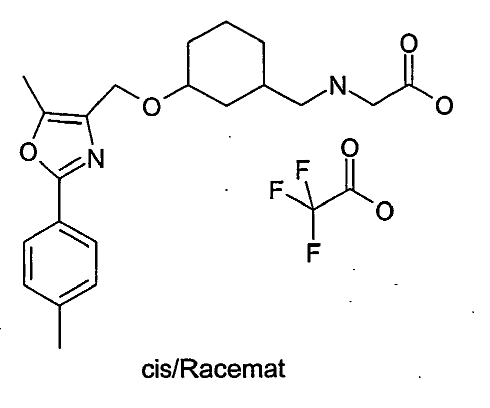

### Beispiel 50:

### (S)-2-{Methyl-[cis-3-(5-methyl-2-p-tolyloxazol-4-ylmethoxy)-cyclohexylmethyl]-amino}-3-methylbuttersäure Trifluoressigsäuresalz

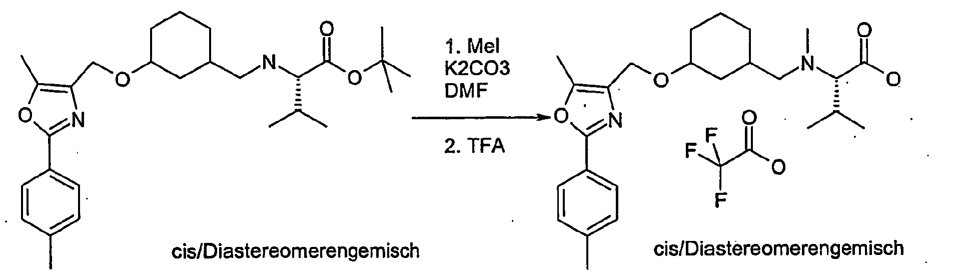

40 mg (S)-3-Methyl-2-{[cis-3-(5-methyl-2-p-tolyl-oxazol-4-ylmethoxy)-cyclohexylmethyl]-amino}-buttersäure-tert-butylester (s. Beispiel 42) werden in 0,5 ml DMF mit 60 mg Methyliodid und 12 mg Kaliumcarbonat versetzt und bei RT über Nacht gerührt. Zur Reaktionslösung wird 1 ml TFA zugesetzt und die Mischung 1 h weitergerührt. Die Lösung wird per präparativer HPLC gereinigt, wobei 6,4 mg (S)-2-{Methyl-[cis-3-(5-methyl-2-p-tolyloxazol-4-ylmethoxy)-cyclohexylmethyl]-amino}-3-methylbuttersäure Trifluoressigsäuresalz erhalten werden. C25H36N2O4 (542,60); MS (ESI): 543 (MH+).

### Beispiel 51:

Analog zu Beispiel 50 erhält man aus (S)-3-Methyl-2-{[cis-3-(5-methyl-2-p-tolyl-oxazol-4-ylmethoxy)-cyclohexylmethyl]-amino}-buttersäure-tert-butylester und Benzylbromid (S)-2-{Benzylyl-[cis-3-(5-methyl-2-p-tolyloxazol-4-ylmethoxy)-cyclohexylmethyl]-amino}-3-methylbuttersäure Trifluoressigsäuresalz. C31H40N2O4 (618,70), MS(ESI): 619 (MH+).

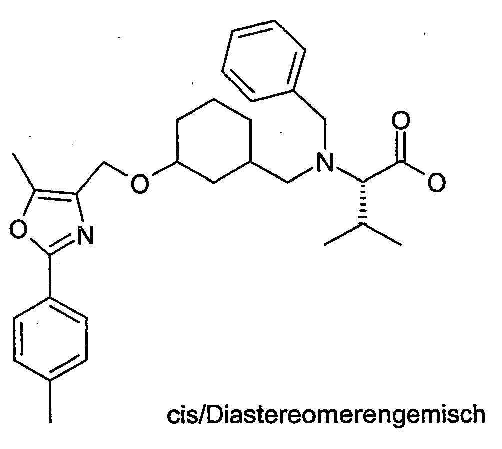

### Beispiel 52:

### 2-{Benzyl-[cis-3-(5-methyl-2-p-tolyloxazol-4-ylmethoxy)-cyclohexylmethyl]-amino}-essigsäure

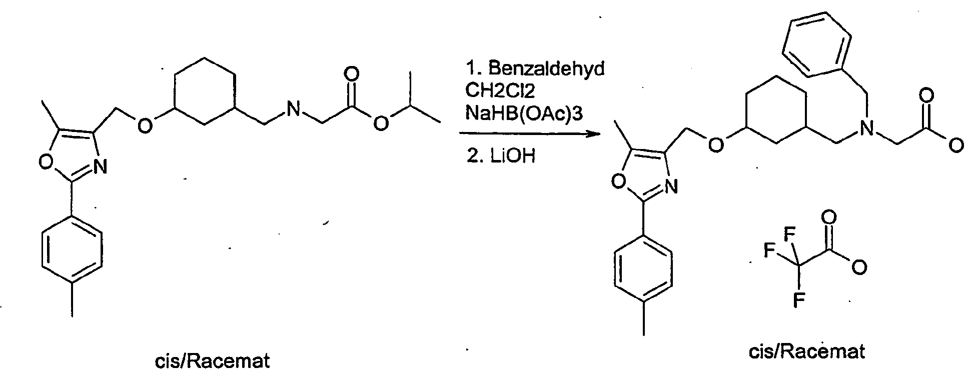

120 mg 2-{[cis-3-(5-Methyl-2-p-tolyl-oxazol-4-ylmethoxy)-cyclohexylmethyl]-amino}-essigsäureisopropylester werden in 1,5 ml Dichlormethan gelöst und nacheinander mit 62 mg Benzaldehyd, einer Spatelspitze MgSO4 und 68 mg Natriumtriacetoxyborhydrid versetzt. Die Suspension wird über Nacht bei RT gerührt und dann mit Wsser versetzt. Die organische Phase wird abgetrennt, mit Na2CO3-Lösung gewaschen, über MgSO4 getrocknet und eingeengt. Der Rückstand wird in 0,7 ml Methanol und 0,23 ml Wasser gelöst, mit 20 mg LiOH versetzt und über Nacht bei RT gerührt. Die Lösung wird eingeengt, der Rückstand per präparativer HPLC gereinigt, wobei 15 mg 2-{Benzyl-[cis-3-(5-methyl-2-p-tolyloxazol-4-ylmethoxy)-cyclohexylmethyl]-amino}-essigsäure erhalten werden. C28H34N2O4 (462,59); MS(ESI): 463 (MH+).

### Beipiel 53:

Analog zu Beispiel 52 erhält man aus mg 2-{[cis-3-(5-Methyl-2-p-tolyl-oxazol-4-ylmethoxy)-cyclohexylmethyl]-amino}-essigsäureisopropylester und Thiophen-2-carbaldehyd 2-{2-Thienylmethyl-[cis-3-(5-methyl-2-p-tolyloxazol-4-ylmethoxy)-cyclohexylmethyl]-amino}-essigsäure Trifluoressigsäuresalz. C26H32N2O4 (468,62), MS(ESI): 469 (MH+).

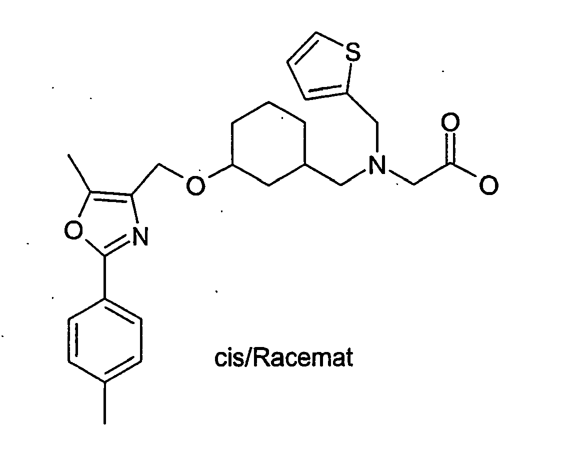

### Beipiel 54:

Analog zu Beispiel 52 erhält man aus mg 2-{[cis-3-(5-Methyl-2-p-tolyl-oxazol-4-ylmethoxy)-cyclohexylmethyl]-amino}-essigsäureisopropylester und Cyclohexancarbaldehyd 2-{Cyclohexylmethyl-[cis-3-(5-methyl-2-p-tolyloxazol-4-ylmethoxy)-cyclohexylmethyl]-amino}-essigsäure Trifluoressigsäuresalz. C28H40N2O4 (468,64), MS(ESI): 469 (MH+).

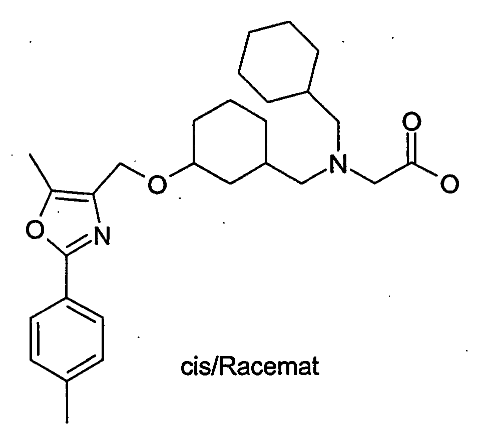

### Beispiel 55:

### 2-[cis-3-(5-Methyl-2-p-tolyl-oxazol-4-ylmethoxymethyl)-cyclohexylmethoxy]-2-methylpropionsäure:

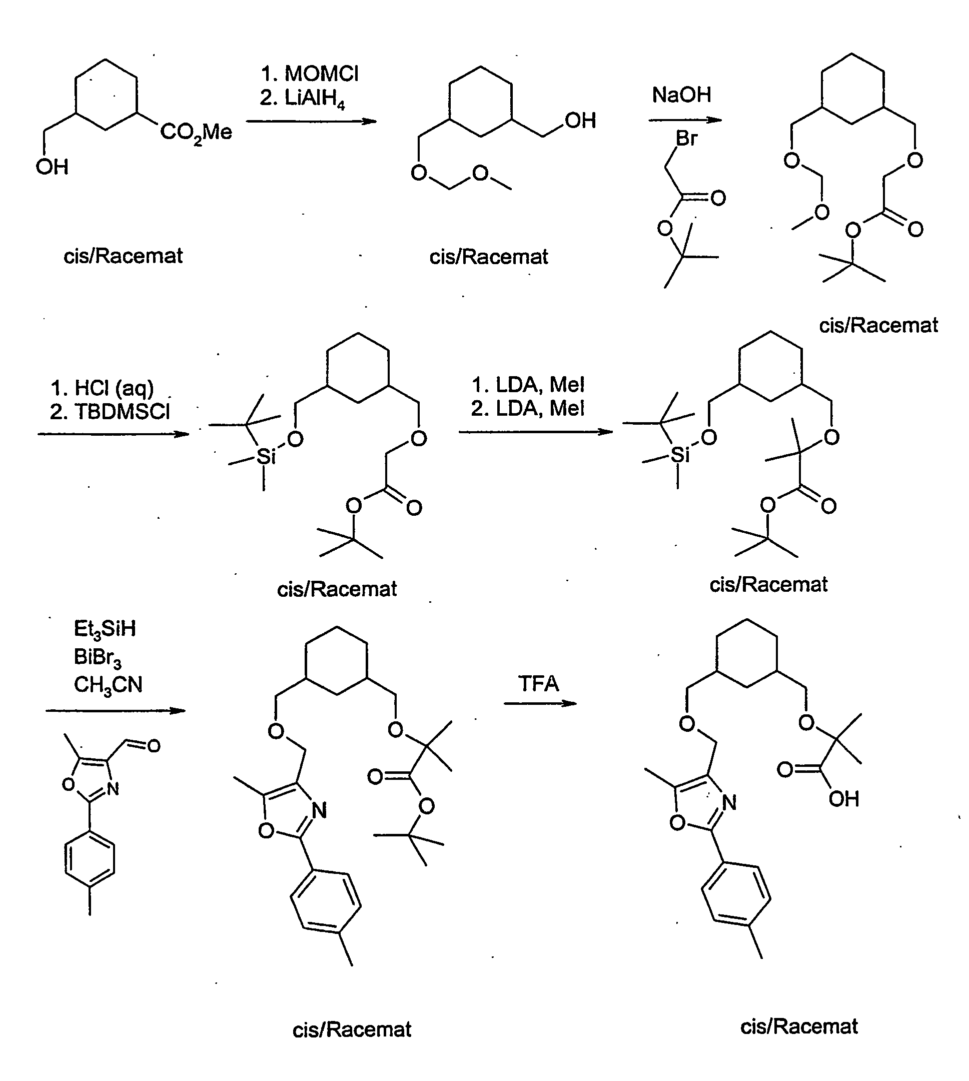

### (cis-3-Methoxymethoxymethylcyclohexylmethyl)-methanol:

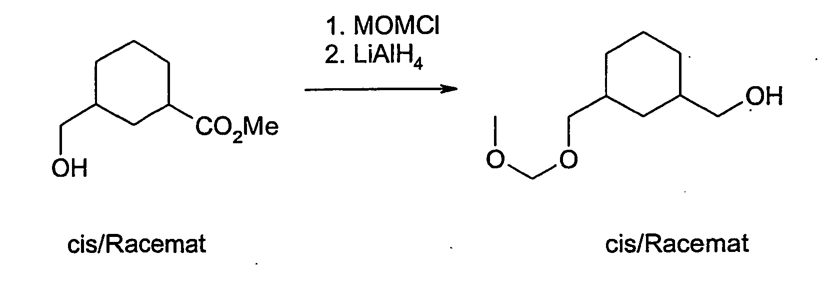

1,70g g cis-3-Hydroxymethylcyclohexancarbonsäuremethylester werden in 20 ml Dichlormethan gelöst und mit 1,60 g Methoxymethylchlorid und 2,60 g Diisopropylethylamin versetzt und 15 h bei Raumtemperatur gerührt. Die Lösung wird mit 50 ml gesättigter NH4Cl-Lösung und 50 ml Wasser vesetzt, die organische Phase wird abgetrennt. Die wäßrige Phase wird mit Dichlormethan extrahiert, die vereinigten organischen Phasen werden über Magnesiumsulfat getrocknet und eingeengt. Man erhält 2,0 g cis-3-Methoxymethoxymethylcyclohexancarbonsäuremethylester als gelbes Öl. Dieses wird in 50 ml Diethylether gelöst und mit 350 mg LiAlH4 versetzt und bei Raumtemperatur gerührt. Nach 2 h werden bei 0°C 5 ml Ethylacetat, 40 ml Methyl-tert-butylether und 3 g MgSO4 zugegeben. Anschließend werden 15 ml 10N KOH zugetropft. Die Suspension wird 3 h gerührt, über Celite filtriert, und das Filtrat eingeengt, wobei 1,65 g (cis-3-Methoxymethoxymethylcyclohexyl)-methanol als farbloses Öl erhalten werden. C10H20O3 (188,27), MS(ESI): 189,2 (MH+).

### [cis-3-(Methoxymethoxymethyl)-cyclohexylmethoxy]-essigsäure-tert-butylester:

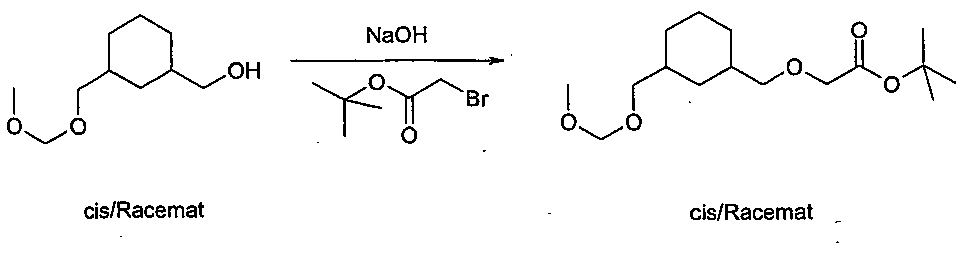

1,65 g (cis-3-Methoxymethoxymethylcyclohexyl)-methanol und 5,1 g Bromessigsäure-tert-butylester werden in 20 ml Toluol gelöst und mit 1,50 g
Tetrabutylammoniumhydrogensulfat versetzt. Die Suspension wird auf 10°C gekühlt. 20 ml 50% NaOH werden zur Suspension gegeben. Die Mischung wird 6 h bei 10 °C gerührt, dann wird die wäßrige Phase abgetrennt und mit Methyl-tert-butylether extrahiert. Die vereinigten organischen Phasen werden über MgSO4 getrocknet und eingeengt. Nach Flash-Säulenchromatographie an Kieselgel (Heptan/Ethylacetat 10/1 -> 2/1) werden 2,23 g [cis-3-(Methoxymethoxymethyl)-cyclohexylmethoxy]-essigsäure-tert-butylester als farbloses Öl erhalten. C16H30O5 (302,41), MS(ESI): 320,30 (M+NH4+).

### [cis-3-(tert-Butyldimethylsilanyloxymethyl)-cyclohexylmethoxy]-essigsäure-tert-butylester:

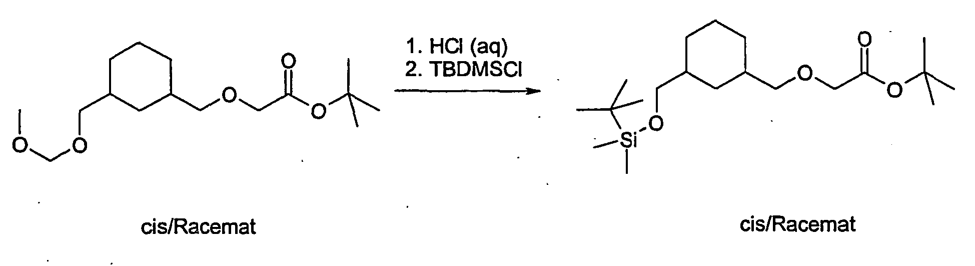

1,9 g [cis-3-(Methoxymethoxymethyl)-cyclohexylmethoxy]-essigsäure-tert-butylester werden in 10 ml Tetrahydrofuran gelöst, mit 5 ml konz. HCl versetzt und 2 h bei Raumtemperatur gerührt. Anschließend werden 10 ml ges. NaCl-Lösung, 10 ml Wasser und 30 ml Methyl-tert-butylether zugegeben, die Phasen getrennt, und die wäßrige Phase wird mit Methyl-tert-butylether extrahiert. Die vereinigten organischen Phasen werden über MgSO4 getrocknet und eingeengt. Nach Flash-Chromatographie an Kieselgel (Heptan/Ethylacetat 3/1) werden 600 mg (cis-3-Hydroxymethylcyclohexylmethoxy)-essigsäure-tert-butylester als farbloses Öl erhalten (DC(Heptan/Ethylacetat 2/1): Rf,Edukt = 0.68, Rf,Produkt = 0.18). 260 mg davon werden in 5 ml Dimethylformamid gelöst und mit 170 mg tert-Butyldimethylsilylchlorid versetzt. Dann wird die Lösung auf 0 °C gekühlt, und 160 mg Imidazol werden zugegeben. Die Lösung wird bei Raumtemperatur 15 h gerührt, dann mit 20 ml ges. NaCl-Lösung, 10 ml Wasser und 30 ml Methyl-tert-butylether versetzt. Die Phasen werden getrennt, die organische Phase wird mit ges. NaCl-Lösung gewaschen, über MgSO4 getrocknet und eingeengt. Man erhält 350 mg [cis-3-(tert-Butyldimethylsilanyloxymethyl)-cyclohexylmethoxy]-essigsäure-tert-butylester als farbloses Öl. C20H40O4Si (372,36); LCMS (ESI): 390,3 (M+NH4+).

### 2-[cis-3-(tert-Butyldimethylsilanyloxymethyl)-cyclohexylmethoxy]-2-methylpropionsäure-tert-butylester:

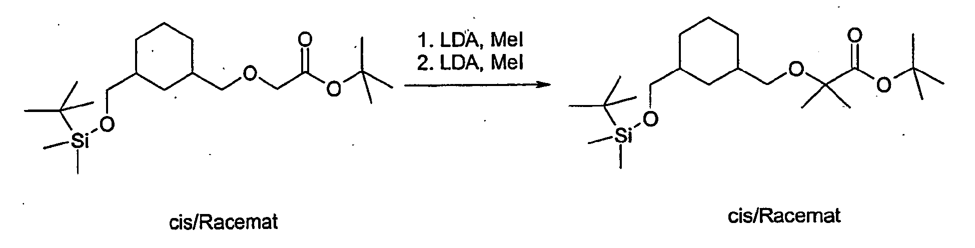

250 mg [cis-3-(tert-butyldimethylsilanyloxymethyl)-cyclohexylmethoxy]-essigsäure-tert-butylester werden in 10 ml abs. Tetrahydrofuran gelöst und auf -78°C (Trockeneis/Aceton-Bad) gekühlt. Anschließend werden 1,70 ml 2M Lithiumdiisopropylamid-Lösung in Tetrahydrofuran/Hexanfraktion zugetropft. Die Lösung wird zunächst bei -78°C 20 min gerührt, dann auf 0°C erwärmt (Eisbad) und mit 950 mg Methyliodid versetzt. Die Lösung wird 1 h bei 0°C gerührt. 1 ml ges.
NH4Cl-Lösung und 10 ml Wasser werden zugesetzt und die Phasen werden getrennt. Die wäßrige Phase wird mit Ethylacetat extrahiert. Die vereinigten organischen Phasen werden über MgSO4 getrocknet und eingeengt. Das Rohprodukt wird in 10 ml abs.
Tetrahydrofuran gelöst und auf -78°C (Trockeneis/Aceton-Bad) gekühlt. Anschließend werden 1,70 ml 2M Lithiumdiisopropylamid-Lösung in Tetrahydrofuran/Hexanfraktion zugetropft. Die Lösung wird zunächst bei -78°C 20 min gerührt, dann auf 0°C erwärmt (Eisbad) und mit 950 mg Methyliodid versetzt. Die Lösung wird 1 h bei 0°C gerührt. 1 ml ges. NH4Cl-Lösung und 10 ml Wasser werden zugesetzt und die Phasen werden getrennt. Die wäßrige Phase wird mit Ethylacetat extrahiert. Die vereinigten organischen Phasen werden über MgSO4 getrocknet und eingeengt. 220 mg 2-[cis-3-(tert-butyldimethylsilanyloxymethyl)-cyclohexylmethoxy]-2-methylpropionsäure-tert-butylester werden als hellgelbes Öl erhalten. DC(Heptan/Ethylacetat 4/1): R_{f,Edukt} = 0,66, R_{f,Produkt} = 0,80.

### 2-[cis-3-(5-Methyl-2-p-tolyl-oxazol-4-ylmethoxymethyl)-cyclohexylmethoxy]-2-methylpropionsäure:

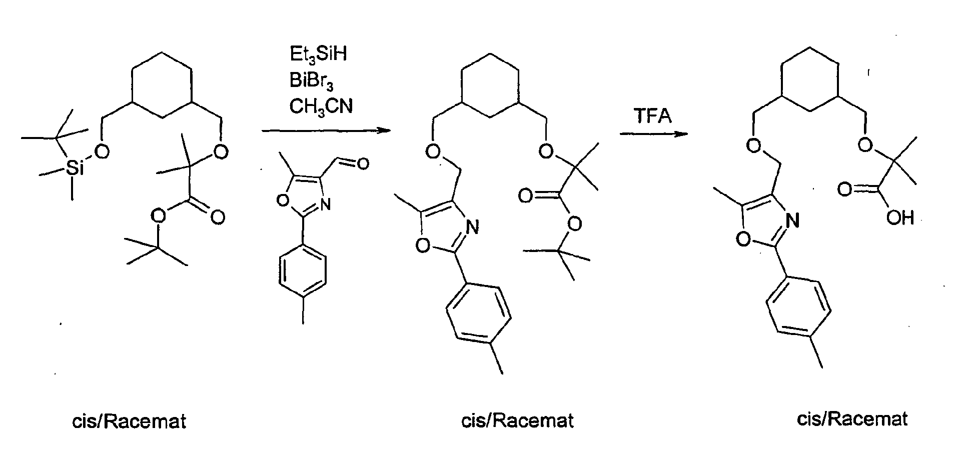

50 mg 2-[cis-3-(tert-butyldimethylsilanyloxymethyl)-cyclohexylmethoxy]-2-methylpropionsäure-tert-butylester werden zu einer Mischung von 20 mg BiBr3 und 30 mg HSiEt₃ in 0,5 ml Acetonitril gegeben. 38 mg 5-Methyl-2-p-tolyl-oxazol-4-carbaldehyd in 0,2 ml Acetonitril werden zugetropft, und die Mischung wird über Nacht bei Raumtemperatur gerührt. Der entstandene schwarze Feststoff wird abfiltriert, das Filtrat eingeengt und mit 1 ml Trifluoressigsäure aufgenommen. Die Lösung wird über Nacht bei Raumtemperatur gerührt. Das Lösungsmittel wird im Vakuum abdestilliert und der Rückstand per HPLC gereinigt. 3,4 mg 2-[cis-3-(5-Methyl-2-p-tolyl-oxazol-4-ylmethoxymethyl)-cyclohexylmethoxy]-2-methylpropionsäure werden als farbloses Öl erhalten. C24H33NO5 (415,24); MS (ES-): 414,25 (M-H⁺).

### Beispiel 56:

Analog zu Beispiel 55 erhält man aus [cis-3-(Methoxymethoxymethyl)-cyclohexylmethoxy)-essigsäure-tert-butylester, Methyliodid, Ethyliodid und 5-Methyl-2-p-tolyl-oxazol-4-carbaldehyd 2-[cis-3-(5-Methyl-2-p-tolyl-oxazol-4-ylmethoxymethyl)-cyclohexylmethoxy]-2-methylbuttersäure. C25H35NO5 (429,25); MS (ES-): 428,22 (M-H⁺).

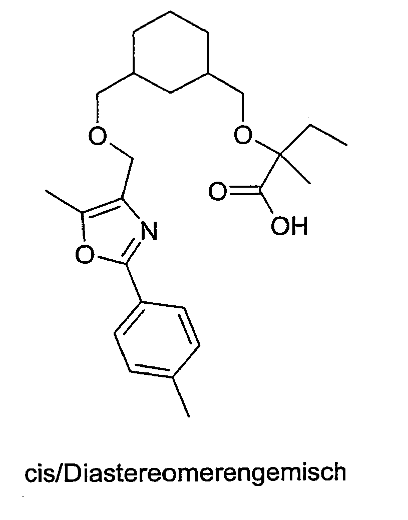

### Beispiel 57:

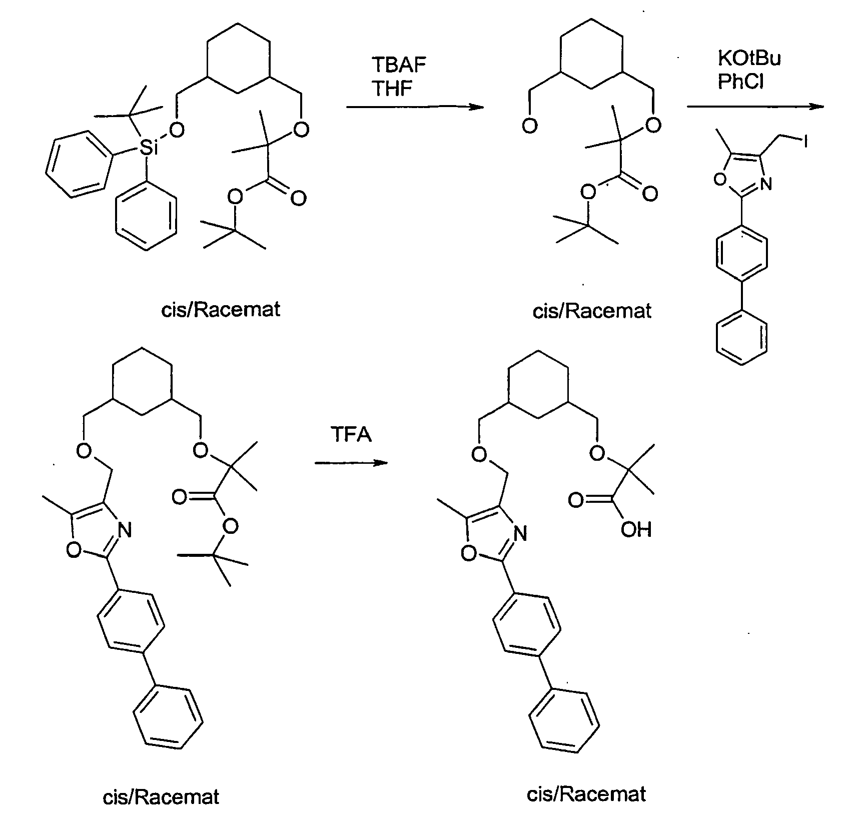

### 2-[cis-3-Hydroxymethylcyclohexylmethoxy]-2-methylpropionsäure-tert-butylester

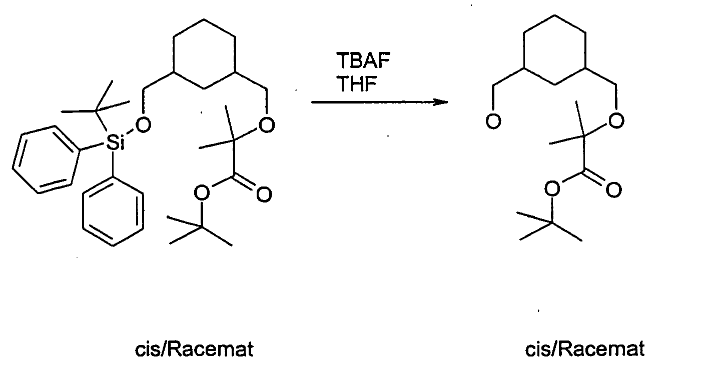

4,5 g 2-[cis-3-(tert-Butyldimethylsilanyloxymethyl)-cyclohexylmethoxy]-2-methylpropionsäure-tert-butylester (Synthese analog zu 2-[cis-3-(tert-Butyldimethylsilanyloxymethyl)-cyclohexylmethoxy]-2-methylpropionsäure-tert-butylester in Beispiel 55) werden in 85 ml THF gelöst und mit 2,24 g TBAF Trihydrat versetzt und bei 60°C 90 min gerührt. Wasser und MTBE werden zugesetzt, die Phasen getrennt, die organische Phase über MgSO4 getrocknet und eingeengt. Der Rückstand wird an Kieselgel chromatographiert (Heptan/Ethylacetat 1:1), wobei 1,45 g 2-[cis-3-Hydroxymethylcyclohexylmethoxy]-2-methylpropionsäure-tert-butylester erhalten werden. C16H30O4 (286,42), MS(ESI): 287 (MH+).

### 2-[cis-3-(5-Methyl-2-(4-biphenyl)-oxazol-4-ylmethoxymethyl)-cyclohexylmethoxy]-2-methylpropionsäure

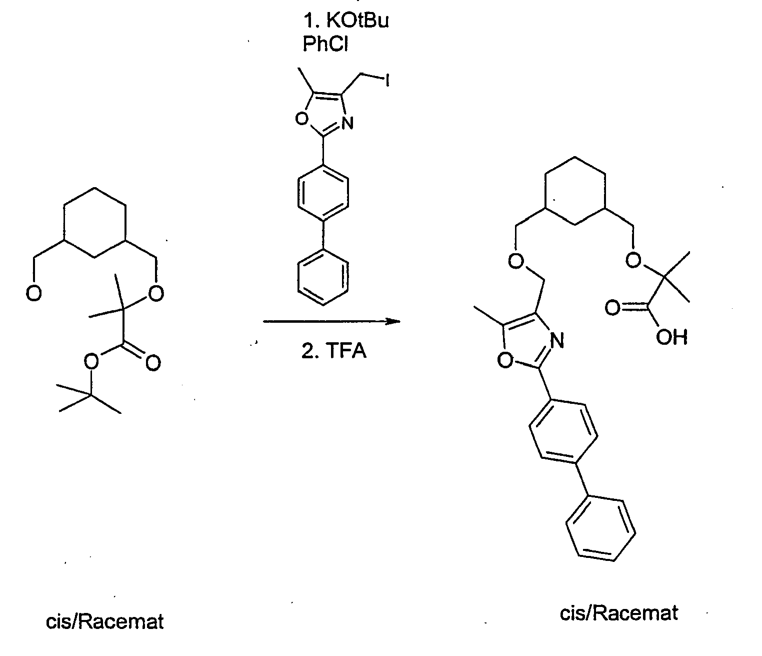

50 mg 2-[cis-3-hydroxymethylcyclohexylmethoxy]-2-methylpropionsäure-tert-butylester und 200 mg 5-Methyl-2-(4-biphenyl)-oxazol-4-ylmethoxymethyliodid werden in 2 ml Chlorbenzol gelöst und mit 59 mg Kalium-tert-butylat versetzt. Die Suspension wird 24 bei RT gerührt, dann werden 2,5 ml TFA zugesetzt und die Lösung weitere 24 h bei RT gerührt. Das Lösungsmittel wird im Vakuum abdestilliert, der Rückstand per präparativer HPLC gereinigt, wobei 20 mg 2-[cis-3-(5-Methy)-2-(4-biphenyt)-oxazol-4-ylmethoxymethyl)-cyclohexylmethoxy]-2-methylpropionsäure erhalten werden. C29H35NO5 (477,61), MS(ESI): 478 (MH+).

### Beispiel 58:

Analog zu Beispiel 57 erhält man aus2-[cis-3-Hydroxymethylcyclohexylmethoxy]-2-methylpropionsäure-tert-butylester und 5-Methyl-2-phenyl-oxazol-4-ylmethoxymethylchlorid 2-[cis-3-(5-Methyl-2-phenyl-oxazol-4-ylmethoxymethyl)-cyclohexylmethoxy]-2-methylpropionsäure. C23H31NO5 (401,51); MS (ESI): 402 (MH⁺).

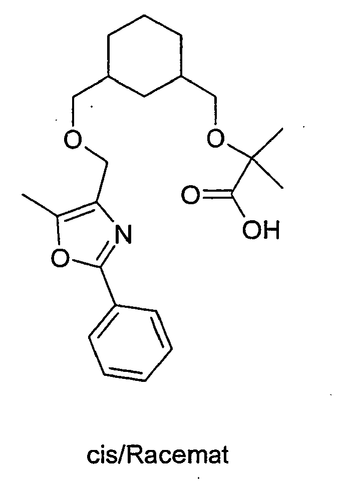

### Beispiel 59:

Analog zu Beispiel 57 erhält man aus2-[cis-3-Hydroxymethylcyclohexylmethoxy]-2-methylpropionsäure-tert-butylester und 5-Methyl-2-naphthyl-oxazol-4-ylmethoxymethylchlorid 2-[cis-3-(5-Methyl-2-naphthyl-oxazol-4-ylmethoxymethyl)-cyclohexylmethoxy]-2-methylpropionsäure. C27H33NO5 (451,57); MS (ESI): 458 (MH⁺).

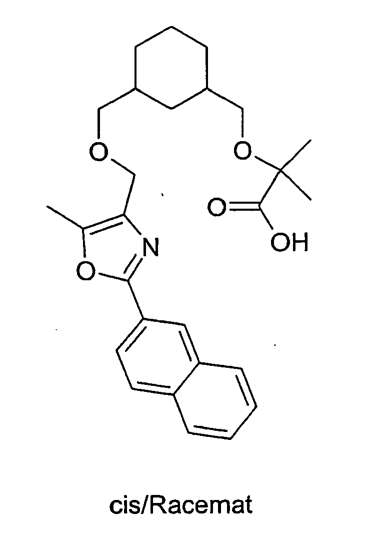

### Beispiel 60:

Analog zu Beispiel 57 erhält man aus2-[cis-3-Hydroxymethylcyclohexylmethoxy]-2-methylpropionsäure-tert-butylester und 5-Ethyl-2-(3-methoxyphenyl)-oxazol-4-ylmethoxymethylchlorid 2-[cis-3-(5-Ethyl-2-(3-methoxyphenyl)-oxazol-4-ylmethoxymethyl)-cyclohexylmethoxy]-2-methylpropionsäure. C25H35NO5 (445,56); MS (ESI): 446 (MH⁺).

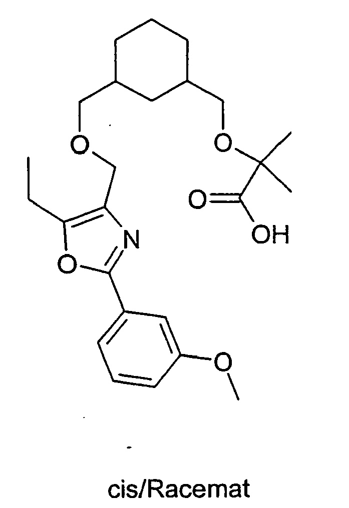

### Beispiel 61:

Analog zu Beispiel 57 erhält man aus2-[cis-3-Hydroxymethylcyclohexylmethoxy]-2-methylpropionsäure-tert-butylester und 5-Methyl-2-(4-isopropylphenyl)-oxazol-4-ylmethoxymethylchlorid 2-[cis-3-(5-Ethyl-2-(4-isopropylphenyl)-oxazol-4-ylmethoxymethyl)-cyclohexylmethoxy]-2-methylpropionsäure. C26H37NO5 (443,59); MS (ESI): 444 (MH⁺).

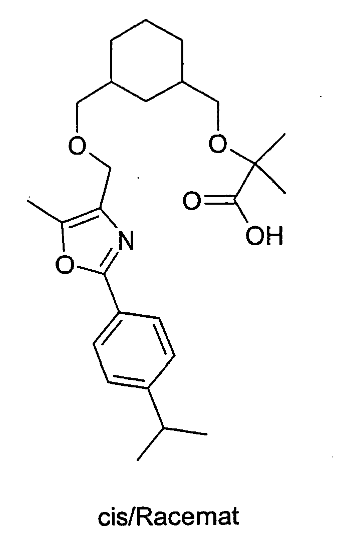

### Beispiel 62:

Analog zu Beispiel 57 erhält man aus2-[cis-3-Hydroxymethylcyclohexylmethoxy]-2-methylpropionsäure-tert-butylester und 5-Cyclohexyl-2-p-tolyl-oxazol-4-ylmethoxymethylchlorid 2-[cis-3-(5-Ethyl-2-p-tolyl-oxazol-4-ylmethoxymethyl)-cyclohexylmethoxy]-2-methylpropionsäure. C29H41NO5 (483,65); MS (ESI): 484 (MH⁺).

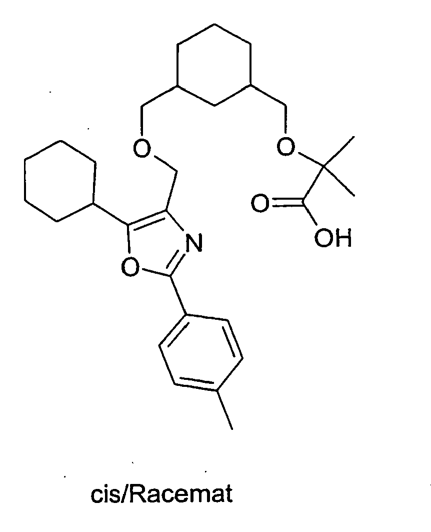

### Beispiel 63:

Analog zu Beispiel 57 erhält man aus2-[cis-3-Hydroxymethylcyclohexylmethoxy]-2-methylpropionsäure-tert-butylester und 5-Ethyl-2-p-isopropyl-oxazol-4-ylmethoxymethylchlorid 2-[cis-3-(5-Ethyl-2-p-isopropyl-oxazol-4-ylmethoxymethyl)-cyclohexylmetlioxy]-2-methylpropionsäure. C27H39NO5 (457,62); MS (ESI): 458 (MH⁺).

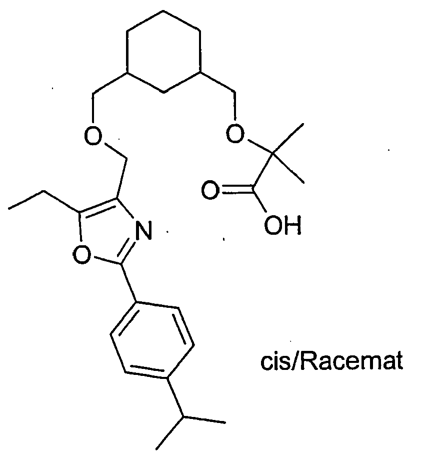

### Beispiel 64:

Analog zu Beispiel 57 erhält man aus2-[cis-3-Hydroxymethylcyclohexylmethoxy]-2-methylpropionsäure-tert-butylester und 5-Ethyl-2-(2-naphthyl)-oxazol-4-ylmethoxymethylchlorid 2-[cis-3-(5-Ethyl-2-(2-naphthyl)-oxazol-4-ylmethoxymethyl)-cyclohexylmethoxy]-2-methylpropionsäure. C28H35NO5 (465,59); MS (ESI): 466 (MH⁺).

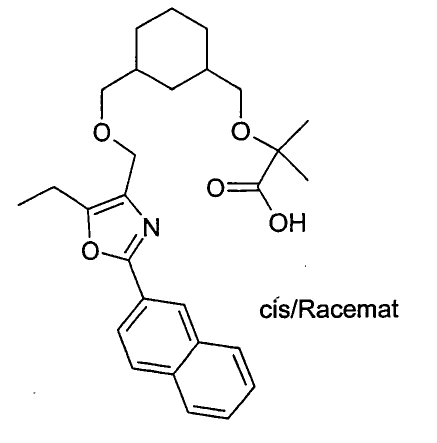

### Beispiel 65:

Analog zu Beispiel 57 erhält man aus2-[cis-3-Hydroxymethylcyclohexylmethoxy]-2-methylpropionsäure-tert-butylester und 5-Ethyl-2-p-tolyl-oxazol-4-ylmethoxymethylchlorid 2-[cis-3-(5-Ethyl-2-p-tolyl-oxazol-4-ylmethoxymethyl)-cyclohexylmethoxy]-2-methylpropionsäure. C25H35NO5 (429,56); MS (ESI): 430 (MH⁺).

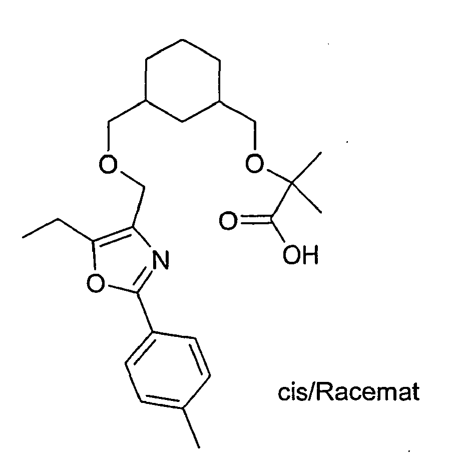

### Beispiel 66:

Analog zu Beispiel 57 erhält man aus2-[cis-3-Hydroxymethylcyclohexylmethoxy]-2-methylpropionsäure-tert-butylester und 5-Ethyl-2-(3-trifluormethyl)-oxazol-4-ylmethoxymethylchlorid 2-[cis-3-(5-Ethyl-2-(3-trifluormethyl)-oxazol-4-ylmethoxymethyl)-cyclohexylmethoxy]-2-methylpropionsäure. C26H34F3NO5 (497,56); MS (ESI): 498 (MH⁺).

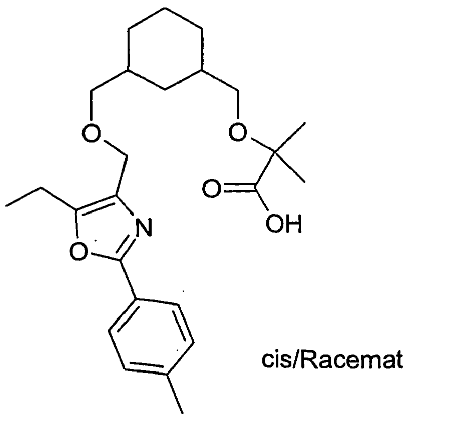

### Beispiel 67:

### 4-{cis-3-[2-(3-Methoxy-phenyl)-5-methyl-oxazol-4-ylmethoxy]-cyclohexyl}-buttersäure

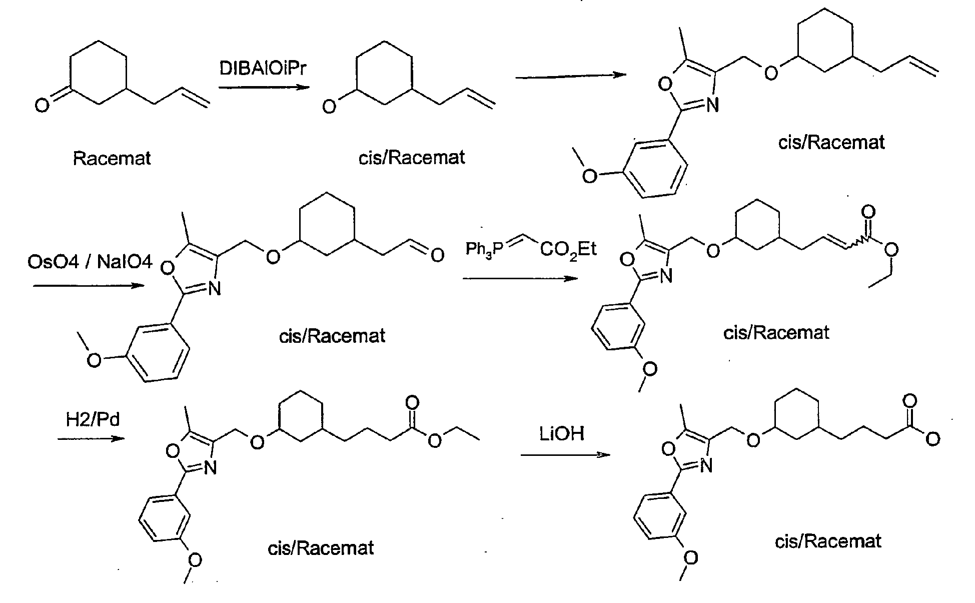

### cis-3-Allyl-cyclohexanol

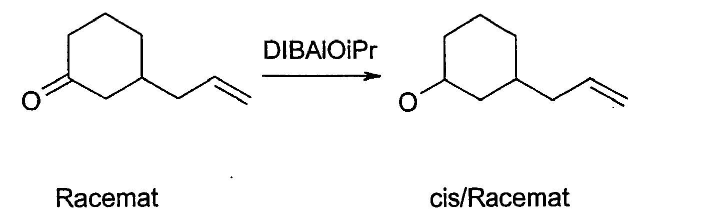

87 ml einer 1 molaren Lösung von Lithiumdiiobutylaluminiumhydrid in n-Hexan werden in 100 ml Diethylether gelöst und bei 0°C mit 7 ml Isopropanol versetzt. Nach beendeter Gasentwicklung werden 12.4 g cis-3-Allylcylohexanon, gelöst in 50 ml Diethylether, zugegeben. Man rührt 48 Stunden bei Raumtemperatur nach. Das Reaktionsgemisch wird durch Zugabe 1M HCl abgelöscht, die wäßrige Phase mit Natriumchlorid gesättigt und fünfmal mit je 200 ml Ethylacetat extrahiert. Die vereinigten organischen Phasen werden mit 2N NaOH gewaschen, über MgSO4 getrocknet und anschließend das Lösungsmittel im Vakuum entfernt. Der Rückstand wird an Kieselgel mit dem Laufmittel n-Heptan:Ethylacetat = 15:1 => 5:1 gereinigt. Man erhält 6.8 g cis-3-Allyl-cyclohexanol als Öl. C9H16O (140.23), MS(ESI): 141 (M+H⁺), R_{f}(n-Heptan:Ethylacetat = 2:1) = 0.22.

### 4-(cis-3-Allyl-cyclohexyloxymethyl)-2-(3-methoxy-phenyl)-5-methyl-oxazol

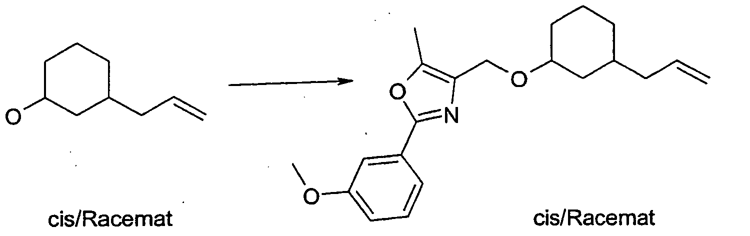

1.8 g cis-3-Allyl-cyclohexanol werden in 20 ml Dimethylformamid gelöst und mit 770 mg Natriumhydrid (60%ige Suspension in Paraffinöl) versetzt. Nach 30 Minuten werden 4 g 4-lodmethyl-5-methyl-2-(3-methoxy-phenyl)-oxazol gelöst in 20 ml Dimethylformamid zugetropft. Man rührt 1 Stunde bei Raumtemperatur nach.Dann wird 200 ml Methy-tert-buthylether zum Reaktionsgemisch gegeben und dreimal mit Wasser gewaschen. Die organische Phase wird über MgSO4 getrocknet und anschließend das Lösungsmittel im Vakuum entfernt. Der Rückstand wird an Kieselgel mit dem Laufmittel n-Heptan:Ethylacetat= 10:1 gereinigt. Man erhält 750 mg 4-(cis-3-Allyl-cyclohexyloxymethyl)-2-(3-methoxy-phenyl)-5-methyl-oxazol als Öl. C21 H27NO3 (341.45), MS(ESI): 342 (M+H⁺), R_{f}(n-Heptan:Ethylacetat = 2:1) = 0.26.

### {cis-3-[2-(3-Methoxy-phenyl)-5-methyl-oxazol-4-ylmethoxy]-cyclohexyl}-acetaldehyde

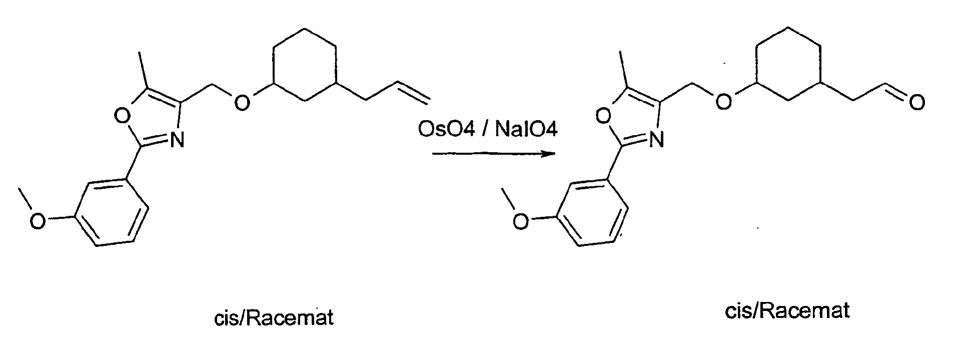

750 mg 4-(cis-3-Allyl-cyclohexyloxymethyl)-2-(3-methoxy-phenyl)-5-methyl-oxazol werden in 20 ml Diethylether gelöst und mit 1.4 g Natriumperiodat, gelöst in 20 ml Wasser versetzt. Man gibt bei 0°C 1 ml einer Osmiumtetroxid-Lösung (2.5Gewichts% in tert-Butanol) hinzu und rührt kräftig bei Raumtemperatur nach. Nach 8 Stunden wird 100 ml Methyl-tert-butyl-ether zugegeben und mit einer gesättigten Natriumthiosulfat-Lösung gewaschen. Die organische Phase wird über MgSO4 getrocknet und anschließend das Lösungsmittel im Vakuum entfernt. Man erhält 740 mg {cis-3-[2-(3-Methoxy-phenyl)-5-methyl-oxazol-4-ylmethoxy]-cyclohexyl}-acetaldehyde als gelbbraunes Öl. C20H25NO4 (343.43), MS(ESI): 344 (M+H⁺), R_{f}(n-Heptan:Ethylacetat =2:1) = 0.10.

### 4-(cis-3-[2-(3-Methoxy-phenyl)-5-methyl-oxazol-4-ylmethoxy]-cydohexyl)-but-2-ensäure-ethylester

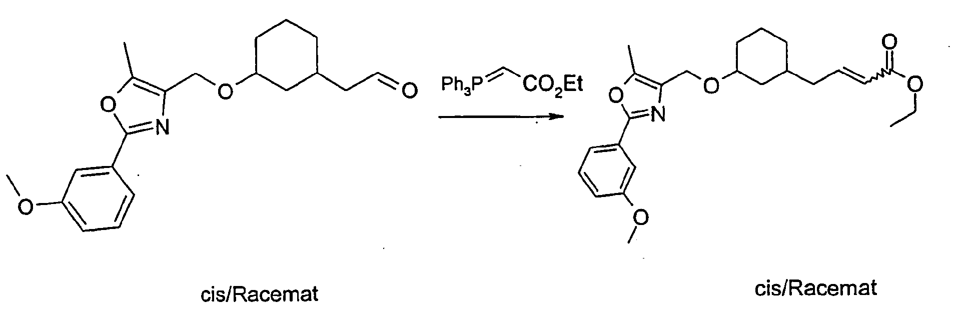

280 mg {cis-3-[2-(3-Methoxy-phenyl)-5-methyl-oxazol-4.-ylmethoxy]-cyclohexyl}-acetaldehyde werden in 10 ml Dichlormethan gelöst und mit 370 mg (Triphenylphosphoranylidene)-essigsäureethylester versetzt. Man rührt 3 Stunden bei Raumtemperatur nach. Das Gemisch wird mit gesättigter Natriumchlorid-Lösung gewaschen, über MgSO4 getrocknet und anschließend das Lösungsmittel im Vakuum entfernt. Der Rückstand wird an Kieselgel mit dem Laufmittel n-Heptan:Ethylacetat= 5:1 gereinigt. Man erhält 190 mg 4-{cis-3-[2-(3-Methoxy-phenyl)-5-methyl-oxazol-4-ylmethoxy]-cyclohexyl}-but-2-ensäure-ethylester als Öl. C24H31NO5 (413.52), MS(ESI): 414 (M+H⁺), R_{f}(n-Heptan:Ethylacetat = 2:1) = 0.30.

### 4-{cis-3-[2-(3-Methoxy-phenyl)-5-methyl-oxazol-4-ylmethoxy]-cyclohexyl}-buttersäureethylester

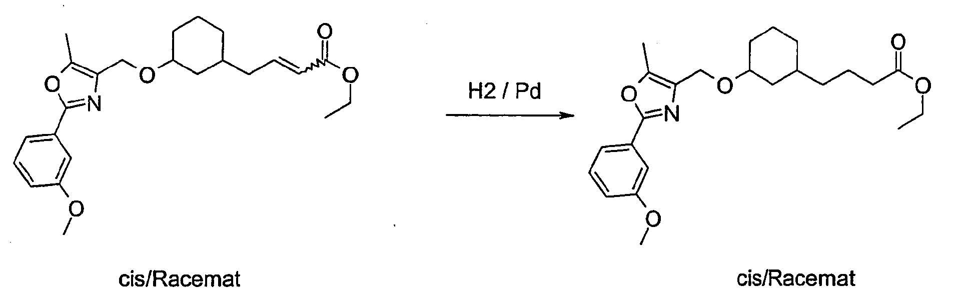

190 mg 4-{cis-3-[2-(3-Methoxy-phenyl)-5-methyl-oxazol-4-ylmethoxy]-cyclohexyl}-but-2-ensäure-ethylester werden in 25 ml Methanol gelöst und mit 20 mg Pd (10% auf Aktivkohle) versetzt. Es wird unter einer Wasserstoffatmosphäre 7 Stunden bei Raumtemperatur gerührt. Der Katalysator wird über Celite abfiltriert und das Filtrat im Vakuum eingeengt. Man erhält 110 mg 4-{cis-3-[2-(3-Methoxy-phenyl)-5-methyl-oxazol-4-ylmethoxy]-cyclohexyl}-buttersäureethylester als Öl. C24H33NO5 (415.53), MS(ESI): 416 (M+H⁺).

### 4-{cis-3-[2-(3-Methoxy-phenyl)-5-methyl-oxazol-4-ylmethoxy]-cyclohexyl}-buttersäure

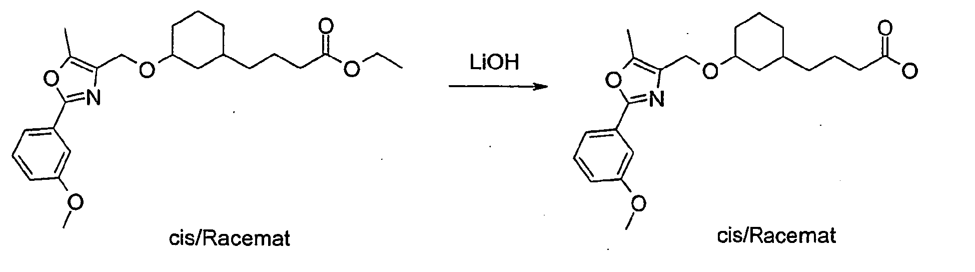

110 mg 4-{cis-3-[2-(3-Methoxy-phenyl)-5-methyl-oxazol-4-ylmethoxy]-cyclohexyl}-buttersäureethylester werden in 5 ml einer Mischung aus Tetrahydrofuran und Wasser im Verhältnis 2:1 gelöst und mit 20 mg Lithiumhydroxid versetzt. Man rührt 12 Stunden bei Raumtemperatur nach. Durch Zugabe von 1 N HCl wird das Gemisch angesäuert und mit Ethylacetat extrahiert. Die organische Phase wird über MgSO4 getrocknet und anschließend das Lösungsmittel im Vakuum entfernt. Der Rückstand wird durch RP-HPLC gereinigt. Nach Gefriertrocknung erhält man 24 mg 4-{cis-3-[2-(3-Methoxyphenyl)-5-methyl-oxazol-4-ylmethoxy]-cyclohexyl}-buttersäure als Lyophilisat.
C22H29NO5 (387.48), MS(ESI): 388 (M+H⁺).

### Beispiel 68:

Analog zu Beispiel 67 wurde aus {cis-3-[2-(3-Methoxy-phenyl)-5-methyl-oxazol-4-ylmethoxy]-cyclohexyl}-acetaldehyde und 2-(Triphenylphosphoranylidene)-propionsäureethylester 4-{cis-3-[2-(3-Methoxy-phenyl)-5-methyl-oxazol-4-ylmethoxy]-cyclohexyl}-2-methyl-buttersäure erhalten. 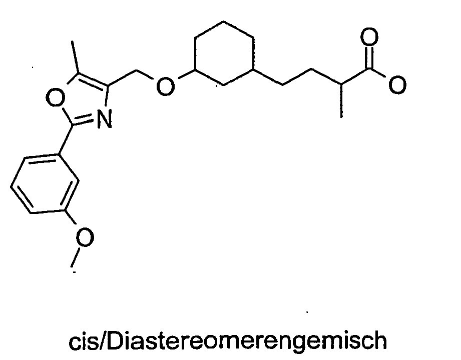 C23H31NO5 (401.51), MS(ESI): 402 (M+H⁺).

### Beispiel 69:

### 2-Ethyl-4-{cis-3-[2-(3-methoxy-phenyl)-5-methyl-oxazol-4-ylmethoxy]-cyclohexyl)-buttersäure

### 2-Ethyl-4-{cis-3-[2-(3-methoxy-phenyl)-5-methyl-oxazol-4-ylmethoxy]-cyclohexyl}-but-2-ensäure-ethylester

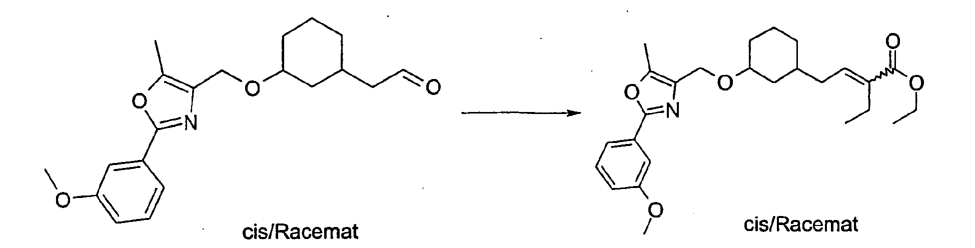

0.4 ml Triethylphosphonobutyrat werden in 5 ml Tetrahydrofuran gelöst und bei -20°C mit 0.5 ml einer 2.5 M n-Butyllithium-lösung in n-Hexan versetzt. Man rührt 1 Stunde bei -20°C nach, dann werden 386 mg {cis-3-[2-(3-Methoxy-phenyl)-5-methyl-oxazol-4-ylmethoxy]-cyclohexyl}-acetaldehyde, gelöst in 4 ml Tetrahydrofuran, zugegeben. Nach 30 Minuten wird das Reaktionsgemisch langsam auf Raumtemperatur erwärmt, es werden 0.5 ml Wasser zugegeben, der Rückstand mit Ethylacetat verdünnt, über MgSO4 getrocknet und anschließend das Lösungsmittel im Vakuum entfernt. Man erhält 750 mg 2-Ethyl-4-{cis-3-[2-(3-methoxy-phenyl)-5-methyl-oxazol-4-ylmethoxy]-cyclohexyl}-but-2-ensäure-ethylester als Öl. C26H35NO5 (441.57), MS(ESI): 442 (M+H⁺).

Analog zu Beispiel 67 wurde aus 2-Ethyl-4-{cis-3-[2-(3-methoxy-phenyl)-5-methyl-oxazol-4-ylmethoxy]-cyclohexyl}-but-2-ensäure-ethylester 2-Ethyl-4-{cis-3-[2-(3-methoxy-phenyl)-5-methyl-oxazol-4-ylmethoxy]-cyclohexyl}-buttersäure erhalten. 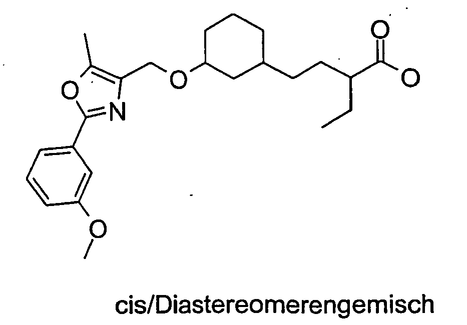 C24H33NO5 (415.53), MS(ESI): 416 (M+H⁺).

### Beispiel 70:

Analog zu Beispiel 69 wurde aus {cis-3-[2-(3-Methoxy-phenyl)-5-methyl-oxazol-4-ylmethoxy]-cyclohexyl}-acetaldehyde und Triethylphosphonopentanoat 2-(2-{cis-3-[2-(3-Methoxy-phenyl)-5-methyl-oxazol-4-ylmethoxy]-cyclohexyl}-ethyl)-pentansäure erhalten. 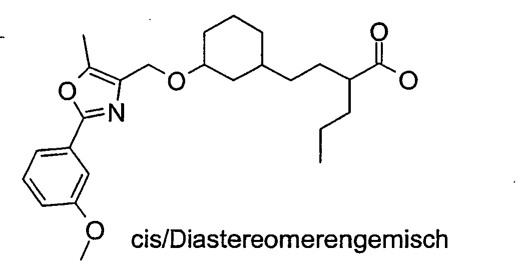 C25H35NO5 (429.56), MS(ESI): 430 (M+H⁺).

### Beispiel 71:

### 2,2-Dimethyl-4-[cis-3-(5-methyl-2-p-tolyl-oxazol-4-ylmethoxy)-cyclohexyl]-buttersäure

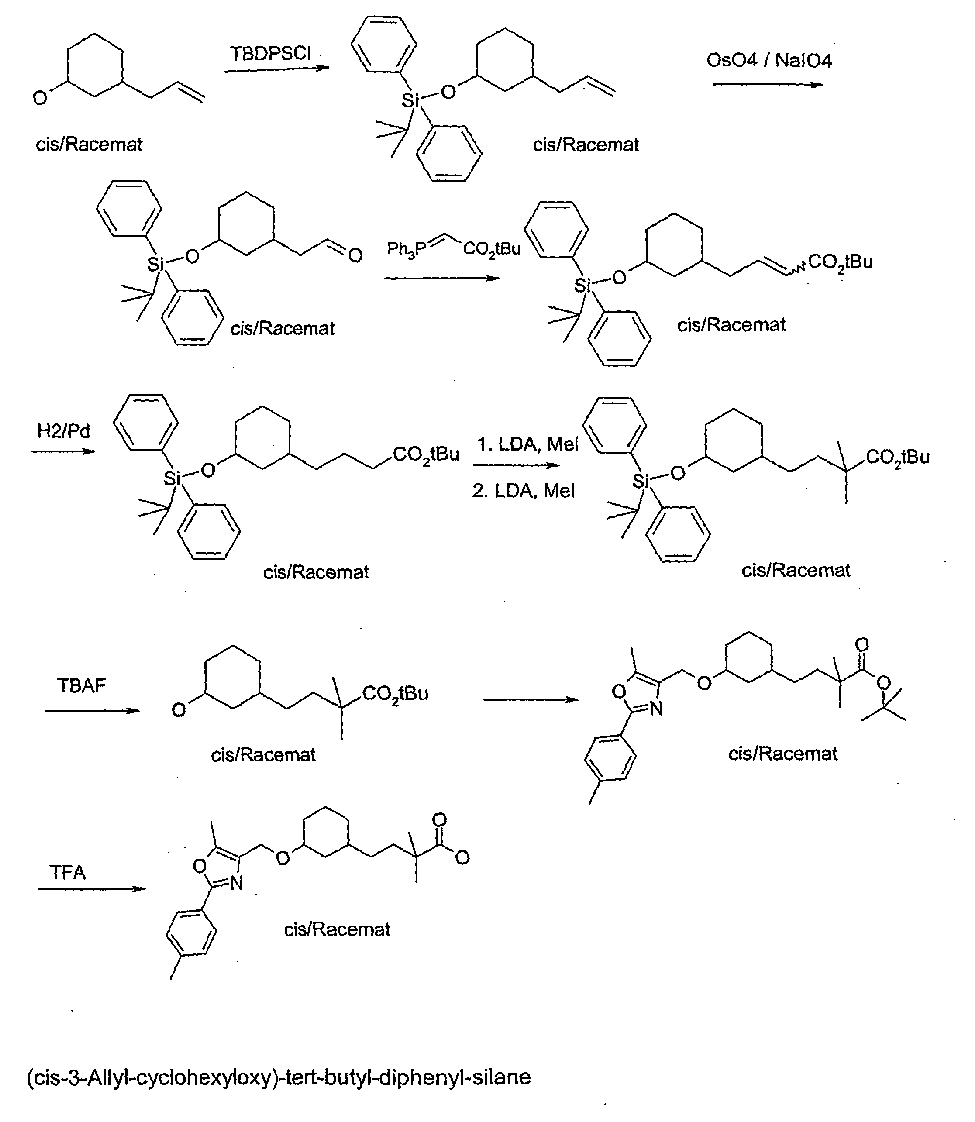

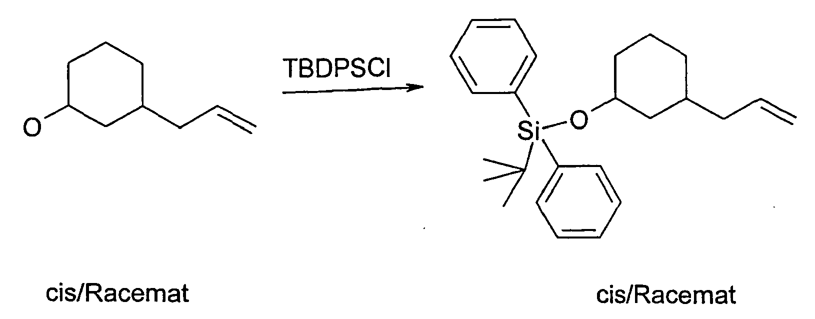

6.8 g cis-3-Allyl-cyclohexanol werden mit 15 ml ter-Butyldiphenylsilylchlorid, 5g Imidazol und 200 mg Dimethylaminopyridin in 100 ml Dimethylformamid gelöst und 12 h bei Raumtemperatur gerührt. Es werden 400 ml Methyltert-buthylether zum Reaktionsgemisch gegeben und dreimal mit Wasser gewaschen. Die organische Phase wird über MgSO4 getrocknet und anschließend das Lösungsmittel im Vakuum entfernt. Man erhält 20.5 g (cis-3-Allyl-cyclohexyloxy)-tert-butyl-diphenyl-silane als Öl. C25H34Osi (378.64), MS(ESI): 379 (M+H⁺), R_{f}(n-Heptan:Ethylacetat = 2:1) = 0.93.

### [cis-3-(tert-Butyl-diphenyl-silanyloxy)-cyclohexyl]-acetaldehyde

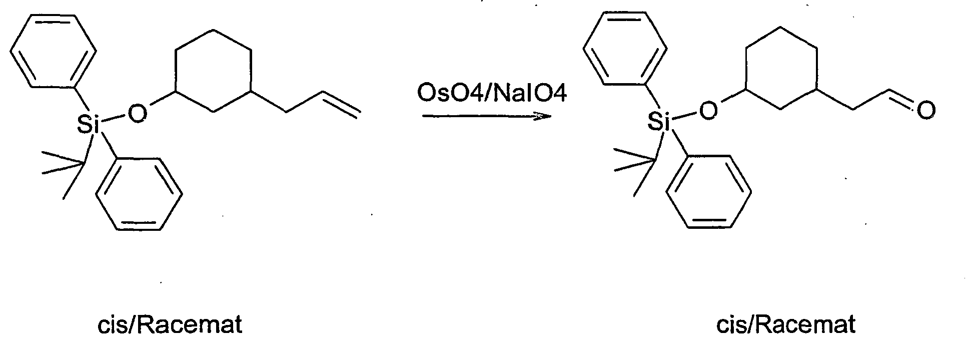

5.5 g (cis-3-Allyl-cyclohexyloxy)-tert-butyl-diphenyl-silane werden in 100 ml Diethylether gelöst und mit 9.4 g Natriumperiodat, gelöst in 100 ml Wasser, versetzt. Man gibt bei 0°C 15 ml einer Osmiumtetroxid-Lösung (2.5Gewichts% in tert-Butanol) hinzu und rührt kräftig bei Raumtemperatur nach. Nach 5 Stunden werden weiter 5g Natriumperiodat zugegeben und nochmals 3 Stunden bei Raumtemperatur gerührt. Danach wird das Reaktionsgemisch durch Zugabe von 300 ml Methyl-tert-buthylether verdünnt und mit gesättigter Natriumthiosulfat-Lösung gewaschen. Die organische Phase wird über MgSO4 getrocknet und anschließend das Lösungsmittel im Vakuum entfernt. Man erhält 6 g [cis-3-(tert-Butyl-diphenyl-silanyloxy)-cyclohexyl]-acetaldehyde als gelbbraunes Öl. C24H32O2Si (380.61), MS(ESI): 381 (M+H⁺), R_{f}(n-Heptan:Ethylacetat = 5:1) = 0.44.

### 4-[cis-3-(tert-Butyl-diphenyl-silanyloxy)-cyclohexyl]-but-2-ensäure-tert-butylester

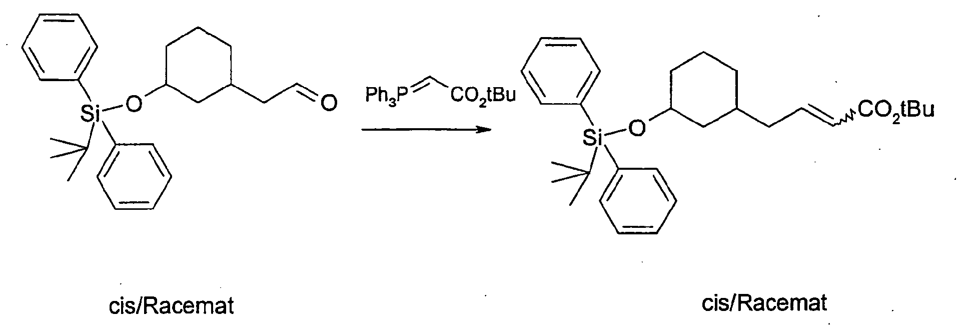

3.4 g [cis-3-(tert-Butyl-diphenyl-silanyloxy)-cyclohexyl]-acetaldehyde werden in 100 ml Dichlormethan gelöst und mit 5g (Triphenylphosphoranylidene)-essigsäuretertbutylester versetzt. Es wird 1 Stunde unter Rückfluss zum Sieden erhitzt. Das Gemisch wird mit gesättigter Natriumchlorid-Lösung gewaschen, über MgSO4 getrocknet und anschließend das Lösungsmittel im Vakuum entfernt. Der Rückstand wird an Kieselgel mit dem Laufmittel n-Heptan:Ethylacetat= 20:1 gereinigt. Man erhält 2.4 g 4-[cis-3-(tert-Butyl-diphenyl-silanyloxy)-cyclohexyl]-but-2-ensäure-tert-butylester als Öl. C30H4203Si (478.75), MS(ESI): 479 (M+H⁺), R_{f}(n-Heptan:Ethylacetat = 5:1) = 0.56.

### 4-[cis-3-(tert-Butyl-diphenyl-silanyloxy)-cyclohexyl]-butansäure-tert-butylester

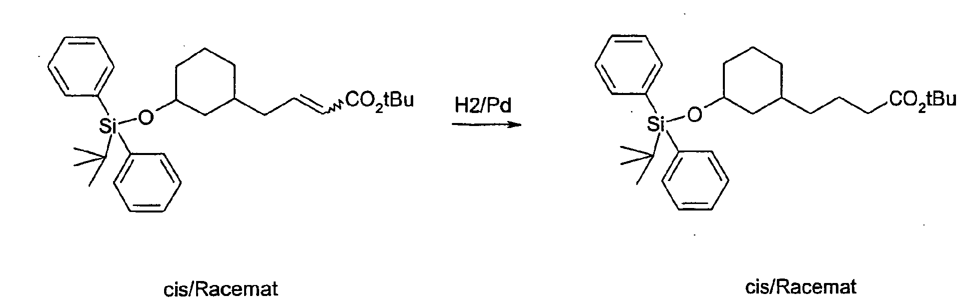

2.4 g 4-[cis-3-(tert-Butyl-diphenyl-silanyloxy)-cyclohexyl]-but-2-ensäure-tert-butylester werden in 35 ml Methanol gelöst und mit 200 mg Pd (10% auf Aktivkohle) versetzt. Es wird unter einer Wasserstoffatmosphäre 7 Stunden bei Raumtemperatur gerührt. Der Katalysator wird über Celite abfiltriert und das Filtrat im Vakuum eingeengt. Man erhält 2.3 g 4-[cis-3-(tert-Butyl-diphenyl-silanyloxy)-cyclohexyl]-butansäure-tert-butylester als Öl. C30H44O3Si (480.75), MS(ESI): 481 (M+H⁺).

### 4-[cis-3-(tert-Butyl-diphenyl-silanyloxy)-cyclohexyl]-2,2-dimethyl-buttersäure-tert-butylester

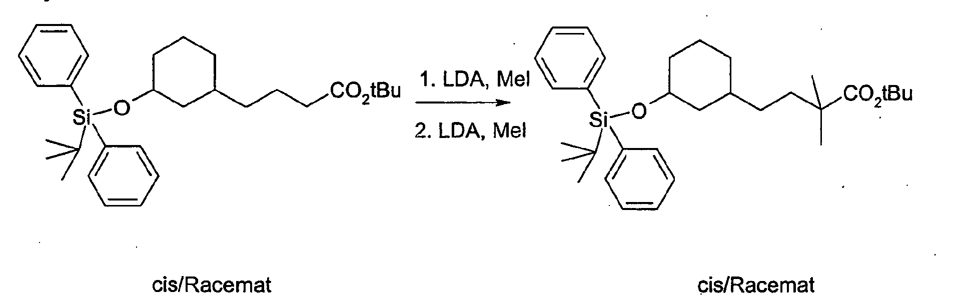

2 g 4-[cis-3-(tert-Butyl-diphenyl-silanyloxy)-cyclohexyl]-butansäure-tert-butylester werden in 20 ml Tetrahydrofuran gelöst und bei -78°C mit 3.1 ml einer 2M Lösung von Lithiumdiisopropylamid in Tetrahydrofuran versetzt. Man rührt 2 Stunden bei -78°C nach, dann wird das Reaktionsgemisch auf -30 °C erwärmt und mit 1.6 ml Methyliodid versetzt. Innerhalb von 12 Stunden lässt man auf Raumtemperatur erwärmen. Danach wird das Reaktionsgemisch durch Zugabe von 150 ml Methyl-tert-buthylether verdünnt und mit gesättigter NaCl-Lösung gewaschen. Die organische Phase wird über MgSO4 getrocknet und anschließend das Lösungsmittel im Vakuum entfernt. Der Rückstand wird an Kieselgel mit dem Laufmittel n-Heptan:Ethylacetat = 10:1 gereinigt. Man erhält 2.1 g des Monomethylierten Produktes. Dieses Produkt wird in 20 ml Tetrahydrofuran gelöst und bei -78°C mit 6 ml einer 2M Lösung von Lithiumdiisopropylamid in Tetrahydrofuran versetzt. Man rührt 2 Stunden bei -78°C nach, dann wird das Reaktionsgemisch auf 0 °C erwärmt und nach 10 Minuten bei 0°C mit 2.5 ml Methyliodid versetzt. Innerhalb von 12 Stunden lässt man auf Raumtemperatur erwärmen. Danach wird das Reaktionsgemisch durch Zugabe von 150 ml Methyl-tert-buthylether verdünnt und mit gesättigter NaCl-Lösung gewaschen. Die organische Phase wird über MgSO4 getrocknet und anschließend das Lösungsmittel im Vakuum entfernt. Der Rückstand wird an Kieselgel mit dem Laufmittel n-Heptan:Ethylacetat = 10:1 gereinigt. Man erhält 1.8 g 4-[cis-3-(tert-Butyl-diphenyl-silanyloxy)-cyclohexyl]-2,2-dimethyl-buttersäure-tert-butylester als Öl. C32H48O3Si (508.82), R_{f}(n-Heptan:Ethylacetat = 5:1) = 0.49.

### 4-(cis-3-Hydroxy-cyclohexyl)-2,2-dimethyl-buttersäure-tert-butylester

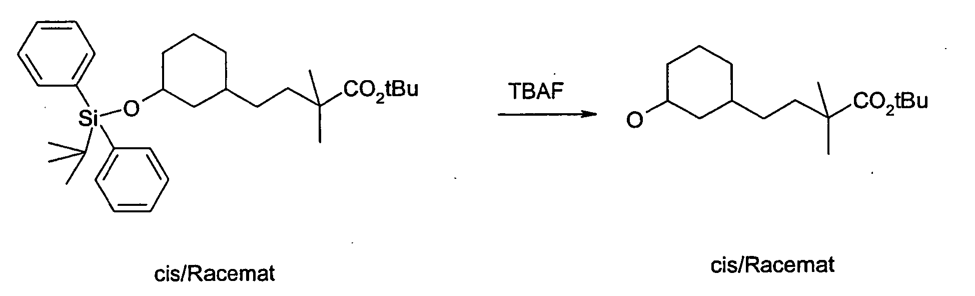

2 g 4-[cis-3-(tert-Butyl-diphenyl-silanyloxy)-cyclohexyl]-2,2-dimethyl-buttersäure-tert-butylester werden in 10 ml Tetrahydrofuran gelöst und mit 8 ml einer 1M Lösung von Tetrabutylammoniumfluorid in Tetrahydrofuran versetzt. Man rührt bei 2 Stunden bei 60°C nach. Das Reaktionsgemisch wird im Vakuum eingeengt und an Kieselgel mit dem Laufmittel n-Heptan:Ethylacetat = 20:1 => 1:1 gereinigt. Man erhält 730 mg 4-(cis-3-Hydroxy-cyclohexyl)-2,2-dimethyl-buttersäure-tert-butylester als Öl. C16H30O3 (270.42), R_{f}(n-Heptan:Ethylacetat = 5:1) = 0.22.

### 2,2-Dimethyl-4-[cis-3-(5-methyl-2-p-tolyl-oxazol-4-ylmethoxy)-cyclohexyl]-buttersäure-tert-butylester

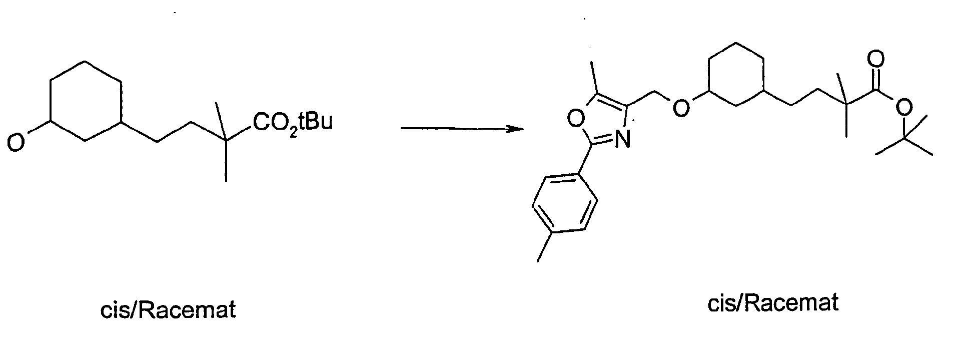

365 mg 4-(cis-3-Hydroxy-cyclohexyl)-2,2-dimethyl-buttersäure-tert-butylester werden zusammen mit 850 mg 4-lodmethyl-5-methyl-2-(4 methyl-phenyl)-oxazol in 5 ml Dimethylformamid gelöst und mit 110 mg Natriumhydrid (60%ig in Paraffin) versetzt. Nach 1 Stunde Rühren bei Raumtemperatur wird 100 ml Methyl-tert-butylether zugegeben und das Reaktionsgemisch dreimal mit Wasser gewaschen. Die organische Phase wird über MgSO4 getrocknet und anschließend das Lösungsmittel im Vakuum entfernt. Der Rückstand wird mittels RP-HPLC gereinigt. Man erhält 330 mg 2,2-Dimethyl-4-[cis-3-(5-methyl-2-p-tolyl-oxazol-4-ylmethoxy)-cyclohexyl]-buttersäure-tert-butylester als weißen Feststoff. C28H41NO4 (455.64), MS(ESI): 456 (M+H⁺).

### 2,2-Dimethyl-4-[cis-3-(5-methyl-2-p-tolyl-oxazol-4-ylmethoxy)-cyclohexyl]-buttersäure

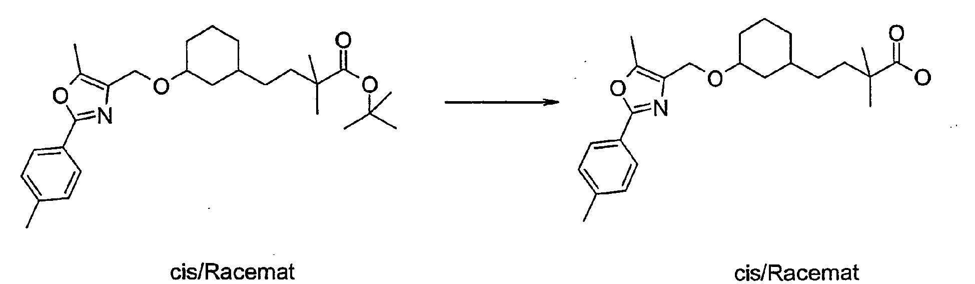

300 mg 2,2-Dimethyl-4-[cis-3-(5-methyl-2-p-tolyl-oxazol-4-ylmethoxy)-cyclohexyl]-buttersäure-tert-butylester werden in 20 ml Dichlormethan gelöst und mit 10 ml Trifluoressigsäure versetzt. Man rührt 1 Stunde bei Raumtemperatur nach. Es werden 200 ml Toluol zugegeben und dann die Lösungsmittel im Vakuum eingeengt. Der Rückstand wird mittels RP-HPLC gereinigt. Man erhält 180 mg 2,2-Dimethyl-4-[cis-3-(5-methyl-2-p-tolyl-oxazol-4-ylmethoxy)-cyclohexyl]-buttersäure als Öl. C24H33NO4 (399.53), MS(ESI): 400 (M+H⁺).

### Beispiel 72:

Analog zu Beispiel 71 wurden aus 4-(cis-3-Hydroxy-cyclohexyl)-2,2-dimethylbuttersäure-tert-butylester und 4-lodmethyl-5-methyl-2-(3-trifluormethyl-phenyl)-oxazol 2,2-Dimethyl-4-{3-[5-methyl-2-(3-trifluoromethyl-phenyl)-oxazol-4-ylmethoxy]-cyclohexyl}-butyric acid erhalten. 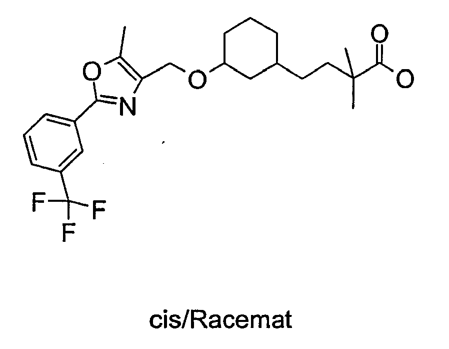 C24H30F3NO4 (453.50), MS(ESI): 454 (M+H⁺).

### Beispiel 73:

### 3-Methyl-2-{2-[cis-3-(5-methyl-2-p-tolyl-oxazol-4-ylmethoxy)-cyclohexyl]-ethyl}-butyric acid

### 2-(Diethoxy-phosphoryl)-3-methyl-buttersäure-tert-butylester

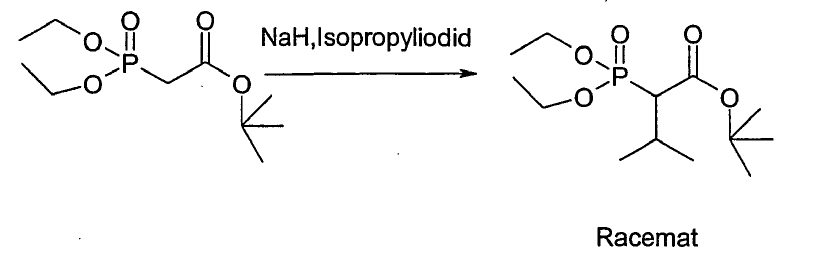

5.5 ml tert-Butyl-diethylphosphonoacetat werden in 20 ml Dimethylformamid gelöst und bei 0°C portionsweise mit 820 mg Natriumhydrid (60%ig in Paraffinöl) versetzt. Die Suspension wird 15 Minuten bei 0°C gerührt und dann mit 2.4 ml Isopropyliodid versetzt. Es wird 12 Stunden bei Raumtemperatur gerührt. Dann werden 250 ml Ethylacetat zugegeben und das Reaktionsgemisch dreimal mit je 150 ml Wasser gewaschen. Die organische Phase wird über MgSO4 getrocknet und im Vakuum eingeengt. Der Rückstand wird an Kieselgel mit dem Laufmittel n-Heptan:Ethylacetat = 5:1 gereinigt. Man erhält 4.2 g 2-(Diethoxy-phosphoryl)-3-methyl-buttersäure-tert-butylester als Öl. C13H27O5P (294.33), MS(ESI): 239 (M-C₄H₈+H⁺), R_{f}(n-Heptan:Ethylacetat = 1:1) = 0.34.

### 4-[cis-3-(tert-Butyl-diphenyl-silanyloxy)-cyclohexyl]-2-isopropyl-butyr-2-ensäure-tert-butylester

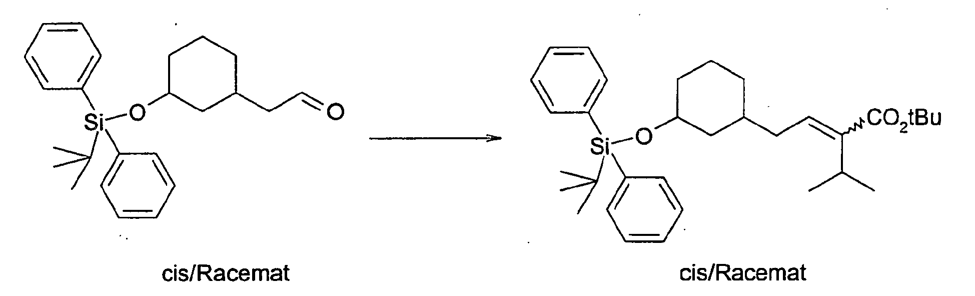

770 mg 2-(Diethoxy-phosphoryl)-3-methyl-buttersäure-tert-butylester werden in 10 ml Tetrahydrofuran gelöst und bei -20°C mit 0.73 ml einer 2.7 M Lösung von n-Butyllithium in n-Hexan versetzt. Nach 1 Stunde Rühren bei -20°C werden 500 mg [cis-3-(tert-Butyl-diphenyl-silanyloxy)-cyclohexyl]-acetaldehyde, gelöst in 5 ml Tetrahydrofuran, zugetropft. Das Reaktionsgemisch wird langsam auf Raumtemperatur erwärmt. Dann wird 20 ml Wasser zugegeben und dreimal mit je 50 ml Ethylacetat extrahiert. Die vereinigten organischen Phasen werden über MgSO4 getrocknet und anschließend das Lösungsmittel im Vakuum entfernt. Der Rückstand wird an Kieselgel mit dem Laufmittel n-Heptan:Ethylacetat = 30:1 gereinigt. Man erhält 340 mg 4-[cis-3-(tert-Butyl-diphenyl-silanyloxy)-cyclohexyl]-2-isopropyl-butyr-2-ensäure-tert-butylester als Öl. C33H48O3Si (520.83), R_{f}(n-Heptan:Ethylacetat = 5:1) = 0.70.

### 4-[cis-3-(tert-Butyl-diphenyl-silanyloxy)-cyclohexyl]-2-isopropyl-buttersäure

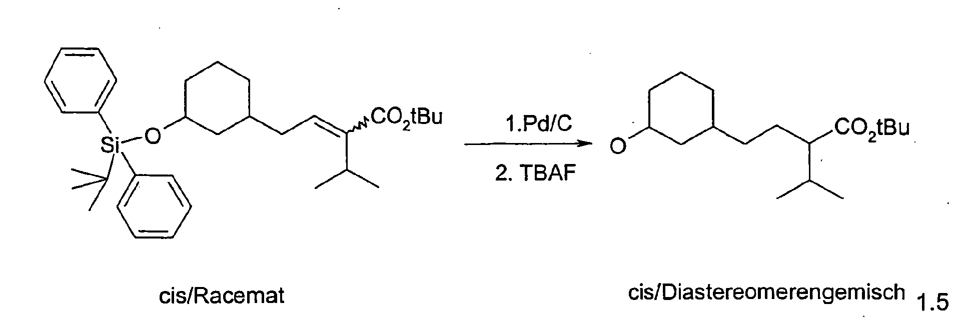

g 4-[cis-3-(tert-Butyl-diphenyl-silanyloxy)-cyclohexyl]-2-isopropyl-butyr-2-ensäure-tert-butylester werden in 30 ml Ethylacetat gelöst und mit 200 mg Perlman's Catalyst versetzt. Es wird 5 Stunden unter einer Wasserstoffatmosphäre (5 bar) gerührt. Der Katalysator wird über Celite abfiltriert und das Filtrat im Vakuum eingeengt. Der Rückstand wird in 15 ml Tetrahydrofuran gelöst und mit 3 ml einer 1M Lösung von Tetrabutylammoniumfluorid in Tetrahydrofuran versetzt. Es wird 2 Stunden bei 60°C gerührt. Das Reaktionsgemisch wird im Vakuum eingeengt und an Kieselgel mit dem Laufmittel n-Heptan:Ethylacetat = 40:1 => 10:1 gereinigt. Man erhält 400 mg 4-[cis-3-(tert-Butyl-diphenyl-silanyloxy)-cyclohexyn-2-isopropyl-buttersäure als Öl. C17H32O3 (284.44), MS(ESI): 211 (M-C₄H₉O⁻), R_{f}(n-Heptan:Ethylacetat = 10:1) = 0.15.

Analog zu Beispiel 73 wurde aus 4-lodmethyl-5-methyl-2-(4 methyl-phenyl)-oxazol und 4-[cis-3-(tert-Butyl-diphenyl-silanyloxy)-cyclohexyl]-2-isopropyl-buttersäure 3-Methyl-2-{2-[cis-3-(5-methyl-2-p-tolyl-oxazol-4-ylmethoxy)-cyclohexyl]-ethyl}-butyric acid erhalten. 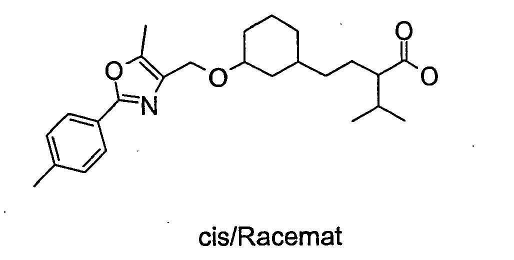 C25H35NO4 (413.56), MS(ESI): 414 (M+H⁺).

### Beispiel 74:

Analog zu Beispiel 73 wurde aus 4-lodmethyl-5-methyl-2-(3 methoxy-phenyl)-oxazol und 4-[cis-3-(tert-Butyl-diphenyl-silanyloxy)-cyclohexyl]-2-isopropyl-buttersäure 2-(2-{cis-3-[2-(3-Methoxy-phenyl)-5-methyl-oxazol-4-ylmethoxy]-cyclohexyl}-ethyl)-3-methyl-buttersäure erhalten. 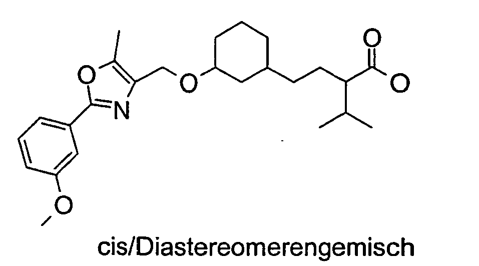 C25H35NO5 (429.56), MS(ESI): 430 (M+H⁺).

### Beispiel 75:

### 2-Benzyl-4-[cis-3-(5-methyl-2-p-tolyl-oxazol-4-ylmethoxy)-cyclohexyl]-buttersäure

Analog zu Beispiel 73 wurde aus tert-Butyl-diethylphosphonoacetat und Benzylbromid 2-(Diethoxy-phosphoryl)-3-phenyl-propionsäure-tert-butylester erhalten. 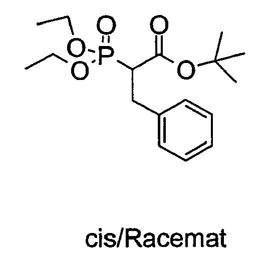 C17H27O5P (342.38), R_{f}(n-Heptan:Ethylacetat = 1:1) = 0.53.

Analog zu Beispiel 73 wurde aus 2-(Diethoxy-phosphoryl)-3-phenyl-propionsäure-tert-butylester, [cis-3-(tert-Butyl-diphenyl-silanyloxy)-cyclohexyl]-acetaldehyde und 4-lodmethyl-5-methyl-2-(4 methyl-phenyl)-oxazol 2-Benzyl-4-[cis-3-(5-methyl-2-p-tolyl-oxazol-4-ylmethoxy)-cyclohexyl]-buttersäure erhalten. 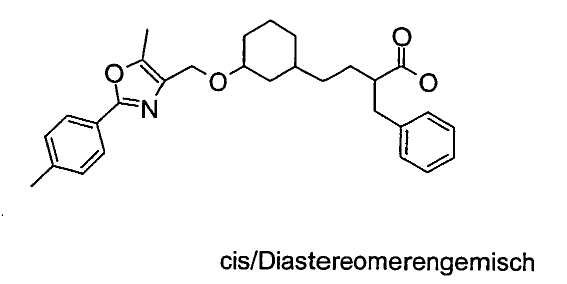 C29H35NO4 (461.61), MS(ESI): 462 (M+H⁺).

### Beispiel 76:

### 4-Methyl-2-{2-[cis.3-(5-methyl-2-p-tolyl-oxazol-4-ylmethoxy)-cyclohexyl]-ethyl}-pentansäure

Analog zu Beispiel 73 wurde aus 4-[cis-3-(tert-Butyl-diphenyl-silanyloxy)-cyclohexyl]-butansäure-tert-butylester, 3-Bromo-2-methyl-propene und 4-lodmethyl-5-methyl-2-(4 methyl-phenyl)-oxazol 4-Methyl-2-{2-[cis-3-(5-methyl-2-p-tolyl-oxazol-4-ylmethoxy)-cyclohexyl]-ethyl}-pent-4-ensäure-tert-butylester erhalten. 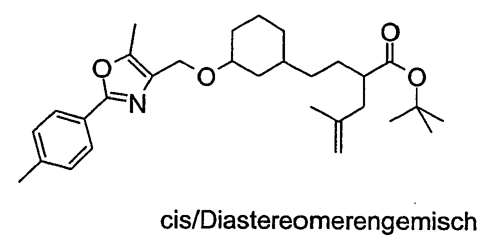 C30H43NO4 (481.68), MS(ESI): 482 (M+H⁺).

### 4-Methyl-2-{2-[cis.3-(5-methyl-2-p-tolyl-oxazol-4-ylmethoxy)-cyclohexyl]-ethyl}-pentansäure

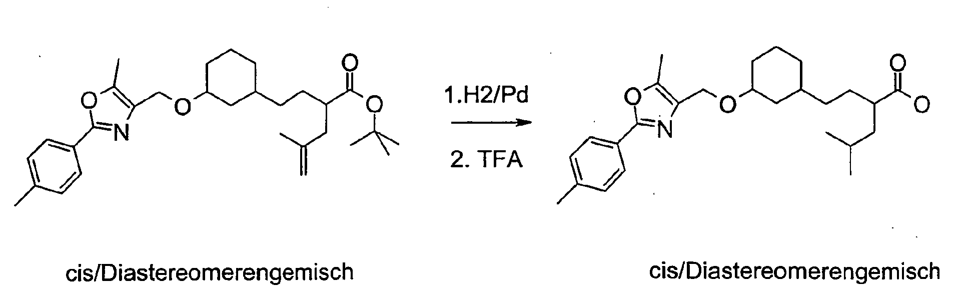

500 mg 4-Methyl-2-{2-[cis-3-(5-methyl-2-p-tolyl-oxazol-4-ylmethoxy)-cyclohexyl]-ethyl}-pent-4-ensäure-tert-butylester werden in 20 ml Ethylacetat gelöst und mit 50 mg Palladium (10% auf Aktivkohle) versetzt. Es wird 5 Stunden unter einer Wasserstoffatmosphäre (5 bar) gerührt. Der Katalysator wird über Celite abfiltriert und das Filtrat im Vakuum eingeengt. Der Rückstand wird in 20 ml Dichlormethan gelöst und mit 10 ml Trifluoressigsäure versetzt. Man rührt 1 Stunde bei Raumtemperatur nach. Es werden 100 ml Toluol zugegeben und dann die Lösungsmittel im Vakuum entfernt. Der Rückstand wird mittels RP-HPLC gereinigt. Man erhält 100 mg 4-Methyl-2-{2-[cis.3-(5-methyl-2-p-tolyl-oxazol-4-ylmethoxy)-cyclohexyl]-ethyl}-pentansäure als Öl. C26H37NO4 (427.59), MS(ESI): 428 (M+H⁺).

### Beispiel 77:

### 2-(2-{cis-3-[5-Methyl-2-(3-trifluoromethyl-phenyl)-oxazol-4-ylmethoxy]-cyclohexyl}-ethyl)-2-propyl-pentansäure

Analog zu Beispiel 71 wurde aus 4-[cis-3-(tert-Butyl-diphenyl-silanyloxy)-cyclohexyl]-butansäure-tert-butylester und Allylbromid 2-Allyl-2-{2-[3-(tert-butyl-diphenylsilanyloxy)-cyclohexyl]-ethyl}-pent-4-ensäure-tert-butylester erhalten. 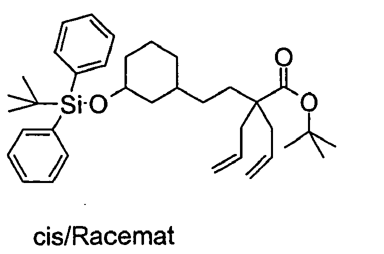 C36H52O3Si (560.90), R_{f}(n-Heptan:Ethylacetat = 20:1) = 0.60.

Analog zu Beispiel 71 und Beispiel 76 wurde aus 2-Allyl-2-{2-[cis-3-(tert-butyl-diphenylsilanyloxy)-cyclohexyl]-ethyl}-pent-4-ensäure-tert-butylester und 4-lodmethyl-5-methyl-2-(3-trifluormethyl-phenyl)-oxazol 2-(2-{cis-3-[5-Methyl-2-(3-trifluoromethyl-phenyl)-oxazol-4-ylmethoxy]-cyclohexyl}-ethyl)-2-propyl-pentansäure erhalten. 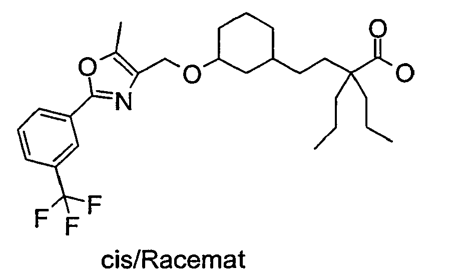 C28H38F3NO4 (509.61), MS(ESI): 510 (M+H⁺).

### Beispiel 78:

### 1-{2-[cis-3-(5-Methyl-2-p-tolyl-oxazol-4-ylmethoxy)-cyclohexyl]-ethyl}-cyclopentancarbonsäure

### 1-{2-[cis-3-(tert-Butyl-diphenyl-silanyloxy)-cyclohexyl]-ethyl}-cyclopent-3-enecarbonsäure-tert-butylester

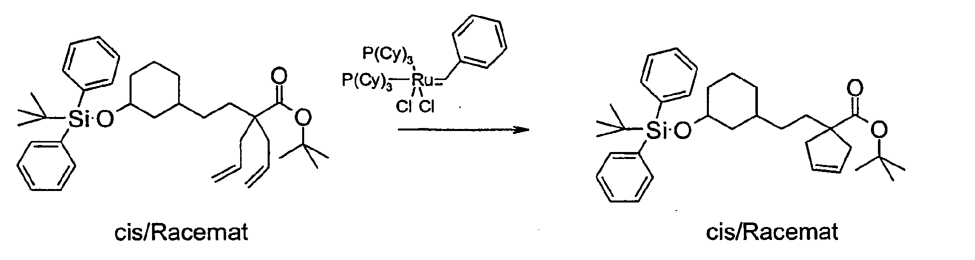

2 g 2-Allyl-2-{2-[cis-3-(tert-butyl-diphenyl-silanyloxy)-cyclohexyl]-ethyl}-pent-4-ensäure-tert-butylester werden in 100 ml Dichlormethan gelöst. Man leitet 5 Minuten Argon durch die Lösung hindurch. Dann wird 100 mg Grubbs Katalysator hinzugesetzt. Es wird 2 Stunden bei 40°C gerührt. Danach wiord das Lösungsmittel im Vakuum entfernt und der Rückstand an Kieselgel mit dem Laufmittel n-Heptan:Ethylacetat = 40:1 gereinigt. Man erhält 1.4 g 1-{2-[cis-3-(tert-Butyl-diphenyl-silanyloxy)-cyclohexyl]-ethyl}-cyclopent-3-enecarbonsäure-tert-butylester als Öl. C34H48O3Si (532.85), R_{f}(n-Heptan:Ethylacetat = 20:1) = 0.56.

Analog zu Beispiel 77 wurde aus 1-{2-[cis-3-(tert-Butyl-diphenyl-silanyloxy)-cyclohexyl]-ethyl}-cyclopent-3-enecarbonsäure-tert-butylester und 4-lodmethyl-5-methyl-2-(4-methyl-phenyl)-oxazol 1-{2-[cis-3-(5-Methyl-2-p-tolyl-oxazol-4-ylmethoxy)-cyclohexyl]-ethyl}-cyclopentancarbonsäure erhalten. 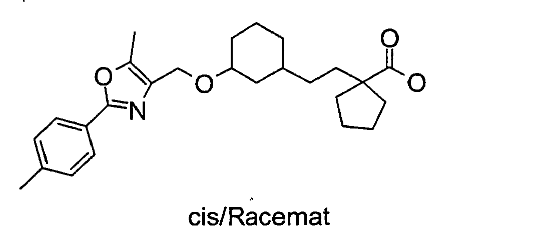 C26H35NO4 (425.57), MS(ESI): 426 (M+H+).

### Beispiel 79:

### 2-(4-Methoxyphenoxycarbonyl)-{2-[cis-3-(5-methyl-2-m-tolyl-oxazol-4-ylmethoxy)-cyclohexyl]-ethyl}-amino)-3-methyl-buttersäure

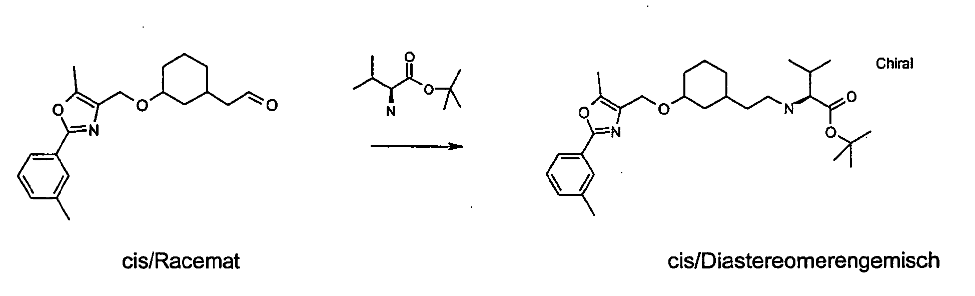

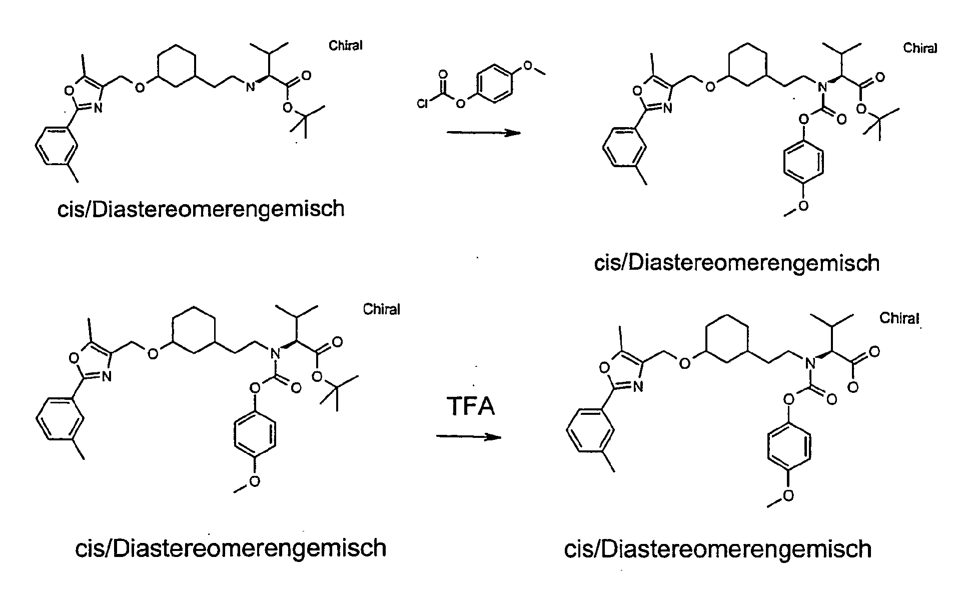

### (S)-3-Methyl-2-{2-[cis-3-(5-methyl-2-m-tolyl-oxazol-4-ylmethoxy)-cyclohexyl]-ethylamino}- buttersäure-tert-butylester

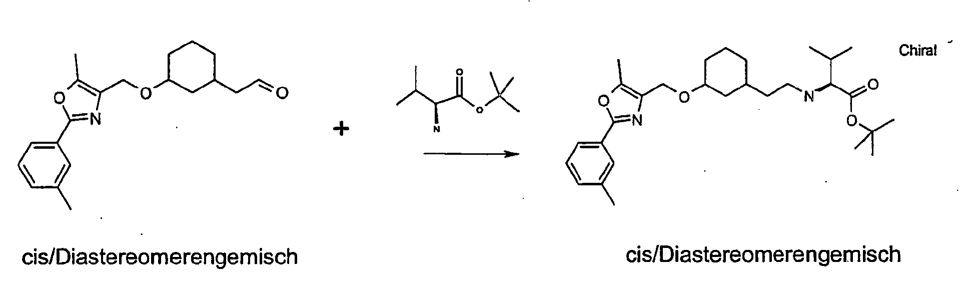

0,1g {cis-3-[2-(3-Methylphenyl)-5-methyl-oxazol-4-ylmethoxy]-cyclohexyl}-acetaldehyde (hergestellt nach dem Verfahren E4) werden in Methylenchlorid vorgelegt und eine Spatelspitze Magnesiumsulfat wird zugesetzt. Danach werden 64 mg Amin zugegeben, die Mischung auf 0°C gekühlt und mit 30 mg Natriumacetat versetzt und 30 min nachgerührt. 84 mg Natriumtriacetoxyborhydrid werden zugegeben und die Mischung über Nacht bei RT gerührt. Man gibt 8 ml Wasser zur Reaktion und filtriert über eine Kieselgurkartusche, wäscht mit 50ml Methylenchlorid nach und entfernt das Lösungsmittel am Vakuum. Man erhält 0,13g (S)-3-Methyl-2-{2-[cis-3-(5-methyl-2-m-tolyl-oxazol-4-ylmethoxy)-cyclohexyl]-ethylamino}-butyric acid tert-butyl ester. C29H44N2O4 (484), MS (ESI): 485 (M+H).

### (S)-2-((4-Methoxy-phenoxycarbonyl)-{2-[cis-3-(5-methyl-2-m-tolyl-bxazol-4-ylmethoxy)-cydohexyl]-ethyl}-amino)-3-methylbuttersäure-tert-butyl ester

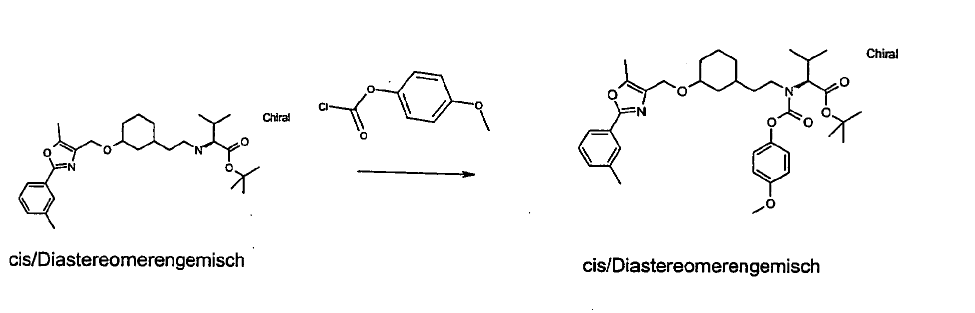

130 mg (S)-3-Methyl-2-{2-[cis-3-(5-methyl-2-m-tolyl-oxazol-4-ylmethoxy)-cyclohexyl]-ethylamino}- buttersäure-tert-butylester Werden in 1 ml Methylenchlorid gelöst, und mit 2 ml ges. Natriumcarbonat-Lösung versetzt und auf 0°C gekühlt, anschließend werden ein Überschuß des Säurechlorids zugegeben und es wird 1 h bei RTgerührt. Die gesamte Reaktionslösung wird über eine Kieselgurkartusche gegeben und mit 20ml Methylenchlorid nachgewaschen. Das Lösemittel wird am Vakuum entfernt. Man erhält 0,1g (S)-2-((4-Methoxy-phenoxycarbonyl)-{2-[cis-3-(5-methyl-2-m-tolyl-oxazol-4-ylmethoxy)-cyclohexyl]-ethyl}-amino)-3-methylbuttersäure-tert-butyl ester.
C37H50N2O7 (635), MS (ESI):636 (M+H)

### (S)-2-((4-Methoxy-phenoxycarbonyl)-{2-[cis-3-(5-methyl-2-m-tolyl-oxazol-4-ylmethoxy)-cyclohexyl]-ethyl}-amino)-3-methylbuttersäure

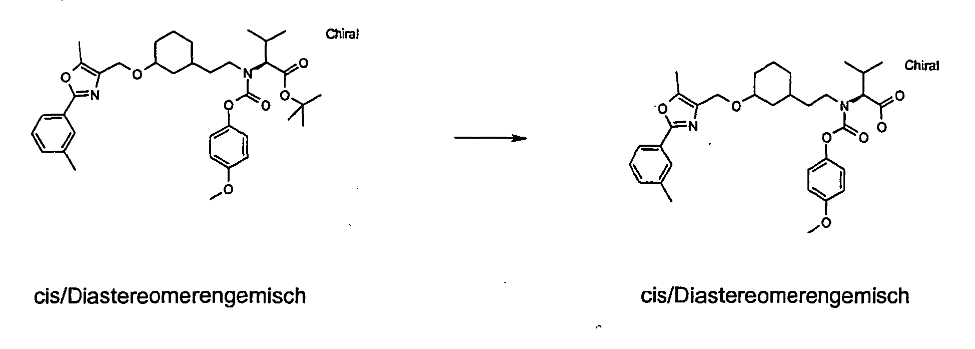

0,10 g (S)-2-((4-Methoxy-phenoxycarbonyl)-{2-[cis-3-(5-methyl-2-m-tolyl-oxazol-4-ylmethoxy)-cyclohexyl]-ethyl}-amino)-3-methylbuttersäure-tert-butyl ester werden in 2ml Dichlormethan gelöst und mit 1 ml Trifluoressigsäure bei RT über Nacht gerührt. Dann wird das Lösemittel komplett entfernt und über die präparative HPLC gereinigt. Man erhält 15,6 mg (S)-2-((4-Methoxy-phenoxycarbonyl)-{2-[cis-3-(5-methyl-2-m-tolyl-oxazol-4-ylmethoxy)-cyclohexyl]-ethyl}-amino)-3-methylbuttersäure. C33H42N2O7 (579), MS (ESI): 580 (M+H)

### Beispiel 80:

Analog zu Beispiel 79 wurden aus [cis-3-(5-Methyl-2-m-tolyl-oxazol-4-ylmethoxy)-cyclohexyl]-acetaldehyde und 1-Amino-cyclopentanecarbonsäuremethylester, 1-(Benzyloxycarbonyl-{2-[cis-3-(5-methyl-2-m-tolyl-oxazol-4-ylmethoxy)-cyclohexyl]-ethyl}-amino)-cyclopentancarbonsäure erhalten. 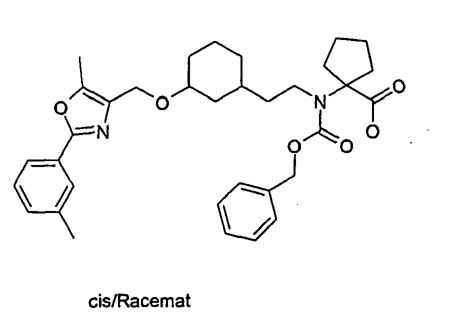 C34H42N2O6 (574,7), MS(ESI): 575 (M+H)

### Beispiel 81:

Analog zu Beispiel 79 wurden aus cis-3-(5-Methyl-2-m-tolyl-oxazol-4-ylmethoxy)-cyclohexyl]-acetaldehyde und 1-Amino-cyclopentanecarbonsäuremethylester, 1-((4-Methoxy-benzyloxycarbonyl)-{2-[cis-3-(5-methyl-2-m-tolyl-oxazol-4-yl methoxy)-cyclohexyl]-ethyl}-amino)-cyclopentancarbonsäure erhalten. 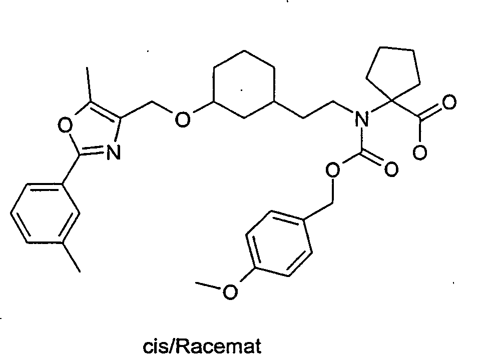 C35H44N2O7 (604,7), MS(ESI): 605 (M+H)

### Beispiel 82:

Analog zu Beispiel 79 wurden aus cis-3-(5-Methyl-2-m-tolyl-oxazol-4-ylmethoxy)-cyclohexyl]-acetaldehyde und (S)-2-Amino-2-methyl-3-phenyl-propionsäuremethylester, (R)-2-((4-Methoxy-benzyloxycarbonyl)-{2-[cis-3-(5-methyl-2-m-tolyl-oxazol-4-ylmethoxy)-cyclohexyl]-ethyl}-amino)-2-methyl-3-phenyl-propionsäure erhalten. 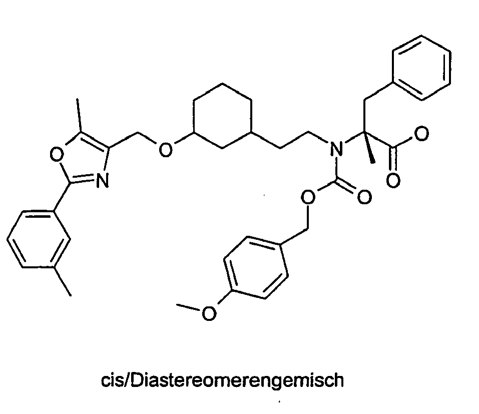 C39H46N2O7 (654,8), MS(ESI): 656 (M+H)

### Beispiel 83:

Analog zu Beispiel 79 wurden aus cis-3-(5-Methyl-2-m-tolyl-oxazol-4-ylmethoxy)-cyclohexyl]-acetaldehyde und (S)-2-Amino-2-methyl-3-phenylpropionsäuremethylester, (Benzyloxycarbonyl-{2-[cis-3-(5-methyl-2-m-tolyl-oxazol-4-ylmethoxy)-cyclohexyl]-ethyl}-amino)-essigsäure erhalten 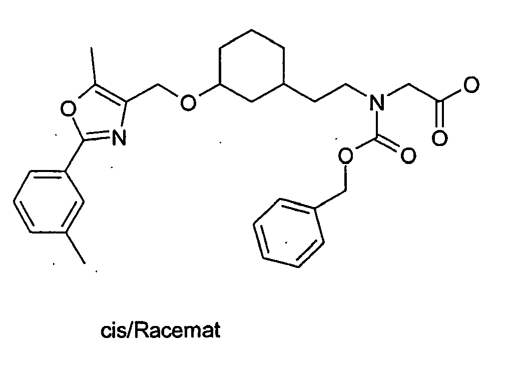 C30H36N2O7 (520,6), MS(ESI): 521 (M+H)

### Beispiel 84:

Analog zu Beispiel 79 wurden aus cis-3-(5-Methyl-2-m-tolyl-oxazol-4-ylmethoxy)-cyclohexyl]-acetaldehyde und (S)-2-Amino-2-methyl-3-phenylpropionsäuremethylester, ({2-[cis-3-(5-Methyl-2-m-tolyl-oxazol-4-ylmethoxy)-cyclohexyl]-ethyl}-phenylacetyl-amino)-essigsäure erhalten. 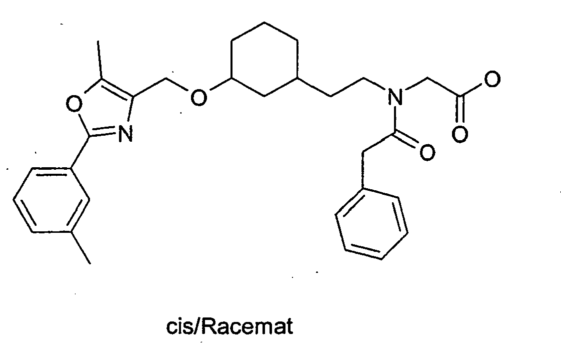 C30H36N2O5 (504,6), MS(ESI): 505 (M+H)

### Beispiel 85:

Analog zu Beispiel 79 wurden aus cis-3-(5-Methyl-2-m-tolyl-oxazol-4-ylmethoxy)-cyclohexyl]-acetaldehyde und (S)-2-Amino-2-methyl-3-phenylpropionsäuremethylester, (1-{2-[cis-3-(5-Methyl-2-m-tolyl-oxazol-4-ylmethoxy)-cyclohexyl]-ethyl}-3-phenyl-ureido)-essigsäure erhalten. 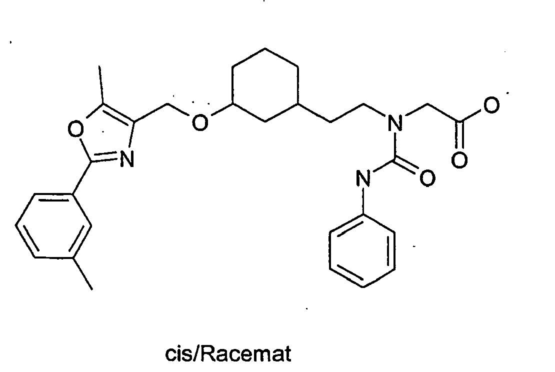 C29H35N2O5 (505,6), MS(ESI): 506 (M+H)

## Patentansprüche

1. Verbindungen der Formel I 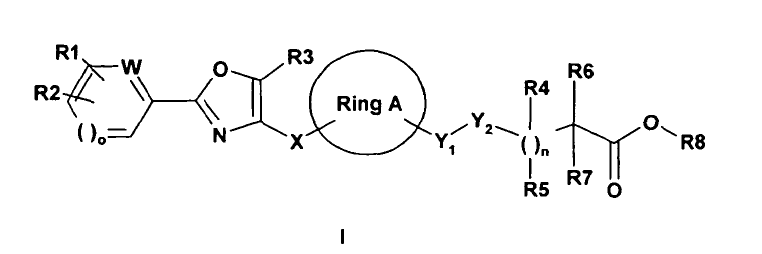 worin bedeuten:
Ring A Cyclohexan-1,3-diyl;
R1, R2 unabhängig voneinander H, F, Cl, Br, CF3, OCF3, (C1-C6)-Alkyl, O-(C1-C6)-Alkyl, SCF3, SF5, OCF2-CHF2, (C6-C10)-Aryl, (C6-C10)-Aryloxy, OH, NO2; oder
R1 und R2 zusammengenommen mit dem Phenyl-, Pyridin-, 1-H-Pyrrol-, Thiophen- oder Furan-Ring kondensiertes, teilweise oder nicht gesättigtes bicyclisches (C6-C10)-Aryl, (C5-C11)-Heteroaryl;
R3 H, (C1-C6)-Alkyl, (C3-C8)-Cycloalkyl, (C1-C3)-Alkyl-(C3-C8)-Cycloalkyl, Phenyl, (C1-C3)-Alkyl-Phenyl , (C5-C6)-Heteroaryl, (C1-C3)-Alkyl-(C5-C6)-Heteroaryl oder (C1-C3)-Alkyl, das durch F ganz oder teilweise substituiert ist;
o 0 oder 1;
W CH, N, falls o = 1;
W O, S, NR10, falls o = 0;
X (C1-C6)-Alkandiyl, wobei in der Alkandiylgruppe ein oder mehrere Kohlenstoffatome durch Sauerstoffatome ersetzt sein können;
Y1 (CR13R14)p;
p 1, 2;
Y2 CH2, O, S, SO, SO2, NR9;
n 0-2;
R4 H, (C1-C6)-Alkyl; oder F, falls Y2 ungleich O, NR9;
R5 H, (C1-C6)-Alkyl; oder F, falls Y2 ungleich O, NR9;
R6 H, (C1-C6)-Alkyl; oder F, falls n ungleich 0;
R7 H, F (falls n ungleich 0), (C1-C6)-Alkyl, (C1-C6)-Alkoxy, (C2-C6)-Alkenyl, (C2-C6)-Alkinyl, (C3-C8)-Cycloalkyl, Phenyl, wobei Alkyl gegebenenfalls substituiert sein kann durch ein oder mehrere Reste aus der Reihe Hydroxy, Phenyl, (C5-C11)-Heteroaryl, (C1-C6)-Alkoxy und NR11R12, und Phenyl gegebenfalls substituiert sein kann durch ein oder mehrere Reste aus der Reihe Hydroxy, (C1-C6)-Alkoxy, F und CF3; mit der Maßgabe, dass R7 ungleich NR11 R12 oder (C1-C6)-Alkoxy, falls R6 = F ist;
R6 und R7 zusammen mit dem sie tragenden C-Atom (C3-C8)-Cycloalkyl;
R8 H, (C1-C6)-Alkyl;
R9 H, (C1-C6)-Alkyl, (C2-C6)-Alkenyl, (C2-C6)-Alkinyl, Aryl-(C1-C4)alkyl, CO-(C1-C6)-Alkyl, CO-(C₆-C₁₀)-Aryl, CO-(C1-C6)-Alkyl-(C6-C10)-Aryl, CO-(C5-C11)-Heteroaryl, C(O)-O-(C1-C6)-Alkyl, C(O)-O-(C1-C6)-Alkyl-(C6-C10)-Aryl, C(O)-O-(C6-C10)-Aryl, C(O)-O-(C5-C11)-Heteroaryl, SO2-(C1-C6)-Alkyl, SO2-(C1-C6)-Alkyl-(C6-C10)-Aryl, SO2-(C1-C6)-Alkyl-S02-(C1-C6)-alkyl, SO2-(C6-C10)-Aryl, SO2-(C5-C11)-Heteroaryl, wobei Aryl und Heteroaryl gegebenenfalls substituiert sein können durch (C1-C6)-Alkyl, O-(C1-C6)-Alkyl, F, Cl, CO-(C1-C6)-Alkyl;
R10 H, (C1-C6)-Alkyl, (C1-C6)-Alkyl-Phenyl;
R11 H, (C1-C6)-Alkyl, (C1-C6)-Alkyl-Phenyl;
R12 H, (C1-C6)-Alkyl, (C1-C6)-Alkyl-Phenyl;
R13 H, (C1-C6)-Alkyl;
R14 H, (C1-C6)-Alkyl;
sowie deren physiologisch verträgliche Salze.

2. Verbindungen der Formel I gemäß Anspruch 1, worin bedeuten
Ring A Cyclohexan-1,3-diyl;
X (C1-C6)-Alkandiyl, wobei in der Alkandiylgruppe das C1- oder C2-Kohlenstoffatom (zum Ring A) durch Sauerstoffatom ersetzt ist.

3. Verbindungen der Formel I gemäß einem oder mehreren der Ansprüche 1 bis 2, worin bedeuten
Ring A cis-Cyclohexan-1,3-diyl,
R1, R2 unabhängig voneinander H, F, CF3, (C1-C6)-Alkyl, O-(C1-C6)-Alkyl, Phenyl, oder
R1 und R2 zusammengenommen mit dem Phenylring = Naphthyl;
R3 (C1-C6)-Alkyl, (C3-C8)-Cycloalkyl, Phenyl;
o 0 oder 1;
W CH, falls o = 1;
W O, S, falls o = 0;
X CH2-O, CH2-O-CH2;
Y1 CH2;
Y2 CH2, O S, SO, SO2, NR9;
n 0;
R4 H;
R5 H;
R6 H, (C1-C6)-Alkyl, Benzyl;
R7 H, (C1-C6)-Alkyl, (C3-C6)-Cycloalkyl, Phenyl, Benzyl,
R6 und R7 zusammen mit dem sie tragenden C-Atom (C3-C6)-Cycloalkyl;
R8 H;
R9 H; (C1-C6)-Alkyl, das gegebenenfalls substituiert sein kann durch (C3-C6)-Cycloalkyl, Phenyl, (C5-C6)-Heteroaryl; CO-(C1-C6)-Alkyl, CO-(C1-C6)-Alkyl-Phenyl, CO-Phenyl, C(O)-O-(C1-C6)-Alkyl, CO-NH-Phenyl, SO2-(C1-C4)-Alkyl, SO2-(C1-C4)-Alkyl-SO2-(C1-C4)-alkyl, SO2-Tolyl, wobei Phenyl wiederum substituiert sein kann durch O-(C1-C3)-Alkyl;
sowie deren physiologisch verträgliche Salze.

4. Arzneimittel enthaltend eine oder mehrere der Verbindungen der Formel I gemäß einem oder mehreren der Ansprüche 1 bis 3.

5. Arzneimittel enthaltend eine oder mehrere der Verbindungen der Formel I gemäß einem oder mehreren der Ansprüche 1 bis 3 und einen oder mehrere Wirkstoffe, die günstige Wirkungen auf Stoffwechselstörungen oder damit assozierte Erkrankungen haben.

6. Arzneimittel enthaltend eine oder mehrere der Verbindungen der Formel I gemäß einem oder mehreren der Ansprüche 1 bis 3 und einen oder mehrere Antidiabetika.

7. Arzneimittel enthaltend eine oder mehrere der Verbindungen der Formel I gemäß einem oder mehreren der Ansprüche 1 bis 3 und einen oder mehrere Lipidmodulatoren.

8. Verwendung der Verbindungen der Formel I gemäß einem oder mehreren der Ansprüche 1 bis 3 zur Herstellung eines Arzneimittels zur Behandlung und/oder Prävention von Störungen des Fettsäurestoffwechsels und Glucoseverwertungsstörungen.

9. Verwendung der Verbindungen der Formel I gemäß einem oder mehreren der Ansprüche 1 bis 3 zur Herstellung eines Arzneimittels zur Behandlung und/oder Prävention von Störungen, bei denen Insulin Resistenz eine Rolle spielt.

10. Verwendung der Verbindungen der Formel I gemäß einem oder mehreren der Ansprüche 1 bis 3 zur Herstellung eines Arzneimittels zur Behandlung und/oder Prävention von Diabetes mellitus und der damit verbundenen Folgeerkrankungen.

11. Verwendung der Verbindungen der Formel I gemäß einem oder mehreren der Ansprüche 1 bis 3 zur Herstellung eines Arzneimittels zur Behandlung und/oder Prävention von Dyslipidämien und deren Folgen.

12. Verwendung der Verbindungen der Formel I gemäß einem oder mehreren der Ansprüche 1 bis 3 zur Herstellung eines Arzneimittels zur Behandlung und/oder Prävention von Zuständen, die mit dem Metabolischen Syndrom assoziert sind.

13. Verwendung der Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

14. Verwendung der Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen, bei denen Insulin Resistenz eine Rolle spielt.

15. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 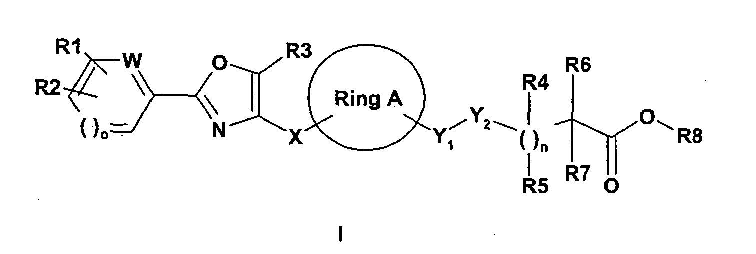 in which:
Ring A is cyclohexane-1,3-diyl;
R1, R2 independently of one another are H, F, Cl, Br, CF3, OCF3, (C1-C6)-alkyl, O-(C1-C6)-alkyl, SCF3, SF5, OCF2-CHF2, (C6-C10)-aryl, (C6-C10)-aryloxy, OH, N02; or
R1 and R2 together with the phenyl, pyridine, 1H-pyrrole, thiophene or furan ring form fused, partially or unsaturated bicyclic (C6-C10)-aryl, (C5-C11)-heteroaryl;
R3 is H, (C1-C6)-alkyl, (C3-C8)-cycloalkyl, (C1-C3)-alkyl-(C3-C8)-cycloalkyl, phenyl, (C1-C3)-alkyl-phenyl, (C5-C6)-heteroaryl, (C1-C3)-alkyl-(C5-C6)-heteroaryl or (C1-C3)-alkyl which is fully or partially substituted by F;
o is 0 or 1;
W isCHorNifo=1;
W is O, S or NR10 if o = 0;
X is (C1-C6)-alkanediyl, where in the alkanediyl group one or more carbon atoms may be replaced by oxygen atoms;
Y1 is (CR13R14)p;
p is 1 or 2;
Y2 is CH2, O, S, SO, SO2 or NR9;
n is 0-2;
R4 is H, (C1-C6)-alkyl; or F if Y2 is not O, NR9;
R5 is H, (C1-C6)-alkyl; or F if Y2 is not O, NR9;
R6 is H, (C1-C6)-alkyl; or F if n is not 0;
R7 is H, F (if n is not 0), (C1-C6)-alkyl, (C1-C6)-alkoxy, (C2-C6)-alkenyl, (C2-C6)-alkynyl, (C3-C8)-cycloalkyl, phenyl, where alkyl may be unsubstituted or substituted by one or more radicals selected from the group consisting of hydroxyl, phenyl, (C5-C11)-heteroaryl, (C1-C6)-alkoxy and NR11R12, and phenyl may be unsubstituted or substituted by one or more radicals from the group consisting of hydroxyl, (C1-G6)-alkoxy, F and CF3; with the proviso that R7 is not NR11 R12 or (C1-C6)-alkoxy if R6 = F;
R6 and R7 together with the carbon atom that carries them are (C3-C8)-cycloalkyl;
R8 is H, (C1-C6)-alkyl;
R9 is H, (C1-C6)-alkyl, (C2-C6)-alkenyl, (C2-C6)-alkynyl, aryl-(C1-C4)-alkyl, CO-(C1-C6)-alkyl, CO-(C6-C10)-aryl, CO-(C1-C6)-alkyl-(C6-C10)-aryl, CO-(C5-C11)-heteroaryl, C(O)-O-(C1-C6)-alkyl, C(O)-O-(C1-C6)-alkyl-(C6-C10)-aryl, C(O)-O-(C6-C10)-aryl, C(O)-O-(C5-C11)-heteroaryl, SO₂-(C1-C6)-alkyl, SO₂-(C1-C6)-alkyl-(C6-C10)-aryl, SO₂-(C1-C6)-alkyl-SO₂-(C1-C6)-alkyl, SO₂-(C6-C10)-aryl, SO₂-(C5-C11)-heteroaryl, where aryl and heteroaryl may be unsubstituted or substituted by (C1-C6)-alkyl, O-(C1-C6)-alkyl, F, Cl, CO-(C1-C6)-alkyl;
R10 is H, (C1-C6)-alkyl, (C1-C6)-alkyl-phenyl;
R11 is H, (C1-C6)-alkyl, (C1-C6)-alkyl-phenyl;
R12 is H, (C1-C6)-alkyl, (C1-C6)-alkyl-phenyl;
R13 is H, (C1-C6)-alkyl;
R14 is H, (C1-C6)-alkyl;
and its physiologically acceptable salts.

2. A compound of the formula I as claimed in claim 1 in which
Ring A is cyclohexane-1,3-diyl;
X is (C1-C6)-alkanediyl, where in the alkanediyl group the C1 or C2 carbon atom (to Ring A) may be replaced by an oxygen atom.

3. A compound of the formula I as claimed in one or more of claims 1 to 2, in which
Ring A is cis-cyclohexane-1,3-diyl
R1, R2 independently of one another are H, F, CF3, (C1-C6)-alkyl, O-(C1-C6)-alkyl, phenyl, or
R1 and R2 together with the phenyl ring = naphthyl;
R3 is (C1-C6)-alkyl, (C3-C8)-cycloalkyl, phenyl;
o is 0 or 1;
W is CH if o = 1;
W is O or S if o = 0;
X is CH₂O or CH₂-O-CH₂;
Y1 is CH2;
Y2 is CH2, O, S, SO, SO2 or NR9;
n is 0;
R4 is H;
R5 is H;
R6 is H, (C1-C6)-alkyl, benzyl;
R7 is H, (C1-C6)-alkyl, (C3-C6)-cycloalkyl, phenyl, benzyl,
R6 and R7 together with the carbon atom that carries them are (C3-C6)-cycloalkyl;
R8 is H;
R9 is H; (C1-C6)-alkyl which may be unsubstituted or substituted by (C3-C6)-cycloalkyl, phenyl, (C5-C6)-heteroaryl; CO-(C1-C6)-alkyl, CO-(C1-C6)-alkyl-phenyl, CO-phenyl, C(O)-O-(C1-C6)-alkyl, CO-NH-phenyl, SO₂-(C1-C4)-alkyl, SO₂-(C1-C4)-alkyl-SO₂-(C1-C4)-alkyl, SO₂-tolyl, where phenyl for its part may be substituted by O-(C1-C3)-alkyl;
and its physiologically acceptable salts.

4. A pharmaceutical, comprising one or more compounds of formula I as claimed in one or more of claims 1 to 3.

5. A pharmaceutical, comprising one or more compounds of the formula I as claimed in one or more of claims 1 to 3 and one or more active compounds having favorable effects on metabolic disorders or diseases associated therewith.

6. A pharmaceutical, comprising one or more compounds of the formula I as claimed in one or more of claims 1 to 3 and one or more antidiabetics.

7. A pharmaceutical, comprising one or more compounds of the formula I as claimed in one or more of claims 1 to 3 and one or more lipid modulators.

8. The use of the compounds of the formula I as claimed in one or more of claims 1 to 3 for preparing a pharmaceutical for the treatment and/or prevention of disorders of the fatty acid metabolism and glucose utilization disorders.

9. The use of the compounds of the formula I as claimed in one or more of claims 1 to 3 for preparing a pharmaceutical for the treatment and/or prevention of disorders where insulin resistance is involved.

10. The use of the compounds of the formula I as claimed in one or more of claims 1 to 3 for preparing a pharmaceutical for the treatment and/or prevention of diabetes mellitus and its sequelae.

11. The use of the compounds of the formula I as claimed in one or more of claims 1 to 3 for preparing a pharmaceutical for the treatment and/or prevention of dyslipidemias and their sequelae.

12. The use of the compounds of the formula I as claimed in one or more of claims 1 to 3 for preparing a pharmaceutical for the treatment and/or prevention of states associated with metabolic syndrome.

13. The use of the compounds as claimed in one or more of claims 1 to 3 in combination with at least one further active compound for preparing a pharmaceutical for the treatment and/or prevention of disorders of the fatty acid metabolism and glucose utilization disorders.

14. The use of the compounds as claimed in one or more of claims 1 to 3 in combination with at least one further active compound for preparing a pharmaceutical for the treatment and/or prevention of disorders in which insulin resistance is involved.

15. A process for preparing a pharmaceutical comprising one or more of the compounds as claimed in one or more of claims 1 to 3, which comprises mixing the active compound with a pharmaceutically acceptable carrier and bringing this mixture into a form suitable for administration.

## Revendications

1. Composés de formule I 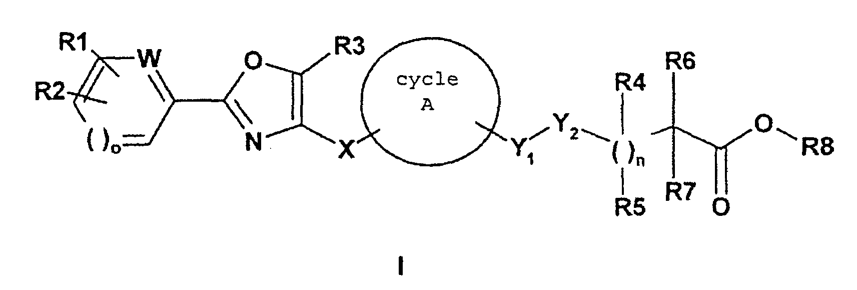 dans laquelle :
le cycle A est cyclohexane-1,3-diyle ;
R1, R2 indépendamment l'un de l'autre, sont H, F, Cl, Br, CF3, OCF3, alkyl(C₁-C₆), O-alkyl(C₁-C₆), SCF3, SF5, OCF2-CHF2, aryl(C₆-C₁₀), aryi(C₆-C₁₀)oxy, OH, N02 ; ou
R1 et R2 conjointement avec le cycle phényle, pyridine, 1H-pyrrole, thiophène ou furane, forment aryl(C₆-C₁₀), hétéroaryl(C₅-C₁₁) bicyclique partiellement saturé ou insaturé, et condensé ;
R3 est H, alkyl(C₁-C₆), cycloalkyl(C₃-C₈), alkyl(C₁-C₃)-cycloalkyl(C₃-C₈), phényle, alkyl(C₁-C₃)-phényle, hétéroaryl(C₅-C₆), alkyl(C₁-C₃)-hétéroaryl(C₅-C₆), ou alkyl(C₁-C₃) qui est totalement ou partiellement substitué par F ;
o vaut 0 ou 1 ;
W est CH ou N si o=1;
W est O, S ou NR10 si o=0 ;
X est alcane(C₁-C₆)diyle, où dans le groupement alcanediyle, un ou plusieurs atomes de carbone peuvent être remplacés par des atomes d'oxygène ;
Y1 est (CR13R14)p ;
p vaut 1 ou 2;
Y2 est CH₂, O, S, SO, SO₂ ou NR9 ;
n vaut 0-2 ;
R4 est H, alkyl(C₁-C₆) ; ou F si Y2 n'est pas O, NR9 ;
R5 est H, alkyl(C₁-C₆) ; ou F si Y2 n'est pas O, NR9 ;
R6 est H, alkyl(C₁-C₆) ; ou F si n n'est pas 0 ;
R7 est H, F (si n n'est pas 0), alkyl(C₁-C₆), alcoxy(C₁-C₆), alcényl(C₂-C₆), alcynyi(C₂-C₆), cycloalkyl(C₃-C₈), phényle, où alkyle peut être non substitué ou substitué par un ou plusieurs radicaux choisis parmi le groupe constitué par hydroxyle, phényle, hétéroaryl(C₅-C₁₁), alcoxy(C₁-C₆) et NR11R12, et phényle peut être non substitué ou substitué par un ou plusieurs radicaux issus du groupe constitué par hydroxy, alcoxy(C₁-C₆), F et CF3; à condition que R7 ne soit pas NR11R12 ou alcoxy(C₁-C₆) si R6 = F ;
R6 et R7, conjointement avec l'atome de carbone les portant, sont cycloalkyl(C₃-C₈);
R8 est H, alkyl(C₁-C₆) ;
R9 est H, alkyl(C₁-C₆), alcényl(C₂-C₆), alcynyl(C₂-C₆), aryl-alkyl(C₁-C₄), CO-alkyl(C₁-C₆), CO-aryl(C₆-C₁₀), CO-alkyl(C₁-C₆)-aryl(C₆-C₁₀), CO-hétéroaryl(C₅-C₁₁), C(O)-O-alkyl(C₁-C₆), C(O)-O-alkyl(C₁-C₆)-aryl(C₆-C₁₀), C(O)-O-aryl(C₆-C₁₀), C(O)-O-hétéroaryl(C₅-C₁₁), SO₂-alkyl(C₁-C₆), SO₂-alkyl(C₁-C₆)-aryl(C₆-C₁₀), SO₂-alkyl(C₁-C₆)-SO₂-alkyl(C₁-C₆), SO₂-aryl(C₆-C₁₀), SO₂-hétéroaryl(C₅-C₁₁), où aryle ou hétéroaryle peuvent être non substitués ou substitués par alkyl(C₁-C₆), O-alkyl(C₁-C₆), F, Cl, CO-alkyl(C₁-C₆) ;
R10 est H, alkyl(C₁-C₆), alkyl(C₁-C₆)-phényle ;
R11 est H, alkyl(C₁-C₆), alkyl(C₁-C₆)-phényle ;
R12 est H, alkyl(C₁-C₆), alkyl(C₁-C₆)-phényle ;
R13 est H, alkyl(C₁-C₆) ;
R14 est H, alkyl(C₁-C₆) ;
et ses sels physiologiquement acceptables.

2. Composés de formule I selon la revendication 1, dans laquelle
le cycle A est cyclohexane-1,3-diyle ;
X est alcane(C₁-C₆)diyle, où dans le groupement alcanediyle, l'atome de carbone C1 ou C2 (par rapport au cycle A) peut être remplacé par un atome d'oxygène.

3. Composés de formule I selon l'une ou plusieurs parmi les revendications 1 à 2, dans laquelle
le cycle A est cis-cyclohexane-1,3-diyle ;
R1, R2, indépendamment l'un de l'autre, sont H, F, CF₃, alkyl(C₁-C₆), O-alkyl(C₁-C₆), phényle ; ou
R1 et R2, conjointement avec le cycle phényle, sont naphtyle ;
R3 est alkyl(C₁-C₆), cycloalkyl(C₃-C₈), phényle;
o vaut 0 ou 1 ;
W est CH si o = 1 ;
W est 0 ou S si o = 0 ;
X est CH₂-O ou CH₂-O-CH₂ ;
Y1 est CH₂ ;
Y2 est CH₂, O, S, SO, SO₂, NR9 ;
n vaut 0 ;
R4 est H;
R5 est H ;
R6 est H, alkyl(C₁-C₆), benzyle ;
R7 est H, alkyl(C₁-C₆), cycloalkyl(C₃-C₆), phényle, benzyle ;
R6 et R7, conjointement avec l'atome de carbone les portant, sont cycloalkyl(C₃-C₆) ;
R8 est H ;
R9 est H ; alkyl(C₁-C₆) pouvant être non substitué ou substitué par cycloalkyl(C₃-C₆), phényle, hétéroaryl(C₅-C₆) ; CO-alkyl(C₁-C₆), CO-alkyl(C₁-C₆)-phényle, CO-phényle, C(O)-O-alkyl(C₁-C₆), CO-NH-phényle, S0₂-alkyl(C₁-C₄), SO₂-alkyl(C₁-C₄)-SO₂-alkyl(C₁-C₄), SO₂-tolyle, où phényle en ce qui le concerne peut être substitué par O-alkyl(C₁-C₃) ;
et ses sels physiologiquement acceptables.

4. Produit pharmaceutique, comprenant un ou plusieurs composés de formule I selon l'une ou plusieurs parmi les revendications 1 à 3.

5. Produit pharmaceutique, comprenant un ou plusieurs composés de formule I selon l'une ou plusieurs parmi les revendications 1 à 3 et un ou plusieurs composés actifs ayant des effets favorables sur des troubles métaboliques ou des maladies y étant associées.

6. Produit pharmaceutique, comprenant un ou plusieurs composés de formule I selon l'une ou plusieurs parmi les revendications 1 à 3 et un ou plusieurs antidiabétiques.

7. Produit pharmaceutique, comprenant un ou plusieurs composés de formule I selon l'une ou plusieurs parmi les revendications 1 à 3 et un ou plusieurs modulateurs de lipides.

8. Utilisation des composés de formule I selon l'une ou plusieurs parmi les revendications 1 à 3 pour la préparation d'un produit pharmaceutique pour le traitement et/ou la prévention de troubles du métabolisme des acides gras et de troubles de l'utilisation du glucose.

9. Utilisation des composés de formule I selon l'une ou plusieurs parmi les revendications 1 à 3 pour la préparation d'un produit pharmaceutique pour le traitement et/ou la prévention de troubles où la résistance à l'insuline est mise en jeu.

10. Utilisation des composés de formule I selon l'une ou plusieurs parmi les revendications 1 à 3 pour la préparation d'un produit pharmaceutique pour le traitement et/ou la prévention du diabète sucré et de ses séquelles.

11. Utilisation des composés de formule 1 selon l'une ou plusieurs parmi les revendications 1 à 3 pour la préparation d'un produit pharmaceutique pour le traitement et/ou la prévention de dyslipidémies et de leurs séquelles.

12. Utilisation des composés de formule I selon l'une ou plusieurs parmi les revendications 1 à 3 pour la préparation d'un produit pharmaceutique pour le traitement et/ou la prévention d'états associés au syndrome métabolique.

13. Utilisation des composés selon l'une ou plusieurs parmi les revendications 1 à 3 en association avec au moins un composé actif supplémentaire pour la préparation d'un produit pharmaceutique pour le traitement et/ou la prévention de troubles du métabolisme des acides gras et de troubles de l'utilisation du glucose.

14. Utilisation des composés selon l'une ou plusieurs parmi les revendications 1 à 3 en association avec au moins un composé actif supplémentaire pour la préparation d'un produit pharmaceutique pour le traitement et/ou la prévention de troubles dans lesquels la résistance à l'insuline est mise en jeu.

15. Procédé de préparation d'un produit pharmaceutique comprenant un ou plusieurs composés selon l'une ou plusieurs parmi les revendications 1 à 3, **caractérisé en ce que** le composé actif est mélangé avec un véhicule pharmaceutiquement acceptable et ce mélange est converti en une forme convenable pour une administration.
